(19) Europäisches Patentamt
European Patent Office
Office européen des brevets

(11) EP 1 878 753 A1

(12) **EUROPEAN PATENT APPLICATION**
published in accordance with Art. 153(4) EPC

(43) Date of publication:
**16.01.2008 Bulletin 2008/03**

(21) Application number: **06728732.6**

(22) Date of filing: **28.02.2006**

(51) Int Cl.:
***C08B 37/06*** *(2006.01)*

(86) International application number:
**PCT/JP2006/304413**

(87) International publication number:
**WO 2006/090935 (31.08.2006 Gazette 2006/35)**

(84) Designated Contracting States:
**DE FR**

(30) Priority: **28.02.2005 JP 2005053479**
**25.03.2005 JP 2005088860**

(71) Applicants:
- **Nichirei Foods Inc.**
  **Tokyo 104-8402 (JP)**
- **Nichirei Biosciences Inc.**
  **Tokyo 104-8402 (JP)**

(72) Inventors:
- **KAWAGUCHI, Masakazu**
  **c/o Res.&Dev. Center, Nichirei Biosciences Inc.**
  **Tokyo, 1890003 (JP)**
- **SASAKI, Akiko**
  **c/o Res.&Dev. Center, Nichirei Biosciences Inc.**
  **Tokyo, 1890003 (JP)**
- **NAGAMINE, Kenichi**
  **c/o Res.&Dev. Center, Nichirei Biosciences Inc.**
  **Tokyo, 1890003 (JP)**
- **KAMIHIGASHI, Jun**
  **c/o Res.&Dev. Division Nichirei Foods Inc.**
  **Chiba, 2618545 (JP)**

(74) Representative: **Woods, Geoffrey Corlett**
**J.A. KEMP & CO.**
**Gray's Inn**
**14 South Square**
**London WC1R 5JJ (GB)**

# (54) ACEROLA FRUIT-DERIVED PECTIN AND USE THEREOF

(57) The present invention relates to a pectin derived from an acerola fruit or a hydrolysate thereof, comprising a complex formed of Aceronidin, which is a novel polyphenol compound. The pectin of the present invention can be used as an active ingredient of an antioxidant or a skin-whitening agent.

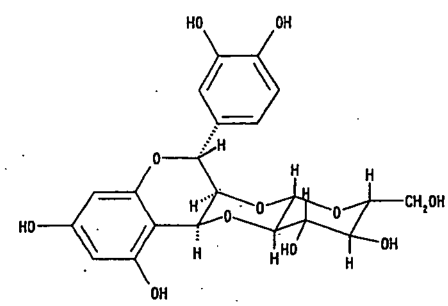



Printed by Jouve, 75001 PARIS (FR)

## Description

Technical Field

The present invention relates to an acerola fruit-derived pectin useful as an antioxidant or a skin-whitening agent for oral administration.

Background Art

Oxidation or hyperoxidation of fat and oil ingredients or the like by oxygen in air is the most troublesome factor in storage, preservation, and processing steps for fats and oils, goods containing them, food products, cosmetics, pharmaceutical preparations, and the like. In particular, unsaturated fatty acid such as linoleic acid or linolenic acid contained in fat and oil is easily hyperoxidated by oxygen to generate hyperoxidated lipids or free radicals, in addition to carcinogenic substances *(*Shokuhin-no-hoso (Food Packaging), vol. 17, p. 106 (1986)). When oxidation or hyperoxidation takes place, not only staining, discoloration, degeneration, abnormal odor, and decreased effectiveness of nutritive value, but also poison generation or the like take place, resulting in deterioration in product quality.
Various antioxidants have been used conventionally to prevent deterioration in product quality via suppression of the oxidation of unsaturated fatty acid. These antioxidants have effects of acting on peroxide radicals that are generated upon oxidation, so as to stop chain oxidation reactions or of acting on free radicals, so as to stop oxidation reactions. As antioxidants, synthetic antioxidants such as butylhydroxyanisol (BHA) and butylhydroxytoluene (BHT), for example, have been generally used conventionally. However, as the chances of using these synthetic antioxidants have increased, the safety thereof has become an issue. The stronger the negative responses of consumers, the less the consumption of such antioxidants. Moreover, these oil soluble antioxidants are also problematic in that they lack solubility in aqueous solutions.
Therefore, expectations for natural-product-derived antioxidants having high safety are growing considerably.
Conventionally known natural antioxidants are vitamin E ($\alpha$-tocopherol) and vitamin C (ascorbic acid), for example. However, vitamin E has high lipid solubility and vitamin C has high water solubility, so that they are inappropriate for suppression of lipid oxidation in the food industry. This is because: since processed products (e.g., fish, meat of livestock, and grains), salt-cured food products, fat-and-oil-containing seasonings, and the like, for which lipid oxidation should be suppressed, each generally form a mixed system containing fats and oils and water-based components, the application of these antioxidants having extreme lipid solubility or water solubility is limited. Moreover, vitamin E is problematic in that it has its own unfavorable flavor in a food product so that the amount thereof to be added and its application are limited. Vitamin E and vitamin C are also problematic in that their anti-oxidation activity do not last long in a stable manner.
Pectins isolated from plants by various methods are known to exert antioxidative activities for lipids under specific conditions. However, the antioxidative activities are thought to be very weak, so that such pectins are not used as general antioxidants. For example, a pectin isolated from bean curd refuse (soybean) and its enzyme-treated product are known to have an effect of preventing lipid oxidation (FOOD SCIENCE, VOL. 36, NO. 11, pp. 93-102 (1994)), but their anti-oxidation activity is insufficient. In addition, pectins are polysaccharides existing in various plants, which are composed of galacturonic acid, its methylester, other neutral sugars, or the like. Examples of a neutral sugar include rhamnose, arabinose, and galactose, but the types and composition ratios thereof are known to significantly differ from each other depending on the plants involved (Written by Takaaki Manabe, First edition, "Science and Food Texture of Pectin," Saiwai Shobo, pp. 8-22 (2001)).
In the meantime, skin-whitening agents have been conventionally developed mainly for cosmetics and quasi-drugs. Hence, many active ingredients such as arbutin and ascorbic acid derivatives have been discovered. However, most of these active ingredients are used as external skin preparations. Currently, an example of a pharmaceutical preparation for suppressing pigmentation due to flecks, sunburn, or the like via oral ingestion is a product containing ascorbic acid (vitamin C) as a major active ingredient with cysteine (which is an amino acid) and vitamin B complex, which are expected to exert an synergistic effect, compounded therewith. Specifically, ascorbic acid is thought to be the most appropriate ingredient that can be expected to safely exert an effect of suppressing pigmentation via oral ingestion.
As a fruit that is rich in ascorbic acid, acerola (scientific name: *Malpighia emarginata* DC) is well known. The use of such acerola as an active ingredient for a skin-whitening agent is described in JP Patent No. 3513871. The application of the acerola is limited to external skin preparations such as cosmetics. Furthermore, the use of a composition as a skin-whitening agent is described in JP Patent No. 3076787, wherein the composition substantially contains no ascorbic acid and is obtained by fermentation of acerola. No skin-whitening agent that is produced using acerola, is composed of ascorbic acid and other ingredients, and is effective for oral administration has been discovered.
There also are reports concerning the relationship between a component derived from a pectin contained in fruit pulp and a skin-whitening effect. It is reported in JP Patent No. 3596953 that oligogalacturonic acid exerted an effect

of suppressing melanin production in an animal cell test. Here, "oligogalacturonic acid" is formed via binding of approximately 2 to 10 galacturonic acids. Furthermore, in Shokuhin-no-hoso (Food Packaging), vol. 17, p.106 (1986), it was demonstrated that a pectin degradation product derived from tomato juice has an effect of suppressing melanin pigment generation. It is also described in this document that the effect of suppressing the melanin pigment has not been confirmed for galacturonic acid and polygalacturonic acid. It was thought that the results in JP Patent No. 3596953 conflict with that in Shokuhin-no-hoso (Food Packaging), vol. 17, p. 106 (1986). This may be because the relevant source plants greatly differ from each other in terms of pectin structure and nature. In both JP Patent No. 3596953 and Eiji Naru et al., Fragrance Journal, Vol. 32, No. 8, pp. 24-30 (2004), only the effect against animal cells was examined. A skin-whitening effect exerted by a combination of a component derived from a pectin and other components has never been reported.

Disclosure of the Invention

Objects to be Achieved by the Invention

An object of the present invention is to provide a water-soluble antioxidant isolated from the natural world and a method for producing such antioxidant.

Another object of the present invention is to provide a skin-whitening agent for oral administration isolated from the natural world and a method for producing such skin-whitening agent.

Means to Achieve the Objects

The present application includes the following inventions.

(1) A pectin derived from an acerola fruit or a hydrolysate thereof, comprising a complex formed of a pectin backbone and a polyphenol compound represented by chemical formula:

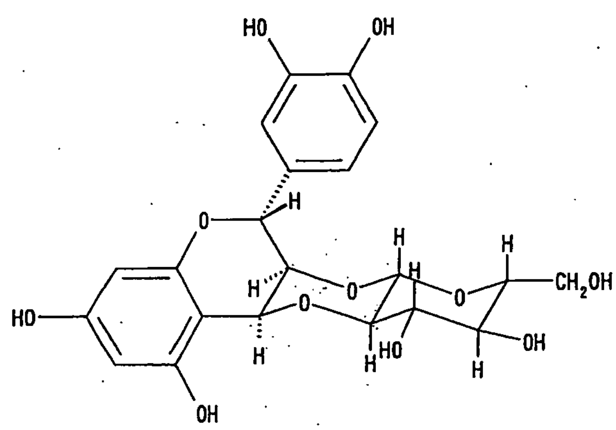

(2) The method for producing the pectin according to (1), comprising a step of isolating or concentrating a pectin from an acerola fruit or a processed product thereof.

(3) The method for producing the pectin hydrolysate according to (1), comprising a step of isolating or concentrating a pectin from an acerola fruit or a processed product thereof and a step of hydrolyzing the pectin.

(4) The method according to (3), comprising a step of hydrolyzing a pectin in puree prepared from an acerola fruit through treatment of the puree with pectinase and a step of isolating or concentrating the hydrolyzed pectin from a supernatant of the processed product in the former step.

(5) The method according to any one of (2) to (4), wherein the step of isolating or concentrating a pectin is a step of precipitating a pectin using ethanol.

(6) The method according to any one of (2) to (4), wherein the step of isolating or concentrating a pectin is a step of isolating or concentrating a pectin using a separation membrane.

(7) The method according to (6), wherein the separation membrane is an ultrafiltration membrane.

(8) The method according to (7), wherein the ultrafiltration membrane has a molecular weight cut-off ranging from 10,000 to 100,000.

(9) A material containing a pectin derived from an acerola fruit, which is produced by a method comprising a step of isolating or concentrating a pectin from an acerola fruit or a processed product thereof.

(10) A material containing a hydrolysate of a pectin derived from an acerola fruit, which is produced by a method comprising a step of isolating or concentrating a pectin from an acerola fruit or a processed product thereof and a step of hydrolyzing the pectin.
(11) The material according to (10), which is produced by a method comprising a step of hydrolyzing a pectin in puree prepared from an acerola fruit through treatment of the puree with pectinase and a step of isolating or concentrating the hydrolyzed pectin from a supernatant of the processed product resulting from the former step.
(12) The material according to any one of (9) to (11), wherein the step of isolating or concentrating a pectin is a step of precipitating a pectin using ethanol.
(13) The material according to any one of (9) to (11), wherein the step of isolating or concentrating a pectin is a step of isolating or concentrating a pectin using a separation membrane.
(14) The material according to (13), wherein the separation membrane is an ultrafiltration membrane.
(15) The material according to (14), wherein the ultrafiltration membrane has a molecular weight cut-off ranging from 10,000 to 100,000.
(16) An antioxidant, containing the pectin or the hydrolysate thereof according to (1) as an active ingredient.
(17) An antioxidant, containing the material according to any one of (9) to (15) as an active ingredient.
(18) An antioxidant for lipids, containing a processed product of an acerola fruit (excluding a processed product of an acerola seed) as an active ingredient.
(19) The antioxidant according to (18), wherein the processed product of an acerola fruit contains polyphenol and/or ascorbic acid.
(20) A food product having an antioxidative effect, to which the antioxidant according to any one of (16) to (19) is added.
(21) A method for producing a food product, comprising a step of enhancing oxidation stability of a food product using the antioxidant according to any one of (16) to (19).
(22) A skin-whitening agent for oral administration, containing the pectin or the hydrolysate thereof according to (1) as an active ingredient.
(23) A skin-whitening agent for oral administration, containing the material according to any one of (9) to (15) as an active ingredient.
(24) The skin-whitening agent for oral administration according to (22) or (23), further containing ascorbic acid.
(25) A food product having a skin-whitening effect, to which the skin-whitening agent for oral administration according to any one of (22) to (24) is added.
(26) A method for producing a skin-whitening agent for oral administration, comprising a step of hydrolyzing a pectin contained in the pulp of an acerola fruit or a processed product of an acerola fruit containing ascorbic acid and such pulp so that the amount of galacturonic acid is 5% by weight or more with respect to ascorbic acid.
(27) The method according to (26), further comprising a step of substantially removing glucose and fructose.

The term "antioxidant (for lipids), containing a predetermined component as an active ingredient" in the present invention indicates both a composition having antioxidative activities (for lipids) in which a predetermined component is contained in a natural condition and a composition having antioxidative activities (for lipids) to which a predetermined component is artificially added.
The term "skin-whitening agent for oral administration, containing a predetermined component as an active ingredient" in the present invention indicates both a composition having a skin-whitening effect in which a predetermined component is contained in a natural condition and a composition having a skin-whitening effect to which a predetermined component is artificially added.
The term "a food product having an antioxidative effect, to which the antioxidant is added" in (20) above means a food product having an antioxidative effect to which a predetermined antioxidant is artificially added.
The term "a food product having a skin-whitening effect, to which the skin-whitening agent for oral administration is added" in (25) above means a food product having a skin-whitening effect to which a predetermined skin-whitening agent for oral administration is artificially added.

Effect of the Invention

The acerola fruit-derived pectin according to the present invention, comprising a complex formed of polyphenol, is useful as an antioxidant and also useful as a skin-whitening agent for oral administration.
This description includes part or all of the contents as disclosed in the description and/or drawings of Japanese Patent Application Nos. 2005-53479 and 2005-88860, which are priority documents of the present application.

Brief Description of the Drawings

Fig. 1 shows the chromatogram of a sample extracted from an acerola fruit-derived pectin, as analyzed by analytical HPLC.
Fig. 2 shows the chromatogram of a sample extracted from an acerola fruit-derived pectin, as analyzed by preparative HPLC.
Fig. 3 shows the chromatogram obtained by subjecting the component fractionated by preparative HPLC to analytical HPLC.
Fig. 4A shows the spectrum data for the peak at 22.4 minutes shown in the chromatogram of Fig. 3.
Fig. 4B shows the spectrum data for Aceronidin.
Fig. 5 shows comparison of the $^1$H NMR spectrum for Aceronidin (upper case) with that for polyphenol (lower case) separated from an acerola fruit-derived pectin.
Fig. 6A shows the total ion chromatogram of Aceronidin.
Fig. 6B shows the high-resolution ESI mass spectrum for Aceronidin.
Fig. 7 shows the $^1$H NMR spectrum for Aceronidin.
Fig. 8 shows the $^{13}$C NMR spectrum for Aceronidin.
Fig. 9 shows the DEPT spectrum for Aceronidin.
Fig. 10 shows the DQF-COSY spectrum for Aceronidin.
Fig. 11 shows the HSQC spectrum for Aceronidin.
Fig. 12 shows the HMBC spectrum for Aceronidin.
Fig. 13 shows the NOESY spectrum for Aceronidin.
Fig. 14 shows the results of determining the ability of suppressing auto-oxidation of linoleic acid of BHA, concentrated acerola juice, and acerola powder.
Fig. 15 shows the results of determining the ability of vitamin C to suppress auto-oxidation of linoleic acid.
Fig. 16 shows the results of determining the ability of acerola-derived C18 column-adsorbed components, an acerola powder from which the C18 column-adsorbed components have been removed, and an acerola powder to suppress auto-oxidation of linoleic acid.
Fig. 17 shows the results of determining the ability of an acerola powder from which C 18 column-adsorbed components have been removed and an acerola powder from which C18 column-adsorbed components and vitamin C have been removed to suppress auto-oxidation of linoleic acid.
Fig. 18 shows the results of determining the ability of an acerola-derived pectin (molecular weight of 2,000,000) treated with acid and a pectin (molecular weight of 20,000 or less) treated with an enzyme to suppress auto-oxidation of linoleic acid.
Fig. 19 shows photographs showing a test group of salt-cured salmon samples to which acerola has been added after storage under fluorescent lighting conditions and a control test group of a salt cured salmon samples.
Fig. 20 shows the transition of the "a" value during storage under fluorescent lighting conditions of salt cured salmon samples that have been treated with an immersion fluid containing an acerola powder.
Fig. 21 shows the results of conducting a pigmentation suppression test using brown guinea pigs.

## Preferred Embodiments of the Invention

### 1. Acerola fruit-derived pectin

The area of production or the varieties of acerola (scientific name: *Malpighia emarginata* DC) fruits to be used in the present invention are not particularly limited. Examples of areas of production include Okinawa, Japan, Brazil, and Vietnam.

Acerola fruits in the present invention may refer to all portions of fruits, including seeds, or acerola fruits subj ected to general treatment such as removal of seeds, peeling, or the like.

As acerola fruits, mature fruits or green fruits can be used. The use of green fruits is preferable. "Green fruit" is a fruit that has sufficiently grown so that juice can be squeezed from such fruit, but has green to yellow coloration because the fruit (immature fruit) is at a stage before the maturity grade thereof becomes high enough to have red coloration.

In addition to an acerola fruit itself, processed products of various types of acerola fruit can be used in the present invention, as long as they contain pectin. For example, puree, fruit juice, or pulp prepared from an acerola fruit, products of crushed or ground acerola fruits, or acerola fruit extracts can be used. As a starting material for production of the pectin according to the present invention, puree, fruit juice, or pulp prepared from an acerola fruit is preferable. Puree is particularly preferable.

Fruit juice can be obtained by squeezing an acerola fruit in accordance with conventional techniques. A residue obtained after squeezing of a fruit to collect fruit juice is referred to as "pulp."

A crushed product of an acerola fruit can be obtained by crushing, using a mixer or the like, edible parts and

seeds of the acerola fruit or edible parts of the same from which seeds have been removed. Furthermore, such crushed product subjected to treatment such as extraction or freeze-drying can also be used.

An acerola fruit extract can be obtained by subjecting an acerola fruit to extraction using water, an organic solvent, or the like. Conditions for extraction are not particularly limited, as long as no pectin is lost under such conditions.

Not only a pectin derived from an acerola fruit but also a general "pectin" has a structure wherein: homogalacturonan composed of polygalacturonic acid formed by $\alpha$-(1$\rightarrow$4)-linked galacturonic acid and rhamnogalacturonan composed of galacturonic acid and rhamnose repeatedly bound to each other form the main chain; and side chains such as galactan and arabinan branch from rhamnose. Carboxyl groups of galacturonic acid are methyl-esterified or acetyl-esterified at different proportions. In addition, pectin is thought to have a cross-linked structure via binding with a polyvalent cation such as calcium or magnesium.

In the present invention, the sugar chain structure of the pectin composed of the main chain comprising homogalacturonan and rhamnogalacturonan and side chains that branch from the main chain is referred to as "pectin backbone."

Surprisingly, the present inventors have discovered that a pectin contained in an acerola fruit is a complex formed of the pectin backbone and a polyphenol compound represented by the chemical formula:

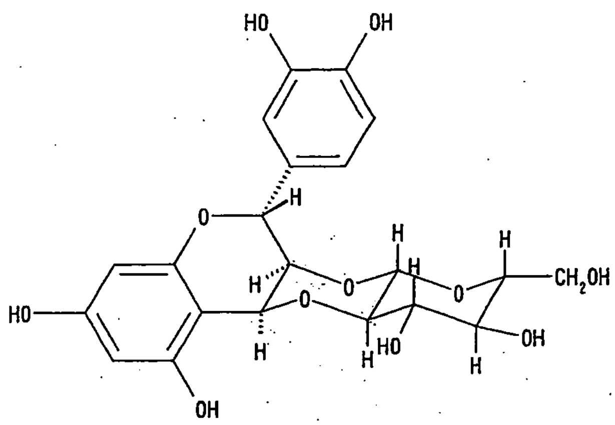

The polyphenol compound is a compound that has been isolated for the first time by the present inventors and named Aceronidin. A patent for this compound was applied on December 22, 2004, under JP Patent Application No. 2004-372266.

"Comprising a complex formed of a pectin backbone and a polyphenol compound" in the present invention means that Aceronidin and the pectin backbone coexist in a manner such that they are inseparable from each other by a general isolation or concentration method for pectin, such as ethanol precipitation or membrane filtration (e.g., ultrafiltration). The specific structure of the Aceronidin-pectin backbone complex has not been elucidated. A possible structure thereof may be a structure wherein a portion of Aceronidin and a portion of the pectin backbone are linked via covalent bond (such as ester linkage or glycoside linkage), a structure wherein they are linked via hydrogen bond, or a structure wherein they are linked via hydrophobic bond, for example. Furthermore, the quantitative ratio of Aceronidin to the pectin backbone in the Aceronidin-pectin backbone complex is not particularly limited. "Pectin derived from an acerola fruit" in the present invention means a complex that is formed by the pectin backbone and Aceronidin, unless particularly limited. Furthermore, "isolating or concentrating a pectin" means to isolate or concentrate a pectin that is a complex of the pectin backbone and Aceronidin. Furthermore, the pectin according to the present invention may be expressed as "anti-oxidative pectin," meaning "pectin having antioxidative potency."

"Hydrolysate of a pectin derived from an acerola fruit" in the present invention refers to a product obtained via chemical or enzymatic hydrolysis of linkage between constituent sugars in the pectin backbone comprising the main chain and the side chains of the acerola fruit-derived pectin, and particularly, linkage between constituent sugars in the main chain. According to studies conducted by the present inventors, even after hydrolysis of the pectin backbone with pectinase (which hydrolyzes the main chain), Aceronidin and a hydrolysate (thought to be mainly composed of side chains) of the pectin backbone are inseparable by treatment for isolating or concentrating pectin, such as ethanol precipitation or ultrafiltration. Specifically, "hydrolysate of a pectin derived from an acerola fruit" also comprises a complex formed of a hydrolysate of the pectin backbone and Aceronidin. Further studies conducted by the present inventors have revealed that both an acerola fruit-derived pectin with a molecular weight of approximately 2,000,000 and a hydrolysate of the acerola fruit-derived pectin with a molecular weight of 20,000 or less have antioxidative activities. Hence, the molecular weight of the acerola fruit-derived pectin or the same of the hydrolysate thereof to be used in the present

invention are not particularly limited.

The acerola fruit-derived pectin according to the present invention is produced by isolating or concentrating the pectin from an acerola fruit or a processed product thereof. Examples of a method for isolating or concentrating such pectin include a method that involves precipitating the pectin via the addition of ethanol to a pectin-containing sample and a method that involves isolating or concentrating the pectin with the use of a separation membrane. Of these methods, a plurality of the same types of or different types of method may be combined. Repetition of a step of precipitating the pectin using ethanol makes it possible to remove water-soluble ascorbic acid, or organic acid or polyphenols that are easily soluble in alcohol. As a separation membrane, an ultrafiltration membrane is preferable. An ultrafiltration membrane is a membrane that can block the passage of particles or polymers in sizes generally ranging from 0.1 $\mu$m to 2 nm (molecular weight of several hundred to multimillions). Such an ultrafiltration membrane preferably has a nominal molecular weight cut-off of 1,000 or higher and more preferably a nominal molecular weight cut-off ranging from 10,000 to 100,000.

When pectin is isolated or concentrated from an acerola fruit or a processed product of an acerola fruit containing pulp such as acerola pulp and acerola puree, a strong acid such as hydrochloric acid or nitric acid is added to a pulp-containing sample before the procedure described in the above last paragraph, so that acid-soluble components may also be dissolved.

A pectin concentrated solution obtained by membrane filtration can be directly used as an active ingredient of an antioxidant or a skin-whitening agent. Such a pectin concentrated solution can also be powderized and then used. Powderization of a pectin concentrated solution can be performed by a freeze-drying method or a spray-drying method. Upon membrane filtration, any concentration degree for a concentrate can be selected, and it is preferably a concentration degree at which the solid content of the pectin in a concentrated solution is 10% (W/W) or higher and preferably 20% (W/W) or higher. For powderization of such a concentrated solution, there may be a need to remove glucose and fructose in the concentrated solution via desugaring using yeast or the like. When a concentration degree is within the above range, desugaring can be easily performed. Furthermore, a molecular weight cut-off of an ultrafiltration membrane and concentration conditions are appropriately selected and then glucose and fructose are removed from the concentrate by membrane filtration, making desugaring unnecessary upon powderization of the concentrated solution.

A hydrolysate of the acerola fruit-derived pectin according to the present invention is produced by a method that comprises the above step of isolating or concentrating the pectin and a step of hydrolyzing the pectin. The hydrolysis step may be performed either before or after the former step. Pectin hydrolysis means to chemically or enzymatically hydrolyze linkage between constitutive sugars in the backbone of the acerola fruit-derived pectin, and, in particular, linkage between constitutive sugars in the main chain. Hydrolysis is preferably performed using pectinase. When pectinase is used, types of pectinase are not particularly limited. For example, pectinase having endo-polygalacturanase activity can be used. Origins of pectinase are not particularly limited. An example of pectinase is derived from a microbe of the genus *Aspergillus* (e.g., *A. Pulverulentus* or *A. niger*). A hydrolysate produced using pectinase of the acerola fruit-derived pectin contains free galacturonic acid that is the digest of the main chain. For separation of only a component that forms a complex with Aceronidin from the thus obtained hydrolysates, such component is adsorbed to a hydrophobic column (e.g., C18 column) and then the adsorbed component can be collected via elution. The thus collected component is also an embodiment of a hydrolysate of the pectin according to the present invention.

In the most preferred embodiment, a method for producing a hydrolysate of the acerola fruit-derived pectin according to the present invention comprises a step of hydrolyzing a pectin in puree prepared from an acerola fruit via treatment using pectinase and a step of isolating or concentrating the hydrolyzed pectin from the supernatant of a product treated in the former step. In this embodiment, it is preferable to filter the supernatant through preferably a 0.2-$\mu$m filter before isolation or concentration of the hydrolyzed pectin. Also in this embodiment, it is preferable to concentrate the hydrolyzed pectin using an ultrafiltration membrane. A concentrated solution obtained by ultrafiltration is preferably further powderized. The thus obtained powder can easily be finally milled and has high fluidity and low hygroscopicity.

2. Application of acerola fruit-derived pectin

The acerola fruit-derived pectin or the hydrolysate thereof according to the present invention have an effect of suppressing auto-oxidation of lipids and an effect of scavenging free radicals, so that it can be used as an active ingredient of an antioxidant.

The acerola fruit-derived pectin or the hydrolysate thereof according to the present invention also has a skin-whitening effect that is exerted via oral administration, so that it can be used as an active ingredient of such a skin-whitening agent.

3 Antioxidant for lipids, containing the processed product of an acerola fruit as an active ingredient

Surprisingly, the present inventors have discovered that components (containing a polyphenol compound) that

are adsorbed to a hydrophobic column and ascorbic acid contained in an acerola fruit are also useful as antioxidants for lipids. Specifically, the present invention further relates to an antioxidant for lipids, which contains a processed product of an acerola fruit as an active ingredient.

Such processed product of an acerola fruit is preferably water-soluble because it can be particularly generally used in the food industry.

In the embodiment of the present invention, such processed product of an acerola fruit can be used as an active ingredient of an antioxidant for lipids, as long as it contains at least one of and preferably both an acerola-fruit-derived polyphenol compound and ascorbic acid. However, in this embodiment of the present invention, such an acerola processed product is derived from portions other than acerola seeds, such as acerola fruit pulp and pericarp.

Specific examples of such acerola-fruit-derived polyphenol compound include Aceronidin, anthocyanin pigments such as cyanidin-3-rhamnoside and pelargonidin-3-rhamnoside, quercetin glycosides such as quercitrin (quercetin-3-rhamnoside), isoquercitrin (quercetin-3-glucoside), and hyperoside (quercetin-3-galactoside), and astilbin. These polyphenols can be used in the form of a mixture comprising a plurality of polyphenol compounds or can be used alone in the form of an individual compound. These polyphenol compounds can be isolated or prepared to have higher concentrations and then used. Methods for isolating polyphenol compounds and methods for preparing the same with higher concentrations are not particularly limited. Examples of such methods include HPLC, synthetic absorbent chromatography, ion exchange chromatography, and gel filtration. In particular, synthetic absorbent chromatography is preferable.

As a processed product of an acerola fruit containing an acerola-fruit-derived polyphenol compound, a fraction containing such polyphenol compound fractionated by one of the above various types of chromatography from acerola fruit juice or the like can be appropriately used for the present invention. Particularly, a C18 column (hydrophobic column)-adsorbed fraction obtained from acerola fruit juice or the like is preferably used in the present invention. Examples of acerola-derived polyphenol-containing fractions such as a C 18 column-adsorbed fraction include eluates, concentrates thereof, and dried products thereof.

In general, polyphenol compounds are said to be highly insoluble in water. An acerola processed product to be used in the present invention contains polyphenol in a state such that it is easily soluble in water.

In general, ascorbic acid alone does not act as an antioxidant for lipids (see Experiment 2.7 in Example 2) because of its high water solubility. However, it is thought that in an acerola processed product, ascorbic acid functions as an antioxidant for lipids (see Experiment 2.9 in Example 2).

4. Modes for use of the antioxidant according to the present invention

As described above, (a) a pectin or a hydrolysate thereof, (b) a C18 column-adsorbed component, and (c) ascorbic acid, which are derived from acerola fruits, are useful as active ingredients of an antioxidant.

The antioxidant of the present invention preferably contains at least 1 type, more preferably 2 types, and most preferably all of the components (a), (b), and (c).

Such material prepared in Experiment 2.1 of Example 2 by removing glucose and fructose from acerola fruit juice and then powderizing the resultant contains components (a), (b), and (c) and has excellent antioxidative activities. The material is a preferred embodiment of the antioxidant (particularly, an antioxidant for lipids) of the present invention.

The antioxidant of the present invention is water-soluble. Hence, the antioxidant can be appropriately used as an antioxidant for lipids upon production of processed products such as fish, meat of livestock, and grains, which often form mixed systems of fats and oils and water-based components, salt-cured food products, and fat-and-oil-containing seasonings. In addition, an acerola powder prepared in Experiment 2.1 in Example 2 has antioxidative activities (for lipids) superior to those of $\alpha$-tocopherol (vitamin E), known as a lipid-soluble antioxidant, when they are compared under the same conditions (see Experiment 2.6 in Example 2).

The present invention further relates to a food product with enhanced oxidation stability containing the above-explained antioxidant and to a method for producing such food product. The antioxidant of the present invention can be used as an additive in production of food products containing lipids and particularly, lipids that are easily oxidized. Examples of such food products include processed products such as fish, meat of livestock, and grains, salt-cured food products, and seasonings (e.g., dressing) containing unsaturated fatty acid such as linoleic acid and linolenic acid. Methods for adding such additive are not particularly limited. For example, such additive can be added, upon production of processed food products, to a pickle solution, a seasoning, or a food material.

The antioxidant of the present invention can be used in the form not only of a food additive, but also of a food product or a pharmaceutical preparation that acts *in vivo* as an antioxidant. Furthermore, the antioxidant can be prepared in the form of an appropriate food product or a preparation in accordance with conventional techniques using an appropriate carrier, excipient, or the like, if necessary.

Such forms of food products may be beverages, solid food products, or semi-solid food products. Specific examples of beverages include fruit juice beverages, soft drink beverages, and alcoholic beverages. Alternatively, a beverage may also be in a form that is diluted with water or the like before ingestion. Examples of solid or semi-solid

food products include tablets, sugar-coated tablets, granules, powdery food products such as powdered beverages and powdered soup, block-shaped confectioneries such as biscuits, capsules, and gels. According to need, various additives that are generally used for preparation of food products can also be compounded. Examples of such additives include stabilizers, pH adjusters, sugars, sweeteners, fragrant materials, various vitamins, minerals, antioxidants, excipients, solubilizers, binders, lubricants, suspensions, moistening agents, film-forming substances, taste corrigents, flavor corrigents, colorants, and preservatives.

The antioxidant of the present invention can be prepared in the form of a preparation in accordance with conventional techniques. In such a case, carriers, excipients, binders, preservatives, oxidative stabilizers, disintegrators, lubricants, taste corrigents, or diluents can be adequately selected from among conventional substances. The form of such a preparation is not particularly limited, and it may be adequately selected according to need. The antioxidant of the present invention can be generally formulated into oral preparations including tablets, capsules, granules, fine granules, powders, pills, liquids, syrups, suspensions, emulsions, elixirs, and the like or parenteral preparations including injections, drops, suppositories, inhalants, transdermal absorbents, transmucosal absorbents, transnasal preparations, enteral preparations, adhesive preparations, ointments, and the like.

## 5 Modes for use of the skin-whitening agent for oral administration according to the present invention

As described in 2 above, the acerola fruit-derived pectin or the hydrolysate thereof is useful as an active ingredient of a skin-whitening agent for oral administration.

In the meantime, it is known that ascorbic acid contained richly in an acerola fruit can also be used as an active ingredient of a skin-whitening agent for oral administration.

The skin-whitening agent for oral administration of the present invention more preferably contains ascorbic acid in addition to the acerola fruit-derived pectin or a hydrolysate thereof.

The skin-whitening agent for oral administration containing a hydrolysate of the acerola fruit-derived pectin and ascorbic acid can be produced using a hydrolysate of the acerola fruit-derived pectin and ascorbic acid that are each independently prepared. Alternatively, the skin-whitening agent can also be produced by the following method. Specifically, such method comprises a step of hydrolyzing an acerola fruit or a processed product of an acerola fruit containing ascorbic acid and pulp. Specifically, in this step, pectin in the pulp is hydrolyzed, so that the amount of galacturonic acid will be 5% by weight or more with respect to ascorbic acid. Here, "pulp" refers to a fibrous component contained in a fruit. Such pulp generally contains a fiber backbone such as pectin or cellulose as a main constituent and has a structure such that the other components bind to the backbone in various patterns. As pectin is hydrolyzed, the amount of free galacturonic acid increases. Hence, the amount of galacturonic acid generated can be an indicator of the advancement of pectin hydrolysis. In the embodiment of the present invention, pectin hydrolysis is preferably performed so that the amount of galacturonic acid is 5% by weight or more and more preferably 10% by weight with respect to ascorbic acid. There is no particular upper limit of the degree of hydrolysis. A typical degree of such hydrolysis is that the amount of galacturonic acid is 20% by weight or less with respect to ascorbic acid. In addition, ascorbic acid can be quantified by a titration test in which the blue coloration of a 0.02% 2,6-dichloroindophenol aqueous solution is changed to become colorless because of the reduction effect of ascorbic acid. Galacturonic acid can be quantified by a 3,5-dimetylphenol method as described in Example 3.

The skin-whitening agent for oral administration according to the present invention is preferably an agent from which glucose and fructose have been substantially removed. When these sugars are substantially removed, the processed (powderized) product has lowered hygroscopicity. Hence, such agent is advantageous in that it enables lower amounts of an excipient or the like to be added and thus enables an increased proportion of active ingredients. Furthermore, the skin-whitening agent according to the present invention is orally ingested. Thus, it is also appropriate in that the agent has fewer calories as a result of the removal of sugars. The expression "glucose and fructose are "substantially removed," means that when a processed product is powderized, glucose and fructose are removed to a degree such that hygroscopicity is sufficiently lowered.

Glucose and fructose can be removed by fermentation using yeast, for example. In fermentation, glucose and fructose are converted to carbon dioxide gas and ethylalcohol and then removed. Such step of removing sugars by fermentation is advantageous because useful components of the skin-whitening agent, such as ascorbic acid, are not lost. A step of degrading pulp and a step of removing sugars can be performed in this order or vice versa, or the steps can be performed simultaneously.

The skin-whitening agent for oral administration of the present invention can be used solely or in combination with other components in the form of a food or beverage composition or a pharmaceutical composition. The skin-whitening agent is expected not only to contribute skin whitening, but also to exert an effect of preventing skin aging or preventing or treating skin cancer, for example.

Examples of forms of food or beverage compositions include beverages, solid food products, and semisolid food products. Such compositions may also be in the form of dietary supplements or food products for specified health

uses. Specific examples of beverages include fruit juice beverages; soft drink beverages, and alcoholic beverages. Alternatively, food or beverage compositions may be in forms that are diluted with water or the like before ingestion. Solid food products can be in various forms. Examples of such forms include tablets such as candies and troches, sugar-coated tablets, granules, powders such as powdered beverages and powdered soup, block-shaped confectioneries such as biscuits, capsules, and gels. Examples of the forms of semisolid food products include pastes such as jams and gum such as chewing gum. These food or beverage compositions can be compounded with, in addition to the skin-whitening agent of the present invention, various ingredients that are generally used as starting materials for food products, within a range such that the desired effects of the present invention are not deteriorated. Examples of such ingredients include water, alcohols, sweeteners, acidulants, colorants, preservatives, perfumes, and excipients. These ingredients can be used solely or in combinations of two or more.

The form of a pharmaceutical composition is not particularly limited, as long as the form is a preparation for oral administration. Examples of possible forms include powders, tablets, granules, fine granules, liquids, capsules, pills, troches, liquid formulations for internal use, suspensions, emulsions, syrups, and elixirs. These forms for preparations can be used solely or in combinations of two or more depending on the symptoms. Preparation into each of these preparation forms thereof is performed in accordance with conventional techniques. Carriers, excipients, binders, preservatives, oxidative stabilizers, disintegrators, lubricants, taste corrigents, diluents, or the like that are used in such a case can be adequately selected from among conventional substances. For example, when powderization is performed, flowability can be enhanced using shellfish calcium.

The dose of the skin-whitening agent for oral administration according to the present invention can be appropriately selected according to symptoms and purposes. When the agent is used as a pharmaceutical preparation for suppressing pigmentation due to flecks or sunburn, it is preferable to ingest the skin-whitening agent for oral administration according to the present invention so that the ingestion dose of ascorbic acid ranges from 300 mg to 600 mg per day.

Example 1

Experiment 1.1. Collection of anti-oxidation pectin from fruit juice

Experiment 1.1.1. Collection of pectin from peach juice grapefruit juice, lemon juice, and grape juice

Pericarps and seeds were removed using a knife from fruits to be used as specimens, so as to obtain edible portions only. Next, the edible portions were crushed using a juicer. Crushed products were centrifuged under conditions of 4950 rpm and 20˚C for 60 minutes. Each supernatant was filtered using a 0.2 μm filter, thereby collecting a clear fruit juice solution. Ethanol was added to the fruit juice solution in an amount 3 times greater than the weight of the solution. The mixture was then agitated, allowed to stand at room temperature overnight, and then centrifuged at 4950 rpm for 20 minutes at 20˚C, thereby collecting a precipitate. The precipitate was pectin derived from the fruit juice specimen. Moreover, to increase the purification degree of pectin, the precipitate was dissolved in purified water in an amount 10 or more times greater than that of the precipitate. Ethanol was added to the solution in an amount twice that of the total weight. The solution was agitated, allowed to stand at room temperature for 30 minutes, and then centrifuged at 4950 rpm for 20 minutes at 20˚C, thereby collecting a precipitate. The precipitate was dried via freeze-drying, so that pectin derived from the fruit juice specimen was collected. Amounts of specimens used and amounts of pectin collected in each experiment are listed in Table 1.

Table 1

| Specimen | Peach juice | Grapefruit juice | Lemon juice | Grape juice |
|---|---|---|---|---|
| Weight of fruit used | 5264 g | 6383 g | 2992 g | 5294 g |
| Weight of fruit juice after filtration | 2942 g | 3468 g | 1232 g | 2886 g |
| Dry pectin weight | 9.93 g | 3.35 g | 1.2 g | 12.12 g |

Experiment 1.1.2. Collection of pectin from green acerola juice

Seeds were removed from immature green acerola fruits (green to yellow fruits before maturation, when they develop red coloration) using a pulp finisher, thereby preparing puree. 18337 g of the puree was centrifuged at 4200 rpm for 45 minutes at 20˚C and then the supernatant was collected. The supernatant was filtered using a 0.2 μm filter, so that a 13632 g of a clear fruit juice solution was collected. Since the amount of the solution was excessive, the solution was concentrated using a vacuum distillation apparatus. Thus, 4258 g of the solution was collected. Ethanol was added

to the fruit juice solution in an amount 3 times greater than the weight of the solution. The mixture was then agitated and then allowed to stand overnight at room temperature. The solid content was collected using stainless mesh. To increase the purification degree of pectin, the precipitate was dissolved in purified water, ethanol was added to the solution in an amount twice the total weight, and then the mixture was agitated. The resultant was allowed to stand at room temperature for 30 minutes and then centrifuged at 4200 rpm for 30 minutes at 20˚C, thereby collecting a precipitate. To further increase the purification degree of pectin, the precipitate was dissolved in purified water, ethanol was added to the solution in an amount 3 times greater than the total weight, and then the mixture was agitated. The resultant was allowed to stand for 30 minutes at room temperature and was then centrifuged at 4200 rpm for 30 minutes at 20˚C, thereby collecting a precipitate. Ethanol precipitation was performed 3 times in total. The precipitate was dried by freeze-drying, thereby collecting 19.7 g of a pectin derived from green acerola juice.

Experiment 1.1.3. Evaluation of antioxidative potency

5 types of pectin derived from fruit juice were evaluated by a test concerning suppression of β-carotene discoloration described in Test method 1 and a DPPH radical scavenging activity test described in Test method 2. Table 2 shows the results. One result was that all types of pectin exerted an effect of suppressing β-carotene discoloration, which is an antioxidative effect. However, DPPH radical scavenging activity was strongly observed only in the pectin derived from acerola juice. Such pectin derived from acerola juice has a DPPH radical scavenging effect, so that it can be expected to have antioxidative potency against many objects. As described above, it was revealed that antioxidative pectins can be produced from acerola juice without losing their anti-oxidation activity by performing an ethanol precipitation method.

Table 2

| Antioxidative activities of pectins derived from fruit juice | | |
|---|---|---|
| Pectin type | Suppression ratio (%) of β-carotene discoloration in a sample with a concentration of 0.025% | DPPH radical scavenging ratio (%) in a sample with a concentration of 0.1 % |
| Peach juice | 14% | 0.8% |
| Grapefruit juice | 57% | 0% |
| Lemon juice | 77% | 0% |
| Grape juice | 82% | 6.6% |
| Green acerola juice | 88% | 83.2% |

Experiment 1.2. Collection of antioxidative pectin from pulp

Experiment 1.2.1. Collection of pectin from peach pulp, grapefruit pulp, lemon pulp, and grape pulp

Pericarps and seeds were removed using a knife from fruits to be used as specimens, so as to obtain edible portions only. Next, the edible portions were crushed using a juicer. Crushed products were centrifuged under conditions of 4950 rpm and 20˚C for 60 minutes, thereby collecting a precipitate. The precipitate was pulp derived from the fruits. Purified water was added to the pulp in an amount 3.5 to 8 times greater than that of the pulp, so as to enable agitation. The resultant was agitated and then concentrated hydrochloric acid was added to adjust the resultant at pH 2.0. The resultant was heated at 80˚C for 2 hours while agitating the resultant, followed by overnight agitation at room temperature. The solution was centrifuged under conditions of 4950 rpm and 20˚C for 60 minutes, thereby collecting a supernatant. The supernatant was filtered using a 0.2 μm filter so that a clear pectin extract was collected. Ethanol was added to the extract in an amount twice the weight of the extract. The mixture was then agitated, allowed to stand at room temperature overnight, and then centrifuged at 4950 rpm for 20 minutes at 20˚C, thereby collecting a precipitate. The precipitate was pectin derived from the pulp specimen. Moreover, to increase the purification degree of pectin, the precipitate was dissolved in purified water in an amount 10 or more times greater than that of the precipitate. Ethanol was added in an amount twice the total weight and then the resultant was agitated. The resultant was allowed to stand at room temperature for 30 minutes, and then centrifuged at 4950 rpm for 20 minutes at 20˚C, thereby collecting a precipitate. The precipitate was dried via freeze-drying, so that pectin derived from the pulp specimen was collected. Amounts of specimens used and amounts of pectin collected in each experiment are listed in Table 3.

Table 3

| Specimen | Peach pulp | Grapefruit pulp | Lemon pulp | Grape pulp |
|---|---|---|---|---|
| Weight of fruit used herein | 5264 g | 6383 g | 2992 g | 5294 g |
| Pulp weight | 1302 g | 1644 g | 826 g | 581 g |
| Dry pectin weight | 10.92 g | 40.62 g | 17 g | 2.87 g |

Experiment 1.2.2 Collection of pectin from green acerola pulp

Seeds were removed from immature green acerola fruits (green to yellow fruits before maturation, when they develop red coloration) using a pulp finisher, thereby preparing puree. 18337 g of the puree was centrifuged at 4200 rpm for 45 minutes at 20˚C and then 3386 g of a precipitate was collected. The precipitate was acerola pulp. Purified water was added to the pulp in an amount 5 times greater than that of the pulp, so as to enable agitation. The mixture was then agitated. Concentrated hydrochloric acid was added to adjust the resultant at pH 2.0. The resultant was heated at 80˚C for 2 hours while agitating the resultant, followed by overnight agitation at room temperature. The solution was centrifuged under conditions of 4200 rpm and 20˚C for 30 minutes, thereby collecting a supernatant. The supernatant was filtered using a 0.2 μm filter so that a clear pectin extract was collected. Ethanol was added to the solution in an amount twice the weight of the solution and then the mixture was agitated. The mixture was allowed to stand overnight at room temperature and then the solid content was collected using stainless mesh. To increase the purification degree of pectin, the precipitate was dissolved in purified water, ethanol was added to the solution in an amount twice the total weight, and then the mixture was agitated. The mixture was allowed to stand at room temperature for 30 minutes and then centrifuged at 4200 rpm for 30 minutes at 20˚C, thereby collecting a precipitate. To further increase the purification degree of pectin, the precipitate was dissolved in purified water, ethanol was added to the solution in an amount twice the total weight, and then the mixture was agitated. The mixture was allowed to stand for 30 minutes at room temperature and then centrifuged at 4200 rpm for 30 minutes at 20˚C, thereby collecting the precipitate. Ethanol precipitation was performed 3 times in total. The precipitate was dried by freeze-drying, thereby collecting 38.63 g of a pectin derived from green acerola pulp.

Experiment 1.2.3. Evaluation of antioxidative potency

The antioxidative potency of 5 types of pectin derived from pulp was evaluated by a test concerning the suppression of β-carotene discoloration described in Test method 1 and a DPPH radical scavenging activity test described in Test method 2. Table 4 shows the results. As a result, all types of pectin exerted the effect of suppressing β-carotene discoloration, which is one of antioxidative effects. However, DPPH radical scavenging activity was strongly observed only in the pectin derived from acerola pulp. Such a pectin derived from acerola pulp has a DPPH radical scavenging effect, so that it can be expected that the pectin has antioxidative potency against oxidation of many objects. As described above, it was revealed that such antioxidative pectins can be produced from acerola pulp without losing their anti-oxidation activity by performing heat treatment using acid and an ethanol precipitation method.

Table 4

| Antioxidative activities of pulp-derived pectin | | |
|---|---|---|
| Pectin type | Suppression ratio (%) of β-carotene discoloration in a sample with a concentration of 0.025% | DPPH radical scavenging ratio (%) in a sample with a concentration of 0.1 % |
| Peach pulp | 31% | 0.4% |
| Grapefruit pulp | 35% | 0% |
| Lemon pulp | 32% | 0% |
| Grape pulp | 55% | 0.4% |
| Green acerola fruit pulp | 80% | 34.6% |

Experiment 1.3. Evaluation of antioxidative pectin derived from acerola fruits differing in the grade of maturity

The antioxidative activity of a green-fruit-juice-derived pectin and a green-fruit-pulp-derived pectin (obtained from green acerola fruits, as prepared in 1.1 and 1.2 above) was evaluated by a DPPH radical 50% scavenging activity test (see Test method 2). Furthermore, the antioxidative activity of pectins prepared by the following method from mature acerola fruits that had matured sufficiently to develop red coloration was evaluated by the same test.

Experiment 1.3.1. Collection of pectin from juice of mature acerola

Seeds were removed from mature acerola fruits that had sufficiently matured to develop red coloration using a pulp finisher, thereby preparing puree. 21861 g of the puree was centrifuged at 4200 rpm for 45 minutes at 20˚C and then a supernatant was collected. The supernatant was filtered using a 0.2 μm filter, so that 15324 g of a clear fruit juice solution was collected. Since the amount of the solution was excessive, the solution was concentrated using a vacuum distillation apparatus. Thus, 5618 g of the concentrated solution was collected. Ethanol was added to the fruit juice solution in an amount 3 times greater than the weight of the solution. The mixture was then agitated and then allowed to stand overnight at room temperature. The solid content was collected using stainless mesh. To increase the purification degree of pectin, the precipitate was dissolved in purified water, ethanol was added to the solution in an amount twice the total weight, and then the mixture was agitated. The mixture was allowed to stand at room temperature for 30 minutes and then centrifuged at 4200 rpm for 30 minutes at 20˚C, thereby collecting a precipitate. To further increase the purification degree of pectin, the precipitate was dissolved in purified water, ethanol was added to the solution in an amount 3 times greater than the total weight, and then the mixture was agitated. The mixture was allowed to stand for 30 minutes at room temperature and then centrifuged at 4200 rpm for 30 minutes at 20˚C, thereby collecting a precipitate. Ethanol precipitation was performed 3 times in total. The precipitate was dried by freeze-drying, thereby collecting 39 g of a pectin derived from juice of mature acerola.

Experiment 1.3.2. Collection of pectin from pulp of mature acerola fruit

Seeds were removed from mature acerola fruits that had sufficiently matured to develop red coloration using a pulp finisher, thereby preparing puree. 21861 g of the puree was centrifuged at 4200 rpm for 45 minutes at 20˚C and then 5022 g of the precipitate was collected. The precipitate was acerola pulp. Purified water was added to the pulp in an amount 5 times greater than that of the pulp, so as to enable agitation. The mixture was then agitated. Concentrated hydrochloric acid was added to adjust the resultant to pH 2.0. The resultant was heated at 80˚C for 2 hours while agitating it, followed by overnight agitation at room temperature. The solution was centrifuged under conditions of 4200 rpm and 20˚C for 30 minutes, thereby collecting a supernatant. The supernatant was filtered using a 0.2 μm filter, so that a clear pectin extract was collected. Since the amount of the solution was excessive, the solution was concentrated by vacuum distillation. Thus, 9159 g of the solution was collected. Ethanol was added to the solution in an amount twice the weight of the solution. The mixture was then agitated and then allowed to stand overnight at room temperature. The solid content was collected using stainless mesh. To increase the purification degree of pectin, the precipitate was dissolved in purified water, ethanol was added to the solution in an amount twice the total weight, and then the mixture was agitated. The mixture was allowed to stand at room temperature for 30 minutes and then centrifuged at 4200 rpm for 30 minutes at 20˚C, thereby collecting a precipitate. To further increase the purification degree of pectin, the precipitate was dissolved in purified water, ethanol was added to the solution in an amount twice the total weight, and then the mixture was agitated. The mixture was allowed to stand for 30 minutes at room temperature and then centrifuged at 4200 rpm for 30 minutes at 20˚C, thereby collecting a precipitate. Ethanol precipitation was performed 3 times in total. The precipitate was dried by freeze-drying, thereby collecting 26 g of pectin derived from pulp of mature acerola fruit.

Experiment 1.3.3. Evaluation of antioxidative potency

The antioxidative activities of 4 types of acerola-derived pectin were evaluated by a DPPH radical 50% scavenging activity test. Table 5 shows the results. Each test result is shown with the concentration of a sample that is required for scavenging 50% of the DPPH radicals. It is indicated that the lower the concentration of a sample, the stronger the antioxidative potency of the relevant pectin. As a result, sufficient antioxidative potency was observed in all types of pectin. However, antioxidative potency was stronger in pectins derived from green fruits than in pectins derived from mature fruits. Therefore, it was concluded that a green acerola fruit is more appropriate as a raw material for extraction of an antioxidative pectin.

Table 5

| Antioxidative potency of acerola fruit pectins with different grades of maturity | |
|---|---|
| Pectin type | Pectin concentration required for scavenging 50% of the DPPH radicals |
| Pectin from green fruit juice | 0.05% |
| Pectin from green fruit pulp | 0.14% |
| Pectin from mature fruit juice | 0.23% |
| Pectin from mature fruit pulp | 0.25% |

Experiment 1.4. Method for producing antioxidative pectin via pectinase treatment

Acerola puree was prepared from green acerola fruits using a pulp finisher (an apparatus for separating fruit juice and pulp from seeds) and then cryopreserved. The acerola puree was thawed. 19899 g of the thawed acerola puree was allowed to return to room temperature. 0.1 % (W/W) pectinase (pectinase A "Amano," Amano Enzyme Inc.) was added to the resultant, followed by 2 hours of agitation at 50˚C. Agitation was continued until the next day at room temperature. Enzyme-treated puree was centrifuged (4950 rpm and 30 minutes), thereby collecting a supernatant. To remove insoluble components, the supernatant was filtered for several times, followed by final filtration with a 0.2 μm filter. Thus, 17420 g of fruit juice was collected via filtration. The solution was then concentrated using a vacuum distillation and concentration apparatus, so that 2981 g of a concentrated solution was collected. Ethanol was added to the concentrated solution in an amount 4 times greater than the weight of the solution. The solution was allowed to stand at room temperature for 1 or more days and then centrifuged (4950 rpm and 5 minutes), thereby collecting 600 g (containing water) of an ethanol precipitate ($1^{st}$ time). Purified water was added to the ethanol precipitate in an amount 20 times greater than the weight of the precipitate, so that the precipitate was dissolved. Ethanol was further added to the precipitate in an amount twice the weight of the precipitate and then the resultant was refrigerated for 1 or more days. The solution was filtered using a glass fiber filter. 544 g of a $2^{nd}$ ethanol precipitate (containing water) that had remained on the filter paper was collected. Purified water was added to the precipitate and then the precipitate was dissolved. Ethanol was added to the solution in an amount equivalent with respect to the solution and then the resultant was refrigerated for 1 or more days. The solution was filtered using a glass fiber filter. 434 g of a $3^{rd}$ ethanol precipitate (containing water) that had remained on the filter paper was collected. The precipitate was frozen at -80˚C. After the precipitate was completely frozen, freeze-drying was performed. Thus, 78 g of an acerola-derived pectin powder was collected.

The antioxidative potency of the thus obtained acerola-derived pectin was compared with that of the green-fruit-juice-derived pectin and the green-fruit-pulp-derived pectin (obtained from green acerola fruits as prepared in Experiments 1.1 and 1.2).

The antioxidative potency of each pectin was evaluated by a DPPH radical 50% scavenging activity test (Test method 2). Table 6 shows the results.

Table 6

| Collection ratio (%) and antioxidative activities of antioxidative pectins | | |
|---|---|---|
| Pectin type | Collection ratio (%) based on puree weight | Pectin concentration required for scavenging 50% of the DPPH radicals |
| Pectin from green fruit juice | 0.11%* | 0.05% |
| Pectin from green fruit pulp | 0.21%* | 0.14% |
| Pectin from green fruit treated with pectinase | 0.39% | 0.067% |
| * The pectin from green fruit juice and the pectin from green fruit pulp were prepared from the same puree. | | |

When the puree was separated into fruit juice and pulp and the pectin was collected from each thereof, the total pectin collection ratio (%) was 0.32% (= 0.11 % + 0.21%). In the meantime, it was demonstrated that a higher collection ratio (%) was obtained such that the collection ratio (%) of the pectin treated with pectinase was 0.39% in this experiment. Furthermore, the pectin treated with pectinase (obtained in this experiment) also had sufficient antioxidative activities.

Experiment 1.5. Method for producing antioxidative pectin solution by ultrafiltration method

Acerola puree was prepared from green acerola fruits using a pulp finisher (an apparatus for separating fruit juice and pulp from seeds) and then cryopreserved. The acerola puree was thawed. 55000 g of the thawed acerola puree was allowed to return to room temperature. 0.1 % (W/W) pectinase (pectinase A "Amano," Amano Enzyme Inc.) was added to the resultant, followed by 2 hours of agitation at 50˚C. Agitation was continued until the next day at room temperature. Enzyme-treated puree was centrifuged (4950 rpm and 30 minutes), thereby collecting a supernatant. To remove insoluble components, the supernatant was filtered several times, followed by final filtration with a 0.2 μm filter. Thus, 47130 g of fruit juice was collected. The collected product was subjected to ultrafiltration using an ultrafiltration membrane (Hydrosart 10 K, SARTORIUS K.K.) with a molecular weight cut-off of 10,000. Thus, 8730 g of a concentrated solution was collected.

The concentration of the solid content of the concentrated solution was 11.77% (concentration of a residue after evaporation). The concentrated solution was subjected to ethanol precipitation, so that an anti-oxidation pectin was collected. 16.87 g of the anti-oxidation pectin was collected from 500g of the concentrated solution. It was confirmed that the anti-oxidation pectin content in the concentrated solution was 3.374%. Based on such concentration, the weight of the pectin in the concentrated solution was calculated to be 294.2 g. The percentage of the pectin collected was calculated to be 0.53% based on the weight of the puree. It was revealed that such collection ratio (%) was better than that in the case of the production method used in Experiment 1.4. Moreover, the anti-oxidation pectin content as a percentage of the total solid content in the anti-oxidation pectin solution was 28.7% in this experiment. In the case of the ultrafiltration method, such content can be regulated by varying the ratio of the amount of stock solution to the amount of the final concentrated solution.

Experiment 1.6. Method for producing acerola powder from solution prepared in Experiment 1.5

600 g of the concentrated solution of the acerola-derived antioxidative pectin prepared in Experiment 1.5 was frozen and then the resultant was freeze-dried by the freeze-drying method. As a result, 63 g of a powder was collected. The concentration of the solid content in the concentrated solution was 11.77%. Hence, theoretically the solid content was 70.62 g and the collection ratio (%) was 89.2%. The concentration of ascorbic acid in the powder was 21.06%, as measured by an indophenol method. Based on the antioxidative pectin content in the concentrated solution, the antioxidative pectin concentration in the powder was calculated to be 28.7%. The thus obtained powder can be finely pulverized under good conditions after freeze-drying, exerts no significant hygroscopicity, and is excellent in flowability. It is considered that the antioxidative pectin acts as an excipient.

Experiment 1.7. Examination (1) of polyphenol in acerola-derived antioxidative pectin

10 g of the acerola-derived antioxidative pectin powder prepared in Experiment 1.4 was dissolved in 500 mL of a 2N sodium hydroxide aqueous solution, followed by 16 hours of hydrolysis at 40˚C using a thermostatic vibrator. To further increase the solubility of acidic polyphenol, concentrated hydrochloric acid was added to adjust the solution to pH 2.0. To remove free sugar content derived from the pectin, ethanol was added to the solution in an amount 4 times greater than the weight of the solution. The solution was allowed to stand for 1 or more days in a refrigerating area so that ethanol precipitation was performed. The procedure was performed under the same conditions as applied upon pectin collection, so that only a product hydrolyzed by alkali would remain unprecipitated and be present in a free state in the supernatant. The solution to which ethanol had been added was centrifuged (4200 rpm and 30 minutes), so as to cause the solid content to be precipitated and to collect a supernatant. The supernatant was filtered using a 0.45 μm filter, thereby completely removing the solid content. The filtered solution was concentrated by vacuum distillation, so that 250 mL of a concentrated solution was collected. Each of two C18 columns (Sep-Pak Vac 35 cc (10 g) C18 Cartridges, Waters Corporation), to which polyphenol can adsorb, was loaded with half the amount of the concentrated solution. After non-adsorbed components were washed with purified water, adsorbed components were eluted using a 25% methanol aqueous solution. The eluate was dried and then solidified using a vacuum distillation apparatus. The resultant was dissolved in 5 mL of 100% methanol, thereby preparing a pectin extract sample. The components in the sample were analyzed using an analytical HPLC column (4.6 mm × 250 mm, ODS-3, GL Sciences Inc.) and a linear gradient of a 0.01 N hydrochloric acid aqueous solution and methanol. Fig. 1 shows the results.

The presence of the major component at 22.4 minutes was confirmed by this analysis. Next, 4.5 mL of the pectin extract sample was subjected to preparative isolation using a preparative column. ODS-3 (20 mm × 250 mm, GL Sciences Inc.) was used as such a preparative column. Preparative isolation was performed at a flow rate of 12 mL/minute using a gradient of a 0.01N hydrochloric acid aqueous solution and methanol. Fig. 2 shows the results of preparative isolation.

The peak at 33.46 minutes in Fig. 2 was collected, dried and solidified using a vacuum distillation apparatus,

dissolved in purified water, and then allowed to stand overnight in a refrigeration area. The thus generated deposit was centrifuged, thereby collecting 28 mg of the deposit. Furthermore, the deposit was dissolved in methanol. Then the components of the sample were analyzed using an HPLC system provided with a photodiode array detector, an analytical HPLC column (4.6 mm × 250 mm, ODS-3, GL Sciences Inc.), and a linear gradient of 0.05% TFA aqueous solution and methanol to which 0.05% TFA had been added. Fig. 3 shows the results. As a result, it was confirmed that the above deposit was the major component of the pectin extract sample.

Furthermore, the spectrum data (Fig. 4A) of the peak at 22.4 minutes shown in Fig. 3 was confirmed. Thus, it was revealed that the data was almost in agreement with the spectrum data (Fig. 4B) for Aceronidin (reference example 1). In addition, spectrum data shown in Fig. 4A and B were collected using a system comprising an HPLC apparatus (the apparatus used herein was LC-2010CHT (Shimadzu Corporation)) with a photodiode array detector (PDA; the detector used herein was an SPD-M20A (Shimadzu Corporation)) included therewith.

It was revealed that based on the above HPLC elution time and spectrum data, polyphenol contained in acerola-derived antioxidative pectin was likely to be Aceronidin.

<u>Experiment 1.8. Examination (2) of polyphenol in acerola-derived antioxidative pectin</u>

The structure of polyphenol extracted from the acerola-derived antioxidative pectin was further analyzed by ESI-MS measurement and NMR measurement. When the molecular weight was analyzed using an LCT mass spectrometer (Micromass), m/z 473 (sodium adduct ion $(M+Na)^+$) was observed in a manner similar to the case of Aceronidin. Hence, it was confirmed that the polyphenol was identical to Aceronidin with a molecular weight of 450. Furthermore, as a result of $^1H$ NMR measurement, peaks (lower case in Fig. 5) derived from the solvent were observed in the vicinity of 3.3 ppm and 4.8 ppm. It was revealed that these peaks agreed well with the peaks for Aceronidin (upper case in Fig. 5).

As described above, it was confirmed that the polyphenol extracted from the acerola-derived antioxidative pectin was Aceronidin.

<u>Experiment 1.9. Preparation of C18 column-bound pectin derived from acerola</u>

50 g of the acerola-derived pectin prepared in Experiment 1.4 was dissolved in 5000 mL of a 1% sodium hexametaphosphate aqueous solution. The resultant was filtered using a 0.2 μm filter. Twenty C18 columns (Sep-Pak Vac 35 cc (10 g) C18 Cartridges, Waters Corporation) were loaded with the filtrate. Non-adsorbed components were washed with purified water and then adsorbed components were eluted using a 50% methanol aqueous solution. The eluate was dried and solidified using a vacuum distillation apparatus. The resultant was dissolved in purified water and then insoluble matter was removed using a 0.2 μm filter. The resultant was then frozen and freeze-dried, thereby obtaining 6.24 g of a freeze-dried powder.

The acerola-derived pectin (sample 1) prepared in Experiment 1.4 and the acerola-derived C 18 column-bound pectin (sample 2) prepared in this experiment were each analyzed in terms of polyphenol concentration, DPPH radical scavenging activity, tyrosinase-inhibiting activity, and sugar composition. Polyphenol concentration was measured by the Folin-Denis method using catechin as a standard substance. DPPH radical scavenging activity was analyzed by Test method 2, a tyrosinase-inhibiting activity test was conducted by Test method 3, and sugar composition was analyzed by Test method 4.

Table 7

| Content and activity of polyphenol, an element of acerola-derived antioxidative pectin | | | | | |
|---|---|---|---|---|---|
| Sample No. | Fraction | Content based on pectin treated with pectinase | Polyphenol content (determined in terms of catechin) | 50% radical scavenging activity concentration | Tyrosinase-inhibiting activity |
| 1 | Pectin treated with pectinase | 100% | 5.4% | 670 ppm | 10.8% |
| 2 | C18-bound pectin | 12.5% | 25.3% | 125 ppm | 33.8% |
| Reference | Aceronidin | - | 59.2% | 80 ppm | 3.1% |
| Reference | α-tocopherol | - | - | 127 ppm | - |

Table 8

| Sugar composition (sugar composition percentage (%)) | | |
|---|---|---|
| Sugar type | Sample 1 (pectin treated with pectinase) | Sample 2 (C18-bound pectin) |
| Neutral sugar | | |
| Rhamnose | 8.85% | 5.21 % |
| Mannose | 1.63% | 3.15 % |
| Arabinose | 17.28 % | 25.60 % |
| Galactose | 14.79 % | 8.60 % |
| Xylose | 3.56 % | 2.45 % |
| Glucose | 8.71% | 47.47% |
| Acidic sugar | | |
| Galacturonic acid | 44.93 % | 7.01 % |
| Glucuronic acid | 0.25% | 0.51% |

Based on the results in Tables 7 and 8, it is considered that the acerola-derived antioxidative pectin is composed of a main chain mainly consisting of polygalacturonic acid and side chains mainly consisting of neutral sugar. Based on the results of analyzing C18 resin-bound components, it is considered that polyphenol may be mainly present in the side chains. Sample 2 with a high polyphenol content had also strong antioxidative activities and a strong skin-whitening effect. Hence, such antioxidative activities and skin-whitening effect were thought to be due to the effects of Aceronidin. However, almost no skin-whitening effect (effect of inhibiting tyrosinase) was observed in Aceronidin. Therefore, it was revealed that the skin-whitening effect represents unique activity of the acerola-derived antioxidative pectin.

Test method 1.1. Test concerning suppression of β-carotene discoloration

This method is a method for determining how a test substance suppresses the effect of the peroxide of linoleic acid to cause β-carotene discoloration. In this experiment, 0.48 mL of a 10% (W/V) linoleic acid/chloroform solution, 1.2 mL of a 0.01% (W/V) β-carotene/chloroform solution, and 2.4 mL of a 20% (W/V) tween 40/chloroform solution were put into a 200-mL Erlenmeyer flask and then mixed. The mixture was sprayed with a nitrogen gas to remove chloroform. 108 mL of purified water and 12 mL of 0.2 M sodium phosphate buffer (pH 6.8) were then mixed. The thus prepared solution was used as a linoleic acid solution. 0.1 mL of a specimen diluted to an appropriate concentration was added to 4.9 mL of the linoleic acid solution and the resultant was then mixed. Absorbance was measured at 470 nm and the measured value was designated the value at 0 minutes. Immediately after measurement, the resultant was heated in a thermostatic bath at 50˚C. 120 minutes later, absorbance was measured at 470 nm. The measured value was designated the value at 120 minutes. A blank value was measured using purified water (with which a specimen was diluted) instead of a specimen. The β-carotene discoloration suppression ratio (%) was calculated by the following equation.

β-carotene discoloration suppression ratio (%)

=100 - (1 - (specimen value at 0 minutes - specimen value at 120 minutes) / (blank value at 0 minutes - blank value at 120 minutes)) × 100

Test method 1.2. DPPH radical scavenging activity test

Antioxidative activities were evaluated using an ethanol solution of diphenyl-p-picrylhydradil (DPPH), which is a stable radical. 1200 μl of ethanol and 400 μl of a specimen (adjusted to any concentration) were mixed with 1600 μl of a 250 mM acetate buffer (pH = 5.5), followed by preincubation at 30˚C for 5 minutes. 800 μl of a 500 μM DPPH/ethanol solution was added to the solution and mixed therewith, and the resultant was then allowed to stand at 30˚C for 30 minutes. Absorbance was measured at 517 nm. A similar procedure was performed also for α-tocopherol and then the result was used as a positive control. The control used herein was prepared by performing a similar procedure using

a solvent instead of a sample solution. Radical scavenging ratio was calculated by the following equation using the thus measured absorbances.

$$\text{Scavenging ratio (\%)} = (1 - [\text{absorbance of sample}] / [\text{absorbance of control}]) \times 100$$

The above measurement for finding scavenging ratios was performed by varying stepwise the concentration of a sample in a solution. The concentration of a sample solution leading to a 50% DPPH radical scavenging ratio was found and designated the concentration required for scavenging 50% of the DPPH radicals. Thus, it can be said that the lower the numerical value, the higher the radical scavenging ability.

Test method 1.3. Effect of inhibiting tyrosinase (skin-whitening activity)

A test concerning activity of inhibiting tyrosinase, which is an enzyme that generates a melanin pigment, was conducted by the following procedure.

(1) *4 mL of an L-DOPA aqueous solution, **4 mL each of samples diluted at different concentrations, and 2 mL of a 0.2 M phosphate buffer (pH 6.8) were mixed. The mixture was heated in a thermostatic bath at 37˚C. The thus heated mixture was designated a sample mixture.

*L-DOPA aqueous solution: L-β-(3,4-dihydroxyphenyl)alanine (Wako Pure Chemical Industries, Ltd.) dissolved in a 0.2 M phosphate buffer (pH 6.8) at a concentration of 3 mM
**Samples were all diluted and dissolved using a 0.2 M phosphate buffer.

(2) 2.5 mL of the sample mixture and *0.5 mL of the tyrosinase solution heated at 37˚C were put into a cell of an absorption spectrometer, followed by measurement at 475 nm. Measurement was performed using kinetics software. Changes in absorbance were automatically measured for over 20 seconds from the start to the end at intervals of 0.1 second. The rise velocity of absorbance between 4 seconds and 10 seconds after the start of measurement was calculated.

*Tyrosinase solution: Solution obtained by dissolving tyrosinase (SIGMA) derived from a mushroom in a 0.2 M phosphate buffer (pH 6.8) at a concentration of 300 units/mL and then conducting filtering using a 0.2 μm filter

(3) To obtain a blank value, measurement was performed using a 0.2 M phosphate buffer instead of a sample and then tyrosinase-inhibiting activity was calculated by the equation below. Furthermore, a substance that could not be dissolved in a phosphate buffer was dissolved in dimethyl sulfoxide (DMSO), diluted with a phosphate buffer, and then measured. Calculation was performed using the measurement results of a blank containing DMSO at the same concentration.

$$\text{Tyrosinase activity inhibition ratio (\%)} = 100 - ((\text{absorbance rise velocity of sample}) / (\text{absorbance rise velocity of blank})) \times 100$$

Analysis was performed as follows. 2 N trifluoroacetic acid was added to each sample and then hydrolysis was performed at 100˚C for 6 hours. The thus hydrolyzed neutral sugar and uronic acid were collected using purified water and then the HPLC method was performed. Analytical conditions were as follows.

Neutral sugar analytical conditions

Detector: Spectrophotofluorometer
Column: TSK-gel Suger AXG 4.6 mm × 150 mm (TOSOH Corporation)
Mobile phase: 0.5 M potassium borate buffer pH 8.7
Mobile phase flow rate: 0.4 mL/min

Post-column labeling: Reaction reagent 1% arginine/3% boric acid
Reaction reagent flow rate: 0.5 mL/min
Reaction temperature: 150˚C
Detection wavelength: EX.320 nm, EM.430 nm

Uronic acid analytical conditions

Detector: Spectrophotofluorometer
Column: Shimpack ISA07 4.6 mm × 250 mm (Shimadzu Corporation)
Mobile phase: 1.0 M potassium borate buffer pH 8.7
Mobile phase flow rate: 0.8 mL/min
Post-column labeling: Reaction reagent 1 % arginine/3% boric acid
Reaction reagent flow rate: 0.8 mL/min
Reaction temperature: 150˚C
Detection wavelength: EX.320 nm, EM.430 nm

A calibration curve of neutral sugar and uronic acid was prepared and then the sugar content of a sample was measured based on the curve. In this analysis, not all sugar chains are able to be hydrolyzed and partial sugars may be hydrolyzed and remain undetected.

Reference example: Isolation and identification of Aceronidin

(1) Isolation of Aceronidin

A raw material used for preparation of Aceronidin was an acerola powder (Nichirei Corporation, Nichirei-acerola powder VC30) prepared by fermenting concentrated acerola juice using yeast, removing glucose and fructose, dissolving as excipients dietary fiber and calcium oxide, and then powderizying the product.

400 g of acerola powder was dissolved in purified water, thereby preparing a 20% (W/W) aqueous solution (2000 g). Ethyl acetate was added to the aqueous solution in a volume half of that of the solution (on a volume basis) and then the solution was agitated. Liquid-liquid fractionation was performed using a separatory funnel so that an aqueous layer fraction was collected. Butanol was added to the aqueous layer fraction in a volume half of that of the fraction (on a volume basis), and then the resultant was agitated. Liquid-liquid fractionation was performed using a separatory funnel, so that a butanol layer fraction was collected. Purified water was added in an appropriate amount to the butanol layer fraction. Vacuum distillation was then performed to dry and solidify the resultant, thereby collecting 24 g of solid content.

The above solid content was dissolved in 50 mL of purified water. The resultant was subjected to partial purification using C18 columns (Sep-Pak Vac 35 cc (10 g) C18 cartridges, Waters Corporation). Specifically, the column was loaded with the sample and then washed with purified water and a 10% methanol aqueous solution, followed by elution with a 20% methanol aqueous solution. Thus the eluted fraction was collected. The fraction was evaporated to dryness using a vacuum distillation apparatus, thereby collecting 0.8 g of solid content.

The solid content was dissolved in 10 mL of a 20% methanol aqueous solution. The specimen was subjected to high purity purification by high performance, liquid chromatography. As a preparative column, Inertsil ODS-3 5 μm 4.6 × 250 mm (GL-science) was used. Preparative isolation was performed by loading the column with 0.5 mL of a specimen per preparative isolation, washing the column with a 10% methanol aqueous solution, eluting with a 10% to 50% methanol concentration gradient, and then collecting the peak containing polyphenol glycoside. This preparative isolation was repeated for 20 times.

The polyphenol-glycoside-containing methanol aqueous solution purified by the above method was dried and solidified using a vacuum distillation apparatus. The resultant was suspended in purified water. Insoluble matter was separated by centrifugation from the supernatant and then collected. The insoluble matter was dissolved in methanol again. The solution was evaporated to dryness using a vacuum distillation apparatus and then suspended again in purified water, thereby collecting insoluble matter. The insoluble matter was collected and then water was removed therefrom using a freeze-dryer, thereby obtaining 10 mg of polyphenol glycoside.

(2) Identification of Aceronidin

The structure of the polyphenol glycoside isolated by the above procedures was determined using various types of spectrum measurement.

Table 9 shows each measurement condition.

Table 9

| | |
|---|---|
| Measurement conditions | |
| High-resolution ESI-MS | |
| Apparatus: | LCT mass spectrometer (Micromass) |
| Mobile phase: | Methanol (0.1 mL/min) |
| Volume of sample solution injected: 5 μl | |
| Ions to be measured: | Positive ions |
| Sample introduction: | Pulse injection |
| Spraying voltage: | 3,000 V |
| Cone voltage: | 30 V |
| Ext. cone voltage: | 2 V |
| Desolvation unit temperature: | 150˚C |
| Ion source temperature: | 120˚C |
| RF Lens: | 200 units |
| Desolvation gas: | Nitrogen (approximately 700 L/hr) |
| Scan range: | m/z 150 to 1,000 (1 sec) |
| Scan interval: | 0.1 sec |
| Internal standard substance: | Leucine enkephalin NMR |
| Apparatus: | UNITY INOVA 500 (Varian) |
| Observation frequency: | $^{1}H$: 499.8 MHz, $^{13}C$: 125.7 MHz |
| Solvent: | $CD_3OD$ |
| Concentration: | 6.3 mg/0.65 mL |
| Standard: | TMS |
| Temperature: | 25˚C |
| $^{1}H$ NMR measurement: | |
| Observation width: | 5 KHz |
| Data point: | 64 K |
| Pulse angle: | 30˚ |
| Pulse repetition time: | 10 sec |
| Repetitions: 16 times | |
| $^{13}C$ NMR measurement: | |
| Observation width: | 30 KHz |
| Data point: | 64 K |
| Pulse angle: | 45˚ |
| Pulse repetition time: | 3 sec |
| Repetitions: 2,400 times | |
| DEPT measurement: (measurement of CH and $CH_3$ with positive signals and of $CH_2$ with negative signals) | |
| Observation width: | 30 KHz |
| Data point: | 64 K |
| Pulse repetition time: | 3 sec |
| Repetitions: 800 times | |
| DQF-COSY measurement: | |
| Observation width: | t2 axis: 5 KHz |
| | t1 axis: 5 KHz |
| Data point: | t2 axis: 2048 |

(continued)

| | |
|---|---|
| Repetitions: 800 times | |
| DQF-COSY measurement: | |
| | t1 axis: 256-x 2 (zero filling to 2048) |
| Pulse waiting time: | 3 sec |
| Repetitions: 16 times | |
| HSQC measurement | |
| Observation width: | t2 axis: 20 KHz |
| | t1 axis: 5 KHz |
| Data point: | t2 axis: 2048 |
| | t1 axis: 256 × 2 (zero filling to 2048) |
| Pulse waiting time: | 2.5 sec |
| Repetitions: 16 times | |
| HMBC measurement: | |
| Observation width: | t2 axis: 25 KHz |
| | t1 axis: 5 KHz |
| Data point: | t2 axis: 2048 |
| | t1 axis: 512 (zero filling to 2048) |
| Pulse waiting time: | 2.5 sec |
| Repetitions: 32 times | |
| NOESY measurement: | |
| Observation width: | t2 axis: 5 KHz |
| | t1 axis: 5 KHz |
| Data point: | t2 axis: 2048 |
| | t1 axis: 256 × 2 (zero filling to 2048) |
| Mixing time: | 1 sec |
| Pulse waiting time: | 3.446 sec |
| Repetitions: 16 times | |

Abbreviations

DEPT: Distortionless Enhancement by Polarization Transfer (A method for determining a carbon type (distinguishing among $CH_3$, $CH_2$, CH, and C))

DQF-COSY: Double Quantum Filtered COrrelation SpectroscopY (A method of $^1H$-$^1H$ COSY)

NOESY: Nuclear Overhauser Effect SpectroscopY

HSQC: Heteronuclear Single Quantum Coherence (A method of $^1H$-$^{13}C$ COSY)

HMBC: Heteronuclear Multiple Bond Correlation (A method of long-range $^1H$-$^{13}C$ COSY)

High-resolution ESI-MS

Fig. 6A shows a total ion chromatogram and Fig. 6B shows a high-resolution ESI mass spectrum. In this measurement, a sodium adduct $(M+Na)^+$ with m/z 473 was strongly observed and then composition calculation was performed using the accurate mass (actual measurement value) thereof, m/z 473.1064. C, H, O, and Na elements were each used for composition calculation. As a result, the compositional formula was determined $C_{21}H_{22}O_{11}Na$. The theoretical accurate mass was m/z 473.1060 with an error of 0.4 mDa. Because of the presence of such ion to which sodium had been added in this measurement, the compositional formula for Aceronidin is $C_{21}H_{22}O_{11}$ and the molecular weight is 450.

NMR measurement

From the high magnetic field side (right side), symbols "a" to "o" were assigned to $^1H$ NMR signals, "A" to "U" were assigned to $^{13}C$ NMR signals, and then analysis was conducted.

$^1$H NMR

Fig. 7 shows an $^1$H NMR spectrum and Table 10 shows the list of signals.

Table 10

| FREQUENCY (PPM) | Hz | SUB | HEIGHT |
|---|---|---|---|
| 6.920 | 3458.710 | -3458.710 | 404.1 |
| 6.916 | 3456.879 | 1.831 | 434.4 |
| 6.854 | 3425.903 | 30.975 | 161.4 |
| 6.850 | 3423.920 | 1.984 | 147.3 |
| 6.838 | 3417.816 | 6.104 | 274.7 |
| 6.834 | 3415.833 | 1.984 | 259.7 |
| 6.795 | 3396.454 | 19.379 | 495.8 |
| 6.779 | 3388.367 | 8.087 | 295.5 |
| 5.987 | 2992.706 | 395.660 | 467.1 |
| 5.983 | 2990.417 | 2.289 | 491.9 |
| 5.802 | 2899.933 | 90.485 | 459.7 |
| 5.797 | 2897.644 | 2.289 | 442.6 |
| 5.325 | 2661.896 | 235.748 | 301.3 |
| 5.303 | 2650.909 | 10.986 | 317.5 |
| 4.872 | 2435.150 | 215.759 | 393.5 |
| 4.865 | 2431.793 | 3.357 | 494.3- |
| 4.850 | 2424.164 | 7.629 | 8167.5 |
| 4.640 | 2319.336 | 104.828 | 301.6 |
| 4.624 | 2311.401 | 7.935 | 312.7 |
| 4.577 | 2287.750 | 23.651 | 23.6 |
| 4.267 | 2133.026 | 154.724 | 180.7 |
| 4.261 | 2129.669 | 3.357 | 178.2 |
| 4.245 | 2122.040 | 7.629 | 177.6 |
| 4.239 | 2118.683 | 3.357 | 173.1 |
| 3.819 | 1909.027 | 209.656 | 175.8 |
| 3.797 | 1897.888 | 11.139 | 213.7 |
| 3.794 | 1896.515 | 1.373 | 206.9 |
| 3.638 | 1818.390 | 78.125 | 125.0 |
| 3.628 | 1813.354 | 5.035 | 144.5 |
| 3.614 | 1806.335 | 7.019 | 121.1 |
| 3.603 | 1800.690 | 5.646 | 190.3 |
| 3.585 | 1791.840 | 8.850 | 162.1 |
| 3.583 | 1790.924 | 0.916 | 161.4 |
| 3.566 | 1782.532 | 8.392 | 137.3 |
| 3.387 | 1692.963 | 89.569 | 34.9 |
| 3.361 | 167-9.840 | 13.123 | 398.1 |
| 3.348 | 1673.431 | 6.409 | 273.7 |
| 3.328 | 1663.666 | 9.766 | 61.3 |
| 3.309 | 1653.748 | 9.918 | 385.3 |
| 3.305 | 1652.222 | 1.526 | 520.1 |
| 3.302 | 1650.696 | 1.526 | 398.0 |
| 3.299 | 1649.170 | 1.526 | 228.9 |
| 3.285 | 1642.151 | 7.019 | 189.1 |
| 3.269 | 1634.064 | 8.087 | 218.5 |
| 3.266 | 1632.538 | 1.526 | 206.6 |
| 3.250 | 1624.603 | 7.935 | 158.9 |

(continued)

| FREQUENCY (PPM) | Hz | SUB | HEIGHT |
|---|---|---|---|
| 1.286 | 642.548 | 982.056 | 18.9 |
| 0.000 | 0.000 | 642.548 | 484.1 |

The $^{1}H$ NMR spectrum demonstrates the presence of the partial structures of 1,2,4-trisubstituted benzene ("o", "n", "m" signals) and 1,2,4,5-tetrasubsfituted benzene ("l" or "k" signal). In addition, "a" to "j" signals were attributed to $CH_n$-O (n = 1 or 2) based on chemical shift values.

$^{13}$<u>CNMR</u>

Fig. 8 shows the $^{13}C$ NMR spectrum and Table 11 shows the list of signals.

Table 11

| FREQUENCY(PPM) | Hz | SUB | HEIGHT |
|---|---|---|---|
| 161.130 | 20251.680 | -20251.680 | 58.1 |
| 159.397 | 20033.812 | 217.868 | 65.1 |
| 157.918 | 19847.983 | 185.829 | 70.4 |
| 147.073 | 18484.933 | 1363.050 | 60.6 |
| 146.527 | 18416.277 | 68.656 | 65.7 |
| 130.016 | 16341.036 | 2075.241 | 49.2 |
| 121.217 | 15235.217 | 1105.819 | 106.0 |
| 116.294 | 14616.398 | 618.819 | 84.4 |
| 116.090 | 14590.766 | 25.632 | 77.5 |
| 101.115 | 12708.678 | 1882.089 | 43.7 |
| 97.313 | 12230.832 | 477.846 | 54.0 |
| 95.733 | 12032.187 | 198.645 | 70.1 |
| 94.626 | 11893.045 | 139.143 | 93.7 |
| 81.355 | 10225.162 | 1667.882 | 100.6 |
| 79.899 | 10042.080 | 183.083 | 112.6 |
| 76.257 | 9584.373 | 457.706 | 74.9 |
| 74.866 | 9409.529 | 174. B44 | 88.3 |
| 74.808 | 9402.206 | 7.323 | 91.8 |
| 72.055 | 9056.180 | 346.026 | 75.9 |
| 68.639 | 8626.851 | 429.329 | 91.2 |
| 62.608 | 7868.889 | 757.962 | 63.1 |
| 49.563 | 6229.385 | 1639.505 | 761.5 |
| 49.396 | 6208.330 | 21.054 | 2610.4 |
| 49.228 | 6187.276 | 21.054 | 4509.3 |
| 49.054 | 6165.306 | 21.970 | 5972.0 |
| 48.886 | 6144.251 | 21.054 | 5092.5 |
| 48.711 | 6122.282 | 21.970 | 2329.7 |
| 48.544 | 6101.227 | 21.054 | 898.4 |
| 0.000 | 0.000 | 6101.227 | 9.8 |

In the case of the $^{13}C$ NMR spectrum, 21 signals were observed and the results agreed with MS measurement results. Signals of ketone carbonyl were not observed.

<u>DEPT</u>

Fig. 9 shows the DEPT spectrum. Based on the spectrum, carbon to which each signal was attributed was determined (see Table 12).

DQF-COSY

Fig. 10 shows the DQF-COSY spectrum. The following partial structures were derived from the spectrum.

(1) j (5.31 ppm) - g (4.25 ppm) - I (4.87 ppm)
- CH(j) - CH(g) - CH(i)-
(2) h (4.63 ppm)-a (3.27 ppm) - d (3.58 ppm) - b (3.35 ppm) or c
f (3.81 ppm) - e (3.62 ppm) - c (3.36 ppm) or b

HSQC

Fig. 11 shows the HSQC spectrum. $^1$H and $^{13}$C coupling at $^1$J ($^1$H, $^{13}$C) was determined from the HSQC spectrum. Table 12 shows the summary of the results.

Table 12

| Types of $^{13}$C, chemical shifts of $^{13}$C, Chemical shifts of $^1$H to be bound to $^{13}$C, and spin coupling constants | | | | |
|---|---|---|---|---|
| $^{13}$C signal | Type of $^{13}$C | Chemical shift of $^{13}$C (ppm) | Chemical shift of $^1$H to be bound $^{13}$C(ppm) | Spin coupling constant J (Hz) |
| A | $CH_2$ | 62.6 | e(3.62), f(3.81) | $J_{e,f}$ = 12.1, $J_{e,c}$ = 5.3, $J_{f,c}$ = 1.4 |
| B | CH | 68.6 | i(4.87) | $J_{i,g}$ = 3.4 |
| C | CH | 72.1 | b(3.35) | |
| D | CH | 74.8 | j(5.31) | $J_{j,g}$ = 11.0 |
| E | CH | 74.9 | d(3.58) | $J_{d,b}$ = 8.4 |
| F | CH | 76.3 | g(4.25) | |
| G | CH | 79.9 | c(3.36) | |
| H | CH | 81.4 | a(3.27) | $J_{a,d}$=9.5 |
| I | CH | 94.6 | h(4.63) | $J_{a,h}$ = 7.9 |
| J | CH | 95.7 | k(5.80) | $J_{k,l}$ = 2.3 |
| K | CH | 97.3 | 1(5.99) | |
| L | C | 101.1 | - | - |
| M | CH | 116.1 | o(6.92) | $J_{o,n}$ = 2.0 |
| N | CH | 116.3 | m(6.79) | $J_{m,n}$ = 8.1 |
| O | CH | 121.2 | n(6.84) | |
| P | C | 130.0 | - | - |
| Q | C | 146.5 | - | - |
| R | C | 147.1 | - | - |
| S | C | 157.9 | - | - |
| T | C | 159.4 | - | - |
| U | C | 161.1 | - | - |

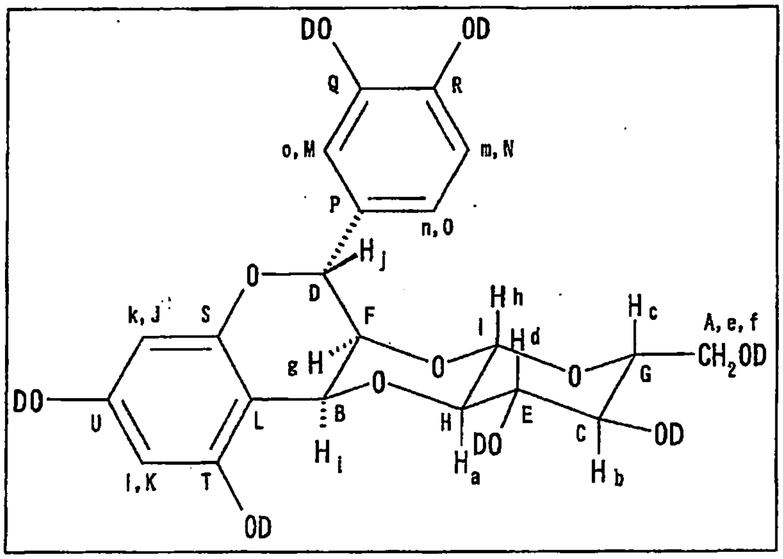

HMBC

Fig. 12 shows the HMBC spectrum. Table 13 shows major long-range correlation signals as observed in the HMBC spectrum.

Table 13

| |
|---|
| a-*B*, C, E, I |
| b-A,E,G |
| c-C,E |
| d-C, H, I |
| e, f-C, G |
| g-B, D, *I*, P |
| h-E, G, H |
| i-D,F,*H*,L,S,T |
| j-B,F,M,O,P,*S* |
| k-K, L, S, U |
| l-J, L, T, U |
| m-P, Q, R |
| n-D, M, R |
| o-D, O, R |

The plane structure of the compound of the present invention was derived from the results.

NOESY measurement

Fig. 13 shows the NOESY spectrum. As shown in the NOESY spectrum, the following correlation signals between protons were observed.

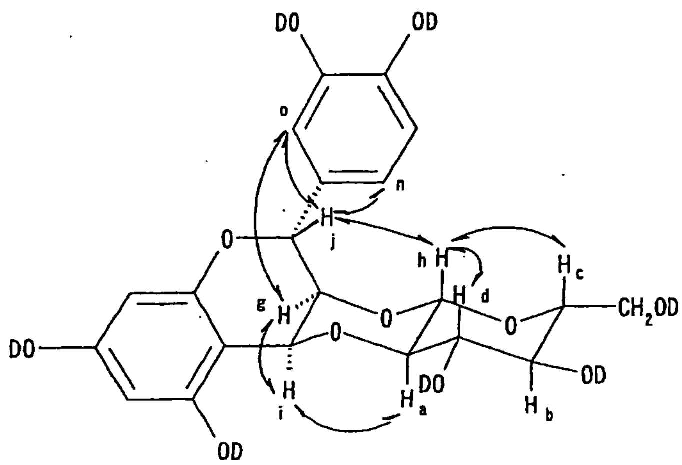

Spin coupling constants of $J_{h,\ a}$= 7.9 Hz, $J_{a,\ d}$ = 9.5 Hz, and $J_{d,\ b}$=8.4 Hz are characteristic to sugars, suggesting its axial-axial form.

NOE was observed between "h" and "c" protons, indicating the presence of "c" proton at an axial position. Therefore, the sugar component was determined to be β-glucose.

A structure in which OH at position 1 and OH at position 2 of glucose are coupled as described above was deduced from the HMBC correlation signals between "g" proton and "I" carbon, "i" proton and "H" carbon, and "a" proton and "B" carbon.

The relative configuration of "j", "g", and "i" protons was deduced as described above from NOE between "A" and "i", "h" and "j", and "g" and "i" protons.

$J_{i,\ g}$ = 11.0 Hz and $J_{g,\ i}$ =3.4 Hz indicate the above relative configuration.

Table 14 is the summary of the list of attribution, in which atoms are numbered.

Table 14

| NMR assignment table | | | |
|---|---|---|---|
| Carbon number | Chemical shift of $^{13}C$ (ppm) | Chemical shift of $^{1}H$ (ppm) | Spin coupling constant J(Hz) |
| 2 | 74.8 | 5.31 | $J_{2,3}$=11.0 |
| 3 | 76.3 | 4.25 | $J_{3,4}$=3.4 |
| 4 | 68.6 | 4.87 | |
| 4a | 101.1 | - | - |
| 5 | 159.4 | - | - |
| 6 | 97.3 | 5.99 | $J_{6,8}$= 2.3 |
| 7 | 161.1 | - | - |
| 8 | 95.7 | 5.80 | $J_{6,8}$=2.3 |
| 8a | 157.9 | - | - |
| 1' | 130.0 | - | - |
| 2' | 116.1 | 6.92 | $J_{2',6'}$ = 2.0 |
| 3' | 146.5 | - | - |
| 4' | 147.1 | - | - |
| 5' | 116.3 | 6.79 | $J_{5',6'}$ = 8.1 |
| 6' | 121.2 | 6.84 | |
| 1" | 94.6 | 4.63 | $J_{1",2"}$ =7.9 |

(continued)

| NMR assignment table | | | |
|---|---|---|---|
| Carbon number | Chemical shift of $^{13}C$ (ppm) | Chemical shift of $^1H$ (ppm) | Spin coupling constant J(Hz) |
| 2" | 81.4 | 3.27 | $J_{2'',3''}$ = 9.5 |
| 3" | 74.9 | 3.58 | $J_{3'',4''}$ = 8.4 |
| 4" | 72.1 | 3.35 | |
| 5" | 79.9 | 3.36 | $J_{5'',6''}$ = 5.3, 1.4 |
| 6" | 62.6 | 3.62, 3.81 | $J_{6'',6''}$=12.1 |

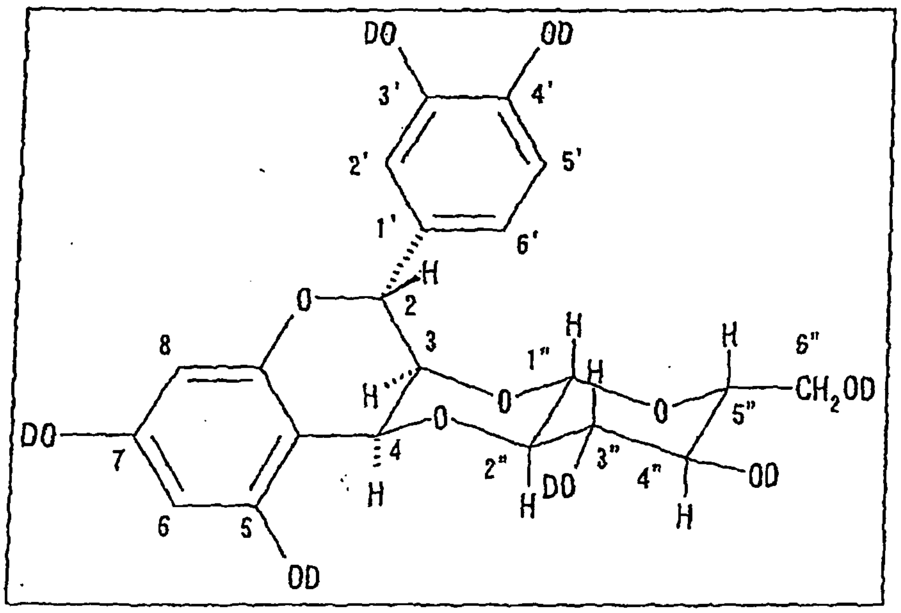

Based on the above results, it was determined that the novel polyphenol glycoside has a structure represented by the structural formula:

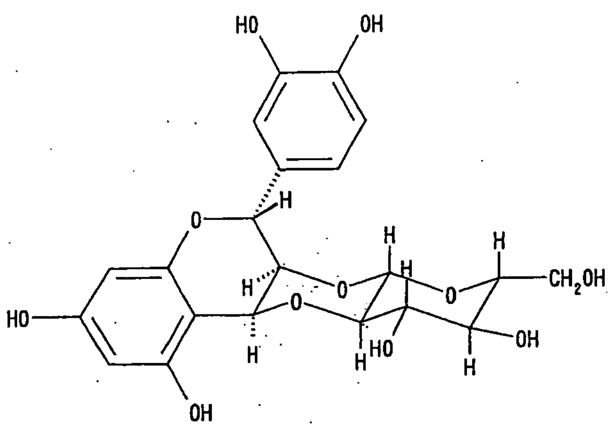

The present inventors designated the novel polyphenol glycoside as Aceronidin.

Example 2

Experiment 2.1. Preparation of acerola powder

1400 kg of concentrated acerola juice (produced in Brazil) was diluted with purified water, so as to prepare a solution with a Brix value of 31%. 1% by weight yeast (*Saccharomyces cerevisiae*) was added to the solution. Fermentation was performed at 30˚C for 20 hours, so as to remove glucose and fructose. After fermentation, centrifugation and filtration were performed. Thus, 2297 kg of processed and concentrated acerola juice, from which glucose and fructose had been removed, was obtained. 4.0% (W/W) dietary fiber (solid content (weight) ratio of dietary fiber to the juice) and 1.5% (W/W) shellfish calcium (solid content (weight) ratio of calcium to the juice) were dissolved as an excipient and an agent for processing, respectively, in the processed and concentrated acerola juice. The solution was powderized by a spray-drying method, thereby obtaining 806 kg of an acerola powder. The acerola powder contained 35.3% (W/W) vitamin C and 1.5% (W/W) polyphenol.

Polyphenol content in the acerola powder was measured by the following procedure. The acerola powder was dissolved in purified water at a concentration of 20% (W/W). A C18 column (Sep-Pak Vac 35 cc (10 g) C18 cartridges, Waters Corporation) was loaded with 50 g of the solution, the column was washed with purified water, and then a fraction eluted with methanol was collected. The amount of polyphenol in the methanol-eluted fraction was measured by the Folin-Denis method using catechin ((+)-Catechin hydrate, Sigma-Aldrich Corporation) as a standard substance. Polyphenol content in the acerola powder was calculated using the weight of the acerola powder used for loading the column, the amount of the methanol eluate collected, and the amount of polyphenol measured. In addition, with the use of this measurement method, Aceronidin that forms a complex with the pectin backbone may not be measured as "polyphenol."

It was considered that the C18 column-adsorbed components contain polyphenol. Hence, in this Example, the amount of polyphenol was measured using the amount of the C18 column-adsorbed components as an indicator.

Experiment 2.2. Preparation of aqueous solution of acerola powder from which C18 column-adsorbed components have been removed

The acerola powder prepared in Experiment 2.1 was dissolved in purified water at 20% (W/W), thereby preparing 50 mL of an aqueous solution. A C18 column (Sep-Pak Vac 35 cc C18 Cartridge Waters Corporation) was loaded with the solution and then C18 column-adsorbing components containing free polyphenol and the like were adsorbed to the column. A fraction that had passed through the column was collected, so that an aqueous solution of acerola powder from which the C18 column-adsorbed components had been removed was prepared. The concentration of the solid content in the solution was found to be 15.3% (W/W). The amount of polyphenol in the aqueous solution was measured by high performance liquid chromatography (C18 column: ODS-3 4.6 mm × 250 mm GL Sciences Inc.). The peak area of polyphenol in the thus obtained specimen was compared with the peak area of polyphenol obtained by the analysis of an aqueous solution of acerola powder (polyphenol concentration: 0.3% (W/W)) before the removal of the C18 column-adsorbed components. When the proportion was calculated after comparison, polyphenol concentration in the specimen was confirmed to be 1% or less (specifically, polyphenol concentration: 0.003% (W/W) or less) of the product compared therewith. Specifically, polyphenol content in the acerola powder from which C18 column-adsorbed components had been removed was 0.02% (W/W) or less on a solid content basis.

The thus obtained aqueous solution was used in the following experiment. The aqueous solution is referred to as "aqueous solution of acerola powder from which C18 column-adsorbed components have been removed" in the description. Moreover, the solid content contained in the aqueous solution may be referred to as "acerola powder from which C18 column-adsorbed components have been removed."

Experiment 2.3. Preparation of aqueous solution of acerola powder from which C18 column-adsorbed components and vitamin C have been removed

The aqueous solution of acerola powder from which C18 column-adsorbed components had been removed (prepared in Experiment 2.2) was diluted with purified water at a solid content concentration of 0.1% (W/W). 100 μl of an ascorbic acid oxidase (TOYOBO Co., Ltd.) solution was added to 5 mL of the aqueous solution, resulting in 30 U of enzyme activity. Furthermore, 400 μl of a 10 mM disodium hydrogenphosphate aqueous solution was added to the solution, followed by overnight reaction at 30˚C in a thermostatic bath. After the completion of the reaction, heat treatment was performed at 120˚C for 10 minutes, thereby deactivating the enzyme. The residual amount of vitamin C was measured by high performance liquid chromatography, so that the amount was confirmed to correspond to 1 % or less of the amount before enzymatic treatment. In addition, vitamin C content before enzymatic treatment was found to be 35.3% (W/W) on a solid content basis. Therefore, the vitamin C content in the acerola powder after the removal of vitamin C was 0.35% (W/W) or less on a solid content basis.

The thus obtained aqueous solution was used in the following experiment. The aqueous solution is referred as "aqueous solution of acerola powder from which C18 column-adsorbed components and vitamin C have been removed" in the description. In addition, the solid content contained in the aqueous solution may also be referred to as "acerola powder from which C18 column-adsorbed components and vitamin C have been removed."

Experiment 2.4. Preparation of C18 column-adsorbed components derived from acerola

The acerola powder prepared in Experiment 2.1 was dissolved in purified water at 20% (W/W), thereby preparing 40 mL of an aqueous solution. A C18 column (Sep-Pak Vac 35 cc C18 Cartridge Waters Corporation) was loaded with the solution and then C18 column-adsorbing components containing free polyphenol and the like were adsorbed to the column. The column was washed with purified water, elution was performed with methanol, and then the eluate was dried and solidified using a vacuum distillation apparatus. Thus, 0.17 g of the C18 column-adsorbed components was collected.

Antioxidative activity test method

Background of the experiment:

Linoleic acid is unsaturated fatty acid that is contained richly also in human body. The unsaturated fatty acid is characterized by being auto-oxidized when it is allowed to stand, so as to be lipid peroxide. In this experiment, linoleic acid is mixed with a sample, the mixture is allowed to stand at 40˚C, and then antioxidative activities are evaluated based on increases in the amounts of lipid oxides purified from linoleic acid.

Test method 2.1

Measurement of anti-oxidation activity (Rhodan-iron method) using linoleic acid

The mixture of 2 ml of 99.5% ethanol and 2 ml of distilled water (a specimen had been previously dissolved in either the 99.5% ethanol or the distilled water) was added to the mixture of 2 ml of 2.5% (w/v) linoleic acid (99.5% ethanol solution) and 4 ml of 0.05 M phosphate buffer (pH 7.0). The resulting mixture was put into a brown screw cap bottle, so that 10 ml of a reaction solution was prepared. When a specimen was a water-insoluble component, the specimen was dissolved in the above 99.5% ethanol, so that a reaction solution was prepared. When a specimen was a water-soluble component, the specimen was dissolved in the above distilled water, so that a reaction solution was prepared. In addition, in this test method, the term "specimen concentration" indicates a specimen concentration in 2 ml of 99.5% ethanol or 2 ml of distilled water. Therefore, the final concentration of each specimen in each reaction solution was one fifth of the predetermined concentration.

Furthermore, regarding specimens positive for antioxidative activities, similar procedures were performed for BHA so that it is contained in appropriate amounts in reaction solutions. Positive control specimens were thus prepared. A control used herein was prepared by adding 2 ml of 99.5% ethanol and 2 ml of distilled water alone to the reaction solution. Such reaction solution stored in the dark at 40˚C was used for the main test and such reaction solution stored at 4˚C was used for a blank test. Test substances were sampled with time and then measured as described below. Tests were conducted for 2 or more weeks.

0.1 ml of $2 \times 10^{-2}$ M ferrous chloride (3.5 % hydrochloric acid solution) was added to the mixture of 0.1 ml of a test substance, 9.7 ml of 75% ethanol, and 0.1 ml of 30% ammonium rhodanate aqueous solution. At precisely 3 minutes after addition, absorbance was measured at 500 nm. Absorbance was similarly measured in a blank test: Δ absorbance = [absorbance in main test] - [absorbance in blank test]. The higher the absorbance, the higher the amount of oxidized lipids. This result indicates the weak antioxidative activity of the relevant specimen. Furthermore, when oxidation of a sample is initiated, the absorbance increases. After the absorbance reaches a peak, the absorbance decreases as the amount of a sample to be oxidized decreases. It can be said that the sooner the absorbance reaches a peak, the weaker the anti-oxidation activity.

Furthermore, anti-oxidation activities of the samples were compared in terms of oxidation ratio (%). Oxidation ratio (%) was obtained by the following formula using the oxidation (absorbance) of the control as 100%.

[Formula 1]

$$\text{Oxidation ratio (\%)} = ([\Delta \text{ absorbance of sample}] / [\Delta \text{ absorbance of control}]) \times 100$$

It can be said that the higher the oxidation ratio (%), the lower the anti-oxidation activity.

Experiment 2.5. Antioxidative activities for lipids of concentrated acerola juice and aqueous solution of acerola powder

The antioxidative activities of a specimen of concentrated acerola juice (Brix 52.2, vitamin C concentration: 18.4% (W/W) on a solid content (weight) basis) and that of a specimen of the acerola powder prepared in Experiment 2.1 were determined with a specimen concentration of 0.02% (W/W) on a solid content basis by Test method 2.1. Fig. 14 shows the results. The Y axis in Fig. 14 refers to absorbance. It is indicated that the higher the numerical value, the more advanced oxidation of linoleic acid. The concentrated acerola juice and the acerola powder exerted sufficient antioxidative activities even after 28 days. In particular, the acerola powder exerted antioxidative activities to a level equivalent to that exerted by BHA, which is a synthetic antioxidant with the same concentration.

Experiment 2.6 Comparison of antioxidative activities of acerola powder and α-tocopherol

The effects of suppressing auto-oxidation of linoleic acid of α-tocopherol (vitamin E) and acerola powder were compared using Test method 2.1. α-tocopherol is a known lipid soluble antioxidant derived from nature.
In this experiment, α-tocopherol ((±)-α-Tocopherol: Wako Pure Chemical Industries, Ltd., first grade reagent), BHA (3(2)-t-Butyl-4-hydroxyanisole: Wako Pure Chemical Industries, Ltd., special grade reagent), which is a synthetic antioxidant, and the acerola powder prepared in Experiment 2.1 were used. Regarding all specimen concentrations, the experiment was conducted under conditions such that the solid content (concentration) of each specimen was 0.02% (W/W). Table 15 shows the experimental results.
Comparison of α-tocopherol with BHA (positive control) in Experiment 1 revealed that BHA had exerted antioxidative activities superior to those of α-tocopherol. When BHA was compared with the acerola powder in Experiment 2, the acerola powder had exerted antioxidative activities at a level equivalent to or even higher than those of BHA. Based on the two experimental results, it was revealed that the acerola powder more strongly suppresses the auto-oxidation of linoleic acid than α-tocopherol (vitamin E).

Table 15

| Test concerning the suppression of auto-oxidation of linoleic acid by α-tocopherol and acerola powder | | | | | | |
|---|---|---|---|---|---|---|
| | Experiment 1 | | | Experiment 2 | | |
| Days of storage | Control | α-tocopherol | BHA | Control | BHA | Acerola powder |
| 1 | 0.0142 | 0.0195 | 0.0000 | 0.0617 | 0.0001 | 0.0038 |
| 4 | 0.5058 | 0.0827 | 0.0105 | 0.7387 | 0.0088 | 0.0433 |
| 5 | 0.8090 | 0.1014 | 0.0111 | 1.0746 | 0.0121 | 0.0438 |
| 8 | 1.4743 | 0.1326 | 0.0313 | 1.5887 | 0.0269 | 0.0537 |
| 11 | 1.8764 | 0.1551 | 0.0559 | - | - | - |
| 14 | - | - | - | 1.8165 | 0.0609 | 0.0537 |
| 15 | 1.7866 | 0.1796 | 0.0752 | - | - | - |
| 18 | - | - | - | 1.7379 | 0.0866 | 0.0520 |

Experiment 2.7. Antioxidative activities of vitamin C (ascorbic acid) for lipids

The antioxidative activities of vitamin C (ascorbic acid, special grade reagent, Wako Pure Chemical Industries, Ltd.) specimens with concentrations of 0.02% (W/W) and 0.04% (W/W) on a solid content (weight) basis was determined by Test method 1. Fig. 15 shows the results. The Y axis in Fig. 15 refers to absorbance, indicating the higher the numerical value, the more advanced oxidation of linoleic acid. The vitamin C specimens with 0.02% (W/W) and 0.04% (W/W) exerted no antioxidative activities at all. Instead, the vitamin C specimen with a concentration of 0.02% (W/W) caused oxidation of linoleic acid earlier than the case of the control during the period from the start of the experiment to 7 days after the start of the experiment.

Experiment 2.8. Comparison of acerola powder, acerola powder from which C18 column-adsorbed components have been removed, and C18 column-adsorbed components derived from acerola in terms of antioxidative potency

The acerola powder prepared in Experiment 2.1 and the C18 column-adsorbed components (derived from acerola) prepared in Experiment 2.4, having different specimen concentrations (W/W), were used as specimens in this experiment. Furthermore, the solid content of the aqueous solution of acerola powder from which C 18 column-adsorbed components had been removed (concentration of solid content: 15.3% (W/W)) prepared in Experiment 2.2 was diluted at different concentrations (W/W). The thus diluted specimens were used in this experiment. These specimens were stored at 40˚C for 28 days and then the antioxidative effect for linoleic acid was determined according to Test method 2.1. Fig. 16 shows the results. Measured values were represented by oxidation ratio (%) of linoleic acid (see Formula 1 above), indicating that the higher the oxidation ratio (%), the weaker the antioxidative potency of a specimen.

An oxidation ratio (%) of 100% means that the amount of the oxide of linoleic acid was the same as that of a control. The horizontal axis refers to sample concentrations used in the test. From the experience of conducting the test for evaluating antioxidants, it can be concluded that an antioxidant significantly suppresses oxidation when the oxidation ratio (%) is 20% or less than that of a control under the same conditions. Hence, based on the results in Fig. 16, it can be concluded that the effective concentration of each specimen is: 0.005% (W/W) or more in the case of the C18 column-adsorbed component specimen derived from acerola; 0.015% (W/W) or more in the case of the acerola powder specimen; and 0.0175% (W/W) or more in the case of the specimen of the acerola powder from which C18 column-adsorbed components had been removed on a solid content (concentration) basis.

The antioxidative activities of the isolated C18 column-adsorbed components derived from acerola were clearly the highest. Moreover, the acerola powder from which C18 column-adsorbed components had been removed also had sufficient antioxidative activities. Hence, it was inferred that components other than the C18 column-adsorbed components also contributed to antioxidative activities. The polyphenol content in a solution with an effective acerola powder concentration (concentration of solid content: 0.015% (W/W)) was as very low as 0.000225% (W/W) on a solution basis. Since the acerola powder has a somewhat higher level of antioxidative activities than the acerola powder from which C18 column-adsorbed components have been removed, it was inferred that such a small amount of the C 18 column-adsorbed components contributes to antioxidative activities. Hence, it was concluded that the effect of the acerola powder to suppress auto-oxidation of linoleic acid is exerted by a combination of the C18 column-adsorbed components and other components.

Experiment 2.9. Test of anti-oxidation for lipids using the aqueous solution of acerola powder from which C18 column-adsorbed components have been removed and the aqueous solution of acerola powder from which C18 column-adsorbed components and vitamin C have been removed

The antioxidative activities of a specimen of the aqueous solution of acerola powder, from which C18 column-adsorbed components had been removed, prepared in Experiment 2.2 and that of a specimen of the aqueous solution of acerola powder, from which C18 column-adsorbed components and vitamin C had been removed, prepared in Experiment 2.3 were determined using a specimen concentration of 0.02% (W/W) on a solid content (weight) basis by Test method 1. Fig. 17 shows the results. It was confirmed that the aqueous solution of acerola powder from which C 18 column-adsorbed components and vitamin C have been removed has sufficient anti-oxidation activities for lipids. The reason why this solution had antioxidative activities lower than those of the acerola powder from which C18 column-adsorbed components had been removed with the same concentration may be due to the removal of vitamin C. On the other hand, as shown in Experiment 2.7, vitamin C alone does not act as an antioxidant for lipids (Fig. 15). Hence, the results of this experiment demonstrate that vitamin C in acerola exerts antioxidative activities for lipids in conjunction with acerola components other than the C18 column-adsorbed components.

Experiment 2.10. Preparation of acid-soluble acerola pectin

Acerola fruits were crushed using a Waring blender while adding 3 kg of purified water to 3 kg of the acerola fruits, so that a crushed acerola product was prepared. Ethanol was added to the product until it accounted for 30% by weight. The resultant was agitated at room temperature overnight so that water and an ethanol soluble component were extracted. The extract was centrifuged at 4200 rpm for 30 minutes, thereby separating solid content. 1500 g of the solid content was collected.

5200 g of purified water was added to 1500 g of the solid content to prepare a suspension. Concentrated hydrochloric acid was added to the suspension to adjust the resultant to pH 2.2. Heat treatment was performed for 2 hours at 80˚C to 90˚C using a plate heater while agitating the suspension. The suspension was allowed to stand to lower the temperature to room temperature and then subjected to centrifugation at 4200 rpm for 30 minutes. Thus, the resultant was separated into solid content and a supernatant and then the supernatant was collected. The supernatant was filtered

using a 0.2 μm filter to remove insoluble components, thereby obtaining a clear extract.

Ethanol was added to the extract in an amount 3 times greater than the weight of the extract, so that an ethanol-insoluble pectin component was deposited. The deposited pectin component was collected using stainless mesh. Furthermore, to remove ethanol- and water-soluble components, the resultant was washed twice with a 90% ethanol aqueous solution, collected, and then dried using a freeze-dryer. Thus, 6.24 g of acid-soluble acerola pectin was collected.

Experiment 2.11. Preparation of acerola pectin digested with pectinase

Acerola fruits were crushed using a Waring blender while adding 2 kg of purified water to 2 kg of the acerola fruits, so that a crushed acerola product was prepared. Ethanol was added to the product until it accounted for 40% by weight. The resultant was agitated at room temperature overnight so that water and an ethanol soluble component were extracted. The extract was centrifuged at 4200 rpm for 30 minutes, thereby separating solid content. 1000 g of the solid content was collected.

1000 g of the solid content was mixed with 3000 g of purified water and then mixed with 4 g of a pectinase powder (pectinase "AMANO A," AMANO ENZYME INC.). The mixture was allowed to stand at 45˚C overnight. The mixture was centrifuged at 4200 rpm for 30 minutes, so that the mixture was separated into solid content and a supernatant. The supernatant was collected. The supernatant was filtered using a 0.2 μm filter to remove insoluble components, thereby obtaining a clear extract.

Ethanol was added to the extract in an amount 3 times greater than the weight of the extract, so that an ethanol-insoluble pectin component was deposited. The deposited pectin component was centrifuged at 4200 rpm for 30 minutes, so that the component was collected as solid content. The solid content was further washed with a 90% ethanol aqueous solution and then dried using a freeze-dryer. Thus 12.6 g of dry solid content was collected. The dry solid content was designated a pectin treated with pectinase. It is considered that the pectin component is a pectin hydrolysate because the pectin component has low viscosity although viscosity is a characteristic of an aqueous pectin solution.

Experiment 2.12. Measurement of the molecular weight of the acerola-derived pectin

The molecular weights of the pectin treated with acid (prepared in Experiment 2.10) and the pectin products that had been treated with acid and then digested with pectinase were measured by gel filtration chromatography.

The pectin treated with acid was dissolved in purified water at 0.5% (W/W). 20 mL of an aqueous solution was thus prepared, filtered using a 0.2 μm filter, and then used. The pectin products that had been treated with acid and then digested with pectinase were prepared as follows. The pectin treated with acid (prepared in Experiment 2.10) was dissolved in purified water at 0.3% (W/W), so that 20 mL of an aqueous solution was prepared. 6 mg of a pectinase powder (pectinase "AMANO A," AMANO ENZYME INC.) was added to the solution, followed by a reaction at 50˚C overnight. After completion of the reaction, heat treatment was performed at 120˚C for 15 minutes to deactivate the enzyme. The resultant was filtered using a 0.2 μm filter. The filtered resultant was concentrated using a vacuum distillation apparatus to 2 mL and then the concentrated product was filtered using a 0.2 μm filter. Sephacryl S-300 High Resolution (Amersham Biosciences) was used as a carrier for gel filtration. A column was loaded with the carrier and then gel filtration measurement was performed. PBS (Dulbecco's phosphate buffered saline) was used as a buffer. In measurement of the molecular weights, molecular weight markers thought to be appropriate for this measurement were used herein. These molecular weight markers are: Blue Dextran 2000, Catalase, Alubmin, and Chymotrypsinogen A in an HMW Gel Filtration Calibration Kit (Amersham Biosciences) and an LMW Gel Filtration Calibration Kit (Amersham Biosciences).

As a result of the measurement, the molecular weight of the pectin treated with acid (prepared in Experiment 2.10) was found to be almost the same as that measured using Blue Dextran 2000. Thus, it was revealed that the pectin treated with acid (prepared in Experiment 2.10) is a molecule with a molecular weight of approximately 2,000,000. Furthermore, a plurality of peaks were observed in the case of the pectin products that had been treated with acid and then digested with pectinase. It was confirmed that all the molecular weights were lower than that of Chymotrypsinogen A with a molecular weight of 20.4 kDa. Therefore, it was inferred that the pectin treated with the enzyme was a mixture of molecules each having a molecular weight of 20,000 or less.

Experiment 2.13. Comparison of acerola-derived pectin specimens in terms of antioxidative potency

The antioxidative activities of the acerola-derived pectin treated with acid (prepared in Experiment 2.10) and the same of the acerola-derived pectin treated with pectinase (prepared in Experiment 2.11) were determined by Test method 2.1 using a specimen concentration of 0.1% (W/W) on a solid content (weight) basis. Fig. 18 shows the results. Both specimens exhibited sufficient antioxidative activities for lipids. Hence, it was revealed that both types of pectin are effective as antioxidants for lipids. As described in Experiment 2.12, although the acerola pectin treated with acid

and the pectin treated with the enzyme completely differ from each other in molecular weight, the former pectin exerted lipid antioxidative activities to a level equivalent to that exerted by the latter pectin. It is inferred that the acerola-derived pectin that had been hydrolyzed by another enzyme or the like also has antioxidative activities for lipids at a level equivalent to those of the other pectin.

Experiment 2.14

The antioxidative activities of acerola-derived pectins were evaluated by a DPPH radical scavenging activity test.
Anti-oxidation activity was evaluated using an ethanol solution of diphenyl-p-picrylhydradil (DPPH) that is a stable radical. 1600 μl of a 250 mM acetate buffer (pH = 5.5) was mixed with 1200 μl of ethanol and 400 μl of a specimen (adjusted at a predetermined concentration), followed by preincubation at 30˚C for 5 minutes. 800 μl of a 500 μM DPPH/ethanol solution was added to the solution and then the solution was mixed. The solution was allowed to stand at 30˚C for 30 minutes and then absorbance was measured at 517 mn. A control used herein was prepared by similar procedures using purified water instead of a sample solution. Specimens used herein were the acerola-derived pectin treated with acid (prepared in Experiment 2.10) and the acerola-derived pectin treated with the enzyme (prepared in Experiment 2.11). Specimens used for comparison were solutions prepared by dissolving an apple-derived pectin (Wako Pure Chemical Industries, Ltd., reagent) and a citrus-derived pectin (Wako Pure Chemical Industries, Ltd., reagent) at 0.3% (W/W) and 0.1% (W/W), respectively, with purified water. Radical scavenging ratios were calculated by the following formula using the thus measured absorbances.

[Formula 2]

$$\text{Scavenging ratio (\%)} = (1 - [\text{absorbance of sample}]/[\text{absorbance of control}]) \times 100$$

Table 16 shows the results. These results revealed that unlike the other pectins, the acerola-derived pectin treated with acid or the pectin treated with the enzyme is an antioxidative substance having radical scavenging activity. Moreover, it was also revealed that the antioxidative activities are enhanced approximately 2-fold through pectinase (enzyme) treatment.

Table 16

| | Concentration | $OD_{517}$ | Radical scavenging ratio (%) |
|---|---|---|---|
| Control (purified water) | | 1.26 | |
| Acerola-derived pectin treated with acid | 0.3%<br>0.1% | 0.73<br>1.00 | 41.9<br>21.1 |
| Acerola-derived pectin treated with enzyme | 0.3%<br>0.1% | 0.13<br>0.77 | 89.9<br>38.9 |
| Apple-derived pectin | 0.3%<br>0.1% | 1.26<br>1.27 | 0.5<br>0.0 |
| Citrus-derived pectin | 0.3%<br>0.1% | 1.25<br>1.26 | 0.6<br>0.2 |

Experiment 2.15

Half bodies of salmons were immersed in saline solutions containing an acerola powder, so as to prepare salt-cured salmon samples. Under fluorescent lighting conditions, changes in appearance, sensuality, color (Hunter Lab), acid value, and peroxide value were measured before and after storage. Based on these changes, the anti-oxidation effect of the acerola powder for lipids was confirmed.
The acerola powder was prepared as follows. 1400 kg of concentrated acerola juice produced in Brazil was diluted with purified water and then Brix value was adjusted to be 31%. 1% by weight yeast *(Sacchanomyces cerevisiae)* was added to the solution and then fermentation was performed at 30˚C for 20 hours, so that glucose and fructose were removed. After fermentation, centrifugation and filtration were performed. Thus, 2297 kg of a processed and concentrated acerola juice was obtained, from which glucose and fructose had been removed. Next, 400 g of dextrin as an excipient,

150g of dietary fiber, and 50 g of processed starch were dissolved in 400 g of the processed and concentrated acerola juice containing acerola-derived solid content. The solution was powderized by a spray-drying method, so that 780 g of an acerola powder for food processing was obtained. This acerola powder contains 0.5% to 1.0% polyphenol. This acerola powder is different from the acerola powder obtained in Experiment 2.1 in that it contains no shellfish calcium that is a bitterness component, so that the acerola powder can be used for processing wide-ranging food products.

As an immersion fluid to be used in an acerola addition test, an aqueous solution containing the above acerola powder (5 % by weight), common salt (20 % by weight), and sodium hydrogencarbonate (5 % by weight) was prepared. Furthermore, as an immersion fluid to be used in a control test, an aqueous solution containing common salt (20 % by weight) was prepared.

Frozen raw material fish (silver salmon (dressed)) was thawed, washed with saline water to wash off the slimy surface, and then cut into fillets. Bones in the abdomen were removed from the fillets, so that samples for this experiment were prepared. One of the salmon half bodies was immersed in the immersion fluid containing the acerola powder. The other half of the same was immersed in the immersion fluid for the control test. After overnight immersion, the fillets were drained off, vacuum-packed, and then cryopreserved until the fillets were subjected to the following fluorescent lighting experiment.

The fluorescent lighting experiment was conducted by the following procedures. First, the above salt cured salmon sample was thawed, cut into an appropriate size, and then put into a foamed polystyrene tray. The tray was packed entirely with a wrap (Shin-etsu Polymer Co., Ltd., "Polymawrap"), placed in a show case, and then subjected to 48 hours of fluorescent lighting with 1500 lux. at 10˚C.

Changes in appearance were observed before and after fluorescent lighting. Before lighting, samples of the group to which acerola had been added were slightly dim colored compared with samples of the control test group, but they were not much different from each other. After the fluorescent lighting experiment, the samples of the group to which acerola had been added were slightly dim- and dark-colored compared with the samples of the same before lighting, however, retained red color. In contrast, the samples of the control test group showed clear discoloration of red color. Hence, it was demonstrated that the acerola powder can prevent discoloration of salmon during storage. Furthermore, changes in sensuality were observed before and after fluorescent lighting. Before lighting, no odor resulting from lipid oxidation was sensed in both the group to which acerola had been added and the control test group. After lighting, flavor resulting from lipid oxidation and lipid deterioration was sensed more strongly in the case of the control test group than in the case of the group to which acerola had been added. The table below shows the results of the above evaluation. 20 samples were prepared for each test group. Numbers in the table are the number of samples corresponding to each item. Furthermore, Fig. 19 shows photographs of samples after storage under fluorescent lighting conditions.

Table 17

| | Test group to which acerola was added | Control test group |
|---|---|---|
| Very good in flavor and appearance | 5 | 1 |
| Good in flavor and appearance | 8 | 1 |
| Yes and No | 5 | 3 |
| Deteriorated flavor and appearance | 1 | 10 |
| Very deteriorated flavor and appearance | 1 | 5 |

Hunter Lab measurement for color measurement was performed at 3 time points: before immersion; after immersion but before lighting (simply "before lighting" in Table 18); and after lighting. Lab measurement was performed using CHROMA METER CR-200 (Minolta Co., Ltd.). Measurement before immersion and measurement after lighting were performed for 5 samples. Measurement after immersion but before lighting was performed for 2 samples. Average values are each summarized in Table 18.

Table 18

| | Test group to which acerola was added | | | Control test group | | |
|---|---|---|---|---|---|---|
| | L | a | b | L | a | b |
| Before immersion | 39.24 | 21.24 | 20.45 | 40.52 | 25.42 | 23.75 |
| Before lighting | 42.53 | 19.96 | 17.46 | 42.68 | 19.58 | 15.12 |
| After lighting | 39.36 | 19.54 | 17.18 | 45.93 | 16.56 | 17.04 |

Only changes in the "a" value (indicating red color) observed before and after lighting are extracted from Table 18 and shown in Fig. 20. As is clear from Fig. 20, whereas almost no decreases were observed in the "a" value in the test group to which acerola had been added, decreases in "a" value were observed in the control test group.

Moreover, the acid value (AV) and the peroxide value (POV) of each sample after fluorescent lighting were measured. Measurement was performed according to the method described in Food Analysis Handbook ($2^{nd}$ ed., KENPAKUSHA). Table 19 shows the results.

Table 19

| Acid value and peroxide value after fluorescent lighting experiment | | |
|---|---|---|
| | Test group to which acerola was added | Control test group |
| Acid value (mgKOH/g) | 2.20 | 2.30 |
| Peroxide value (meq/kg) | 0.00 | 4.33 |

After fluorescent lighting and storage, the acid value and the peroxide value in the test group to which acerola had been added were smaller than those in the control test group. Specifically, it was demonstrated that addition of the acerola powder had suppressed lipid oxidation.

Experiment 2.16

Salted salmon roe was immersed in a seasoning solution containing an acerola powder, so that salted salmon roe that had been seasoned was prepared. Changes in appearance, flavor, acid value, and peroxide value were measured before and after storage under fluorescent lighting conditions. Based on these changes, the anti-lipid-oxidation effect of the acerola powder was confirmed.

As an acerola powder, the acerola powder prepared in Experiment 2.15 was used.

As a seasoning solution to be used for an acerola addition test, an aqueous solution containing the above acerola powder (5 % by weight), *sake* (35% by weight), *shiro-shoyu* (white soy sauce) (35% by weight), *mirin* (Japanese sweet rice wine for cooking) (20% by weight), sodium hydrogen carbonate (4.995 % by weight), and sodium nitrite (0.005 % by weight) was prepared. Furthermore, as a seasoning solution for a control test, an aqueous solution having the same composition except that it contained no acerola powder and no sodium hydrogencarbonate was prepared.

Frozen salted salmon roe (raw salted salmon roe that had been frozen) was thawed, washed with saline water, immersed in the above seasoning solution for 1 hour, refrigerated overnight for maturation, and then sorted into *"san-toku* (triple special)" and *"kuroko* (black salmon roe)." After sorting, the salmon roe was cryopreserved until it was subjected to the following fluorescent lighting experiment.

The fluorescent lighting experiment was conducted by the following procedures. First, *"san-toku"* or *"kuroko"* sorted from salted salmon roe were thawed and then put into a foamed polystyrene tray. The tray was entirely wrapped (Shin-etsu Polymer Co., Ltd., "Polymawrap"), placed in chilled storage, and then subjected to 144 hours of fluorescent lighting with 1500 lux. at 10˚C.

Changes in appearance and flavor were observed before and after fluorescent lighting. Before and after lighting, changes in appearance and deterioration in flavor (generation of lipid oxidation flavor) were suppressed in the test group to which acerola had been added compared with the control test group. The above results of evaluation are shown in the table below. 20 samples were prepared for each test group. The numbers in the table are the numbers of samples corresponding to all items.

Table 20

| | Test group to which acerola was added | Control test group |
|---|---|---|
| Very good in flavor and appearance | 3 | 1 |
| Good in flavor and appearance | 10 | 3 |
| Yes and No | 3 | 4 |
| Deteriorated flavor and appearance | 3 | 7 |
| Very deteriorated flavor and appearance | 1 | 5 |

The acid value (AV) and peroxide value (POV) of each sample were measured after fluorescent lighting (however, in the case of *kuroko* samples, only the acid value was measured). Measurement was performed according to the method described in Food Analysis Handbook (2nd ed., KENPAKUSHA). Table 21 shows the results.

Table 21

| Acid value and peroxide value after fluorescent lighting experiment | | | | |
|---|---|---|---|---|
| | *Santoku* (triple special) | | *Kuroko* (black salmon roe) | |
| | Test group to which acerola was added | Control test group | Test group to which acerola was added | Control test group |
| Acid value (mgKOH/g) | 0.90 | 3.00 | 0.90 | 3.50 |
| Peroxide value (meq/kg) | 0.00 | 10.00 | - | - |

In both *Santoku* and *Kuroko* cases, the acid value and the peroxide value after fluorescent lighting and storage were significantly smaller in the test group to which acerola had been added than those in the control test group. Specifically, it was demonstrated that addition of the acerola powder had suppressed lipid oxidation.

Example 3

Pectinase preparation

In the following experiment, pulp digestion was performed using a pectinase preparation (pectinase A "Amano," AMANO ENZYME INC.). This enzyme preparation contains pectinase 45%, β-amylase 25%, and diatomaceous earth 30%. Hence, the pectin component and the starch component in acerola pulp are digested by the enzyme preparation. The pectinase is an enzyme that is purified from the culture product of molds *Aspergillius pulverulentus* and *Aspergillius niger.* The pectinase is assumed to be a mixture of a plurality of types of pectinase. Since the pectinase causes a rapid decrease in the viscosity of an acerola-derived pectin extracted through treatment with acid and heat, it is considered that the pectinase contains end-polygalacturonase that randomly cleaves the inside of the pectin molecule to immediately lower the molecular weight thereof.

Experiment 3.1. Method for preparing acerola powder VC30 (product for comparison)

Seed portions were removed from acerola fruits produced in Brazil. To enhance flowability, the seed portions were mixed with ion exchange water. The large solid content in the mixture was removed using a stainless mesh filter and then the resultant was put into a tank for pectinase treatment. A 0.01% (W/W) pectinase preparation (pectinase A "Amano," AMANO ENZYME INC.) per 7 Brix as measured using a saccharimeter was put into the tank while heating the tank at 40˚C to 50˚C, so that 1 to 2 hours of enzyme treatment was performed. The thus treated acerola product was heated after enzyme treatment, so that the enzyme was deactivated. The product was sterilized and then subjected to diatomaceous earth filtration, so that clear acerola juice was obtained. The acerola juice was concentrated by a vacuum distillation and concentration method, thereby preparing concentrated acerola juice.

1400 kg of the concentrated acerola juice was diluted with purified water, so as to adjust the Brix value to 31 %. 1 % by weight yeast *(Sacchanomyces cerevisiae)* was added to the diluted solution, followed by 20 hours of fermentation at 30˚C. After fermentation, centrifugation and filtration were performed to remove glucose and fructose. Thus, 2297 kg of processed and concentrated acerola juice was obtained. 4.0% (W/W) dietary fiber (solid content (weight) ratio of dietary fiber to the juice) and 1.5% (W/W) shellfish calcium (solid content (weight) ratio of shellfish calcium to the juice) were dissolved as an excipient and an agent for processing into the processed and concentrated acerola juice. The thus obtained solution was subjected to a spray-drying method, so that 806 kg of an acerola powder was obtained (hereinafter, the acerola powder is also referred to as "acerola powder VC30"). The acerola powder contains 35.0% (W/W) ascorbic acid and 1.07% (W/W) galacturonic acid. Specifically, the acerola powder VC30 contains 3.1 % by weight galacturonic acid with respect to ascorbic acid. Regarding a quantification method for galacturonic acid, see the following description.

3.2. Method for preparing acerola powder A (product of the present invention)

Seeds were removed from acerola fruits produced in Brazil, so that acerola puree was prepared. No procedure to remove the solid content from the acerola puree was performed. Thus, pulp is contained richly in the acerola puree.

A 1% (W/W) pectinase preparation (pectinase A "Amano," AMANO ENZYME INC.) was mixed with 10.8 kg of the acerola puree (Brix value: 8%). The mixture was heated at 50˚C for 4 hours. Through heating of the treated product, the enzyme was deactivated and sterilization was performed. The resultant was separated by centrifugation into solid content and fruit juice. The fruit juice was concentrated by vacuum distillation, so that 3.6 kg of the concentrated solution (Brix value: 25.2%) was collected. 1% by weight yeast *(Saccharomyces cerevisiae)* was added to the concentrated solution, followed by 20 hours of fermentation at 30˚C. After fermentation, centrifugation and filtration were performed to remove glucose and fructose. Thus, 3.3 kg of processed and concentrated acerola juice (Brix value: 21.5%) was obtained. 4.0% (W/W) dietary fiber (solid content (weight) ratio of dietary fiber to the juice) and 1.5% (W/W) shellfish calcium (solid content (weight) ratio of shellfish calcium to the juice) were dissolved as an excipient and an agent for processing in 2.8 kg of the processed and concentrated acerola juice. The solution was subjected to a spray-drying method, so that approximately 0.5 kg of an acerola powder was obtained (hereinafter, it may also be referred to as "acerola powder A"). The acerola powder contains 29.3% (W/W) ascorbic acid and 3.47% (W/W) galacturonic acid. Specifically, the acerola powder A contains 11.8 % by weight galacturonic acid with respect to ascorbic acid. Regarding a quantification method for galacturonic acid, see the following description.

3.3. Method for quantifying galacturonic acid

Galacturonic acid was quantified by the following method.

20 g of a specimen containing galacturonic acid was dissolved in 80 g of purified water that had been added thereto. After the specimen had been sufficiently dissolved, 10 g of the solution was weighed and put into a 50 ml centrifuge tube. 40 ml of ethylalcohol (special grade, Wako Pure Chemical Industries, Ltd.) was added to and mixed sufficiently with the resultant in the tube (ethanol precipitation). After the mixture was allowed to stand for 30 minutes or more, centrifugation was performed at 3000 rpm for 20 minutes (20˚C). Subsequently, the precipitate was completely collected and then dried and solidified using a freeze dryer. The thus dried product was dissolved in purified water at 0.02%, 0.05%, 0.1%, and 0.2% (W/W), thereby preparing solutions for quantification. The amount of galacturonic acid was quantified by a 3,5-dimetyl phenol method. First, 125 μl each of the solutions for quantification was put into a test tube. Subsequently, 125 μl of 2% sodium chloride aqueous solution and 2 ml of concentrated sulfuric acid (special grade, Wako Pure Chemical Industries, Ltd.) were each added to the solutions, followed by 10 minutes of reaction at 70˚C. Each resultant was cooled in water for 20 to 30 seconds. 0.1 ml of a coloring reagent (prepared by dissolving 0.1 g of 3.5-dimethylphenol in 100 ml of glacial acetic acid) was added to the resultant. 10 minutes later, absorbance was measured at 450 nm and 400 nm and then the difference between the two was found. As a standard substance, galacturonic acid monohydrate (special grade reagent, Wako Pure Chemical Industries, Ltd.) was used. Based on the calibration curve (12.5 μg/g to 50 μg/g) of the standard substance, the amount of galacturonic acid in the dried product was calculated. With the use of the thus calculated figure and the weight of the dry product obtained from 2 g of a specimen via ethanol precipitation and drying, the galacturonic acid content in the specimen was calculated. Table 22 shows the quantification results. In addition, ethanol precipitates obtained in this quantification also contained dietary fiber that had been used as an excipient.

Table 22

| | Acerola powder VC30 (product for comparison) | Acerola powder A (product of the present invention) |
|---|---|---|
| Weight of dry product obtained from 2 g of specimen via ethanol precipitation and drying | 1.205 g | 1.36 g |
| Galacturonic acid content (W/W%) in dry product | 1.8% | 5.1% |
| Galacturonic acid content (W/W%) in specimen | 1.08% | 3.47% |

3.4. Skin-whitening test

The effect of suppressing pigmentation after UV irradiation was examined using brown guinea pigs (SPF). The brown guinea pigs are of an animal species that undergoes pigmentation because of UV irradiation in a manner similar to humans and of a line that has been clearly maintained. Six guinea pigs were used for each group in this test. To promote pigmentation, UV (UVB) irradiation was performed from a distance of 40 cm using five SE lamps (wavelength

between 250 nm and 350 nm, FL20S·E, TOSHIBA Corporation) installed in an UV irradiation apparatus (Y-798-II, Orion Electric Co., Ltd.). The shortest time required for erythema to appear on skin due to UV irradiation was measured in a preliminary test and found to be 12 minutes and 30 seconds. This time was designated the time for UV irradiation in this test. Each irradiation site was a 2 cm × 2 cm square area located on either the left or right across the midline of the back of a guinea pig that had been sheared using electric clippers and then shaved using an electric shaver. UV irradiation was performed 3 times in total, including on the day of initial administration (designated day 0) and days 2 and 4 after the initial administration. A test substance was administered via oral administration (using a catheter) of the solution of each test substance, which had been prepared so that the dose of ascorbic acid was 300 mg/kg animal weight/day. Specifically, the experiment was conducted using equal amounts of ascorbic acid. As a blank, water for injection (OTSUKA Pharmaceutical Co., Ltd.) was administered. As test substances, the acerola powder VC30 (831 mg/5 mL) that was the product for comparison, the acerola powder A (1024 mg/5 mL) that was the product of the present invention, and ascorbic acid (special grade reagent, Wako Pure Chemical Industries, Ltd.) (300 mg/5 mL) were used. Oral administration was performed for 42 days. Pigmentation was measured as follows. Before the initial administration (before irradiation) on the day of the initiation of administration and on days 7, 14, 21, 28, 35, and 42 after initial administration, L value (lightness) of irradiated sites were measured using a colorimeter (CR-300, Minolta Co., Ltd.) and then the ΔL value (L value on the day of observation - L value before irradiation) was found. A total of 5 measurement sites used herein include the center and 4 corners located diametrically opposite each other at each irradiation site. The average value thereof was designated the L value of each individual guinea pig. It is indicated that the higher the ΔL value, the stronger the level of pigmentation. Fig. 21 shows the test results. The vertical axis indicates ΔL value and the horizontal axis indicates days after the initiation of the test. Each measurement value is the average value of the ΔL values of 6 guinea pigs of one group.

As a result, in the case of the group to which the acerola powder VC30 (the product for comparison) had been administered, a decrease in the ΔL value was found to be significantly suppressed at each observation time point, compared with the group to which water for injection had been administered. Specifically, the effect of suppressing pigmentation was exerted. In the cases of the group to which acerola powder VC30 (the product for comparison) had been administered and the group (positive control) to which ascorbic acid had been administered, measurement values were almost the same. Therefore, it was inferred that the effect of the acerola powder VC30 to suppress pigmentation is due to ascorbic acid contained therein.

On the other hand, the ΔL values in the case of the group to which the acerola powder A (the product of the present invention) had been administered were smaller than the ΔL values in the case of the group to which ascorbic acid had been administered on all days of observation. Accordingly, it was suggested that the acerola powder A (the product of the present invention) has a component that promotes the effect of ascorbic acid to suppress pigmentation that is not contained in the product for comparison (acerola powder VC30).

All publications, patents, and patent applications cited herein are incorporated herein by reference in their entirety.

## Claims

1. A pectin derived from an acerola fruit or a hydrolysate thereof, comprising a complex formed of a pectin backbone and a polyphenol compound represented by chemical formula:

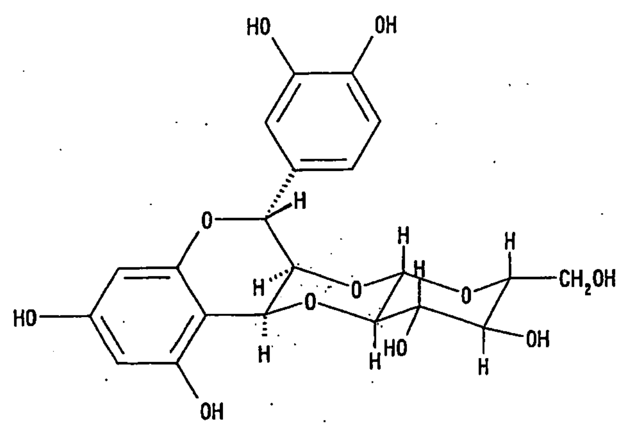

2. The method for producing the pectin according to claim 1, comprising a step of isolating or concentrating a pectin from an acerola fruit or a processed product thereof.

3. The method for producing the pectin hydrolysate according to claim 1, comprising a step of isolating or concentrating a pectin from an acerola fruit or a processed product thereof and a step of hydrolyzing the pectin.

4. The method according to claim 3, comprising a step of hydrolyzing a pectin in puree prepared from an acerola fruit through treatment of the puree with pectinase and a step of isolating or concentrating the hydrolyzed pectin from a supernatant of the processed product in the former step.

5. The method according to any one of claims 2 to 4, wherein the step of isolating or concentrating a pectin is a step of precipitating a pectin using ethanol.

6. The method according to any one of claims 2 to 4, wherein the step of isolating or concentrating a pectin is a step of isolating or concentrating a pectin using a separation membrane.

7. The method according to claim 6, wherein the separation membrane is an ultrafiltration membrane.

8. The method according to claim 7, wherein the ultrafiltration membrane has a molecular weight cut-off ranging from 10,000 to 100,000.

9. A material containing a pectin derived from an acerola fruit, which is produced by a method comprising a step of isolating or concentrating a pectin from an acerola fruit or a processed product thereof.

10. A material containing a hydrolysate of a pectin derived from an acerola fruit, which is produced by a method comprising a step of isolating or concentrating a pectin from an acerola fruit or a processed product thereof and a step of hydrolyzing the pectin.

11. The material according to claim 10, which is produced by a method comprising a step of hydrolyzing a pectin in puree prepared from an acerola fruit through treatment of the puree with pectinase and a step of isolating or concentrating the hydrolyzed pectin from a supernatant of the processed product resulting from the former step.

12. The material according to any one of claims 9 to 11, wherein the step of isolating or concentrating a pectin is a step of precipitating a pectin using ethanol.

13. The material according to any one of claims 9 to 11, wherein the step of isolating or concentrating a pectin is a step of isolating or concentrating a pectin using a separation membrane.

14. The material according to claim 13, wherein the separation membrane is an ultrafiltration membrane.

15. The material according to claim 14, wherein the ultrafiltration membrane has a molecular weight cut-off ranging from 10,000 to 100,000.

16. An antioxidant, containing the pectin or the hydrolysate thereof according to claim 1 as an active ingredient.

17. An antioxidant, containing the material according to any one of claims 9 to 15 as an active ingredient.

18. An antioxidant for lipids, containing a processed product of an acerola fruit (excluding a processed product of an acerola seed) as an active ingredient.

19. The antioxidant according to claim 18, wherein the processed product of an acerola fruit contains polyphenol and/or ascorbic acid.

20. A food product having an antioxidative effect, to which the antioxidant according to any one of claims 16 to 19 is added.

21. A method for producing a food product, comprising a step of enhancing oxidation stability of a food product using the antioxidant according to any one of claims 16 to 19.

**22.** A skin-whitening agent for oral administration, containing the pectin or the hydrolysate thereof according to claim 1 as an active ingredient.

**23.** A skin-whitening agent for oral administration, containing the material according to any one of claims 9 to 15 as an active ingredient.

**24.** The skin-whitening agent for oral administration according to claim 22 or 23, further containing ascorbic acid.

**25.** A food product having a skin-whitening effect, to which the skin-whitening agent for oral administration according to any one of claims 22 to 24 is added.

**26.** A method for producing a skin-whitening agent for oral administration, comprising a step of hydrolyzing a pectin contained in the pulp of an acerola fruit or a processed product of an acerola fruit containing ascorbic acid and such pulp so that the amount of galacturonic acid is 5% by weight or more with respect to ascorbic acid.

**27.** The method according to claim 26, further comprising a step of substantially removing glucose and fructose.

Fig. 1

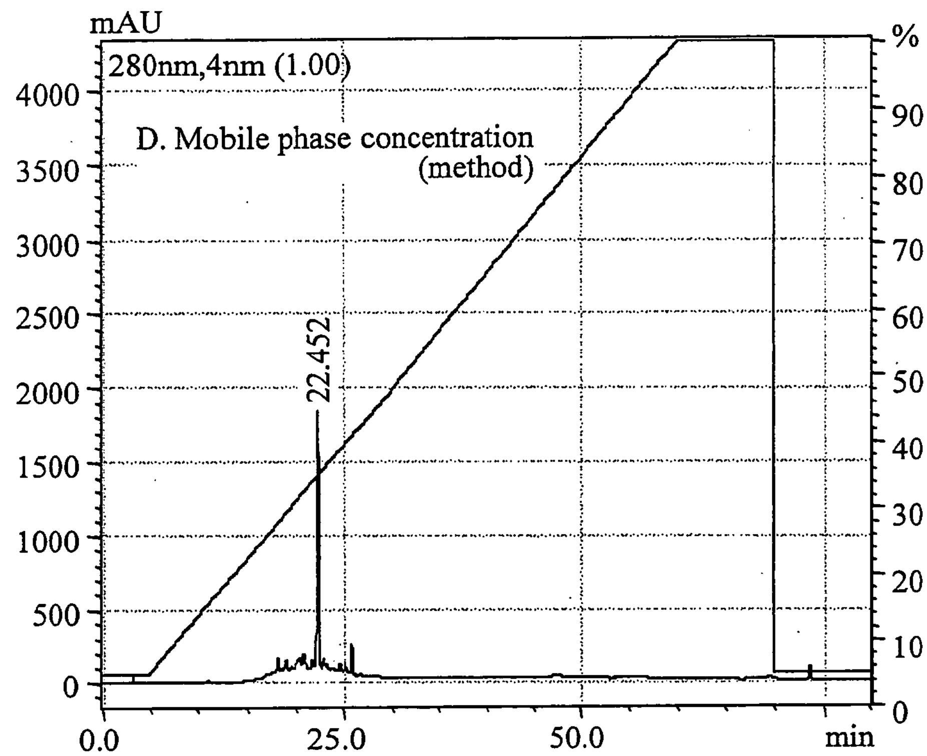
mAU
280nm,4nm (1.00)
D. Mobile phase concentration
(method)
22.452
4000
3500
3000
2500
2000
1500
1000
500
0
%
90
80
70
60
50
40
30
20
10
0
0.0
25.0
50.0
min

Fig. 2

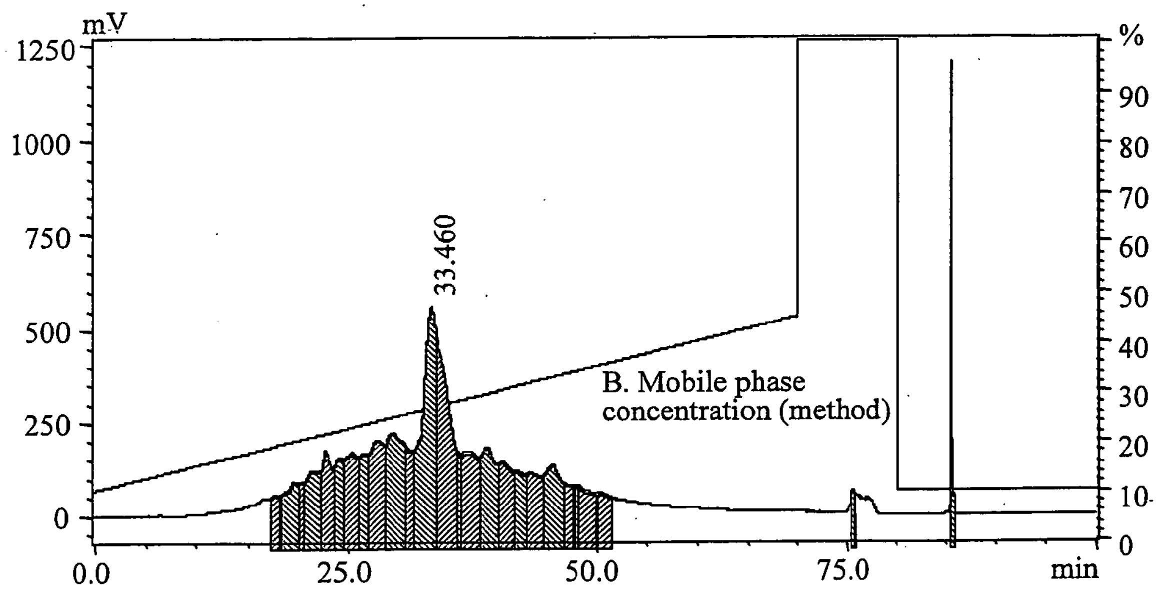
mV
1250
1000
750
500
250
0
33.460
B. Mobile phase
concentration (method)
0.0
25.0
50.0
75.0
min
%
90
80
70
60
50
40
30
20
10
0

Fig. 3

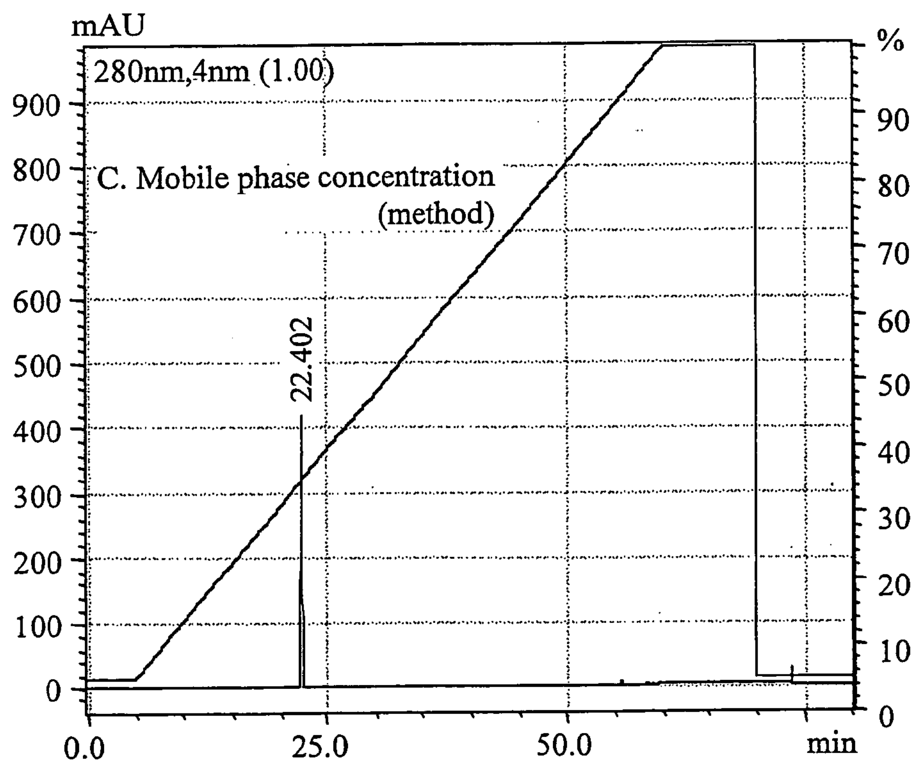
mAU
%
280nm,4nm (1.00)
C. Mobile phase concentration
(method)
22.402
900
800
700
600
500
400
300
200
100
0
90
80
70
60
50
40
30
20
10
0
0.0
25.0
50.0
min

Fig. 4A

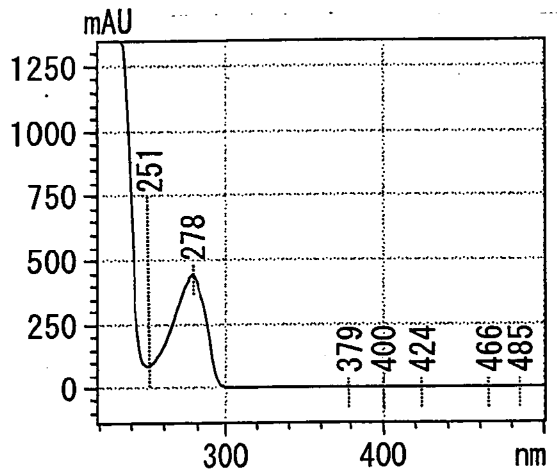
mAU
1250
1000
750
500
250
0
251
278
379
400
424
466
485
300
400
nm

Fig. 4B

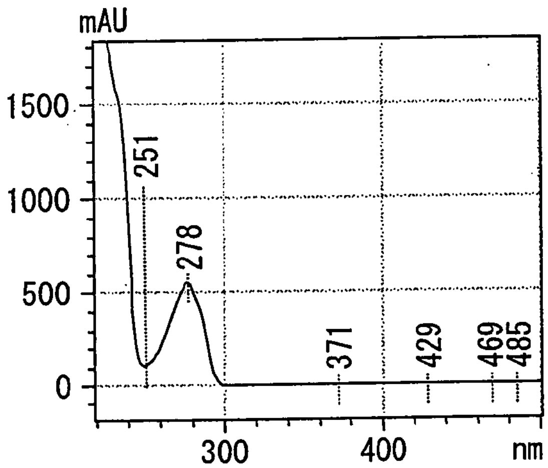

Fig. 5

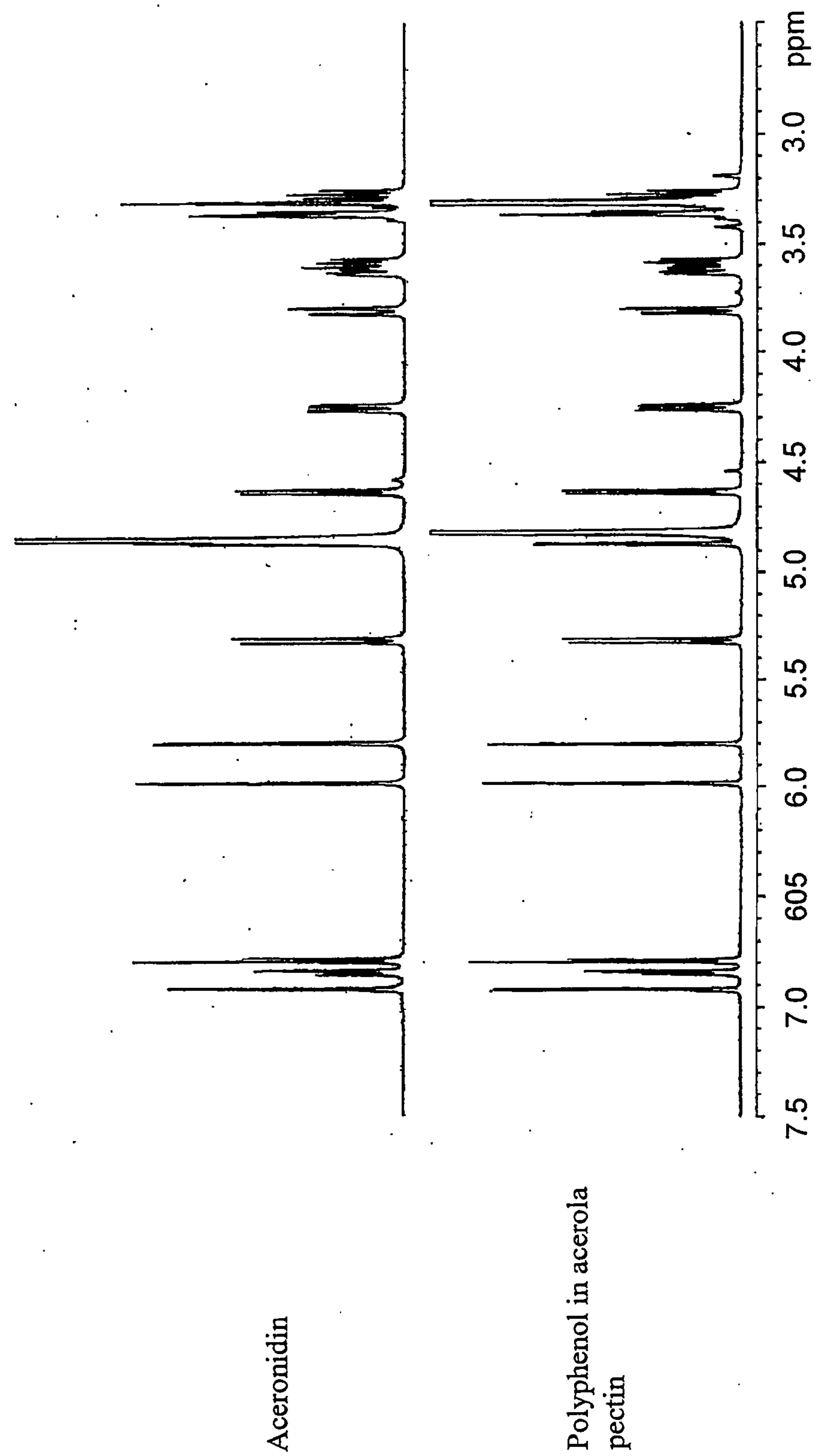

Aceronidin
Polyphenol in acerola pectin
ppm
3.0
3.5
4.0
4.5
5.0
5.5
6.0
605
7.0
7.5

Fig. 6A

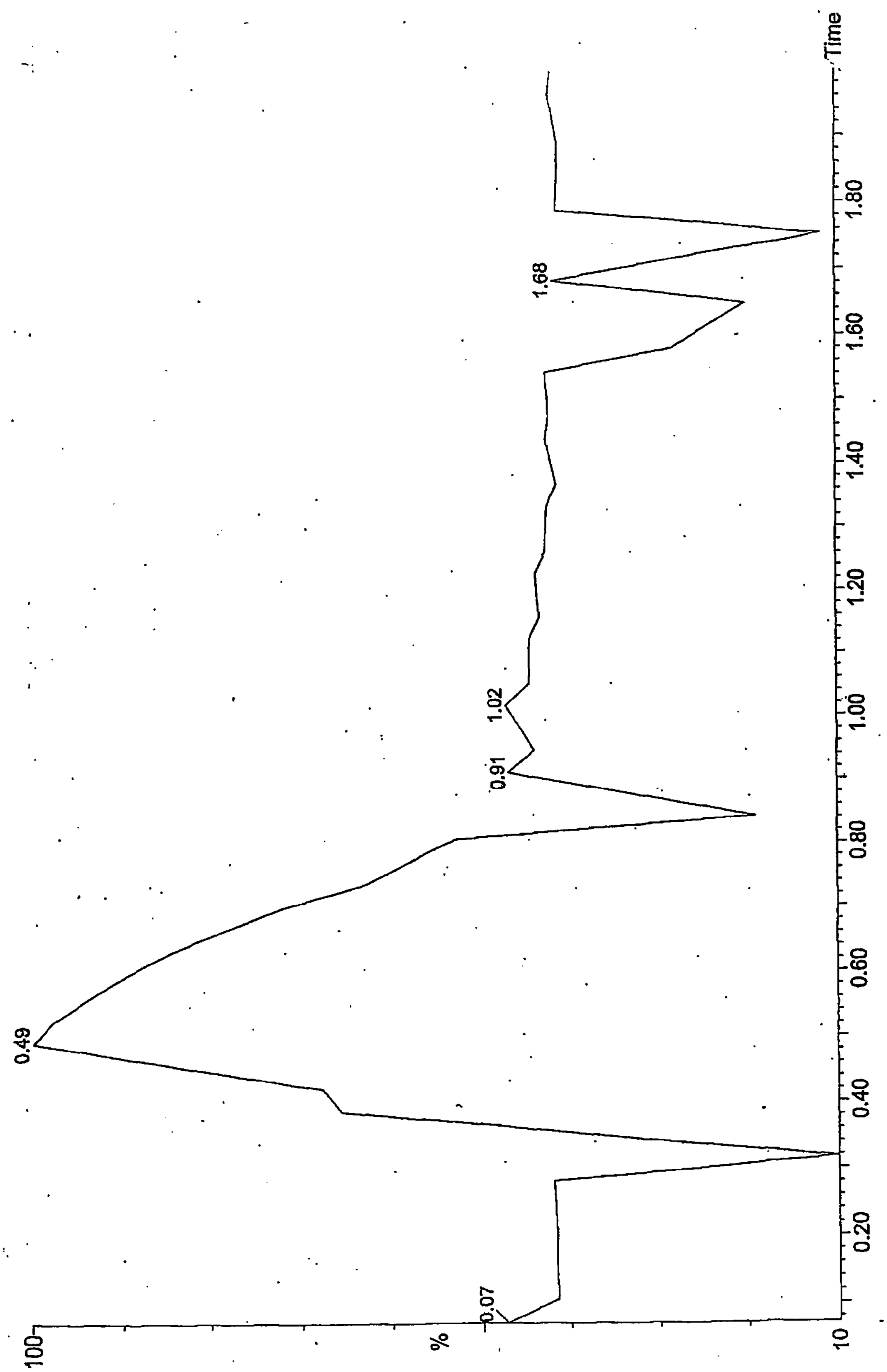

100
%
10
0.07
0.49
0.91
1.02
1.68
0.20
0.40
0.60
0.80
1.00
1.20
1.40
1.60
1.80
Time

Fig. 6B

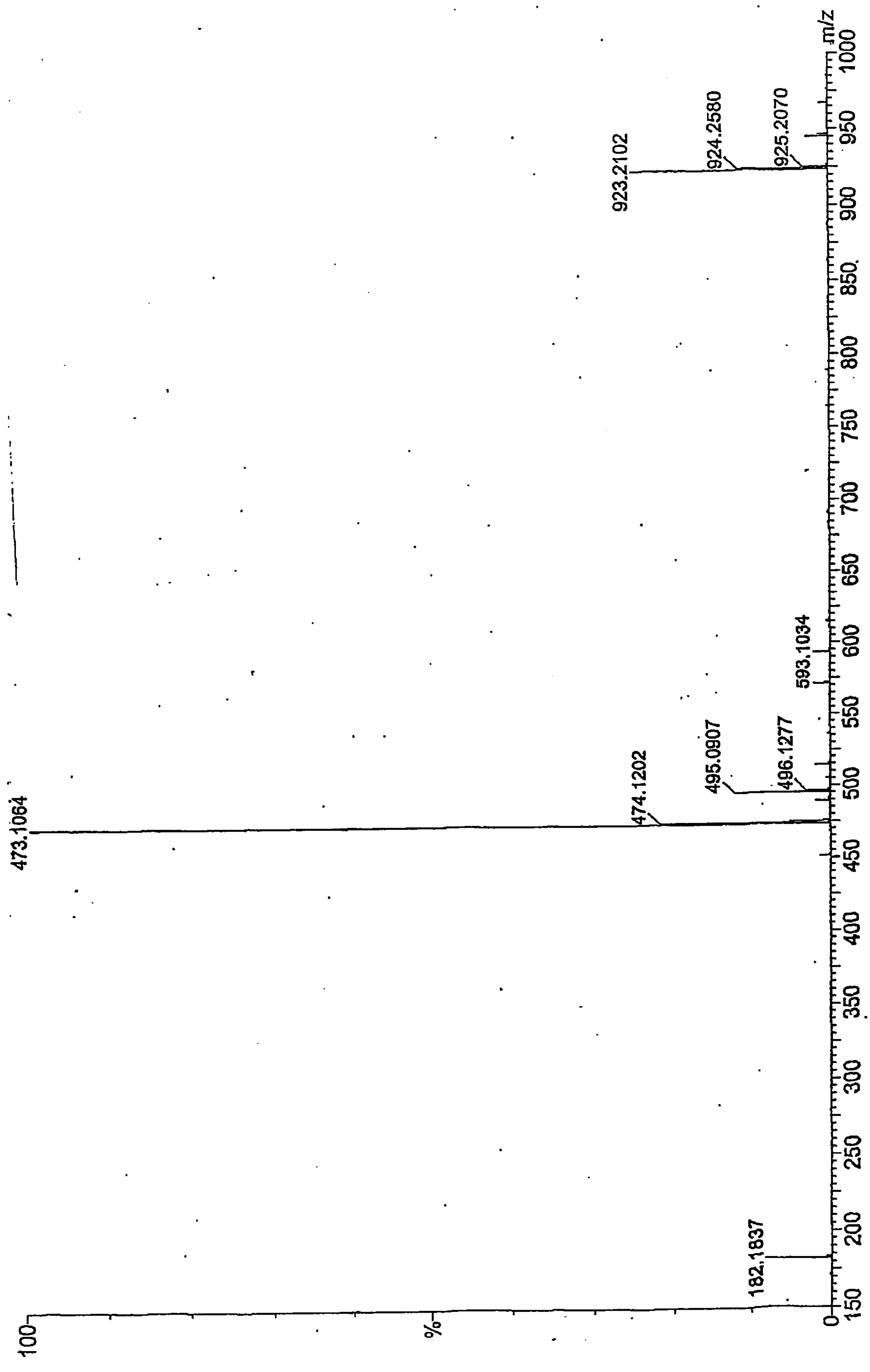

100
%
0
473.1064
474.1202
495.0907
496.1277
593.1034
182.1837
923.2102
924.2580
925.2070
150
200
250
300
350
400
450
500
550
600
650
700
750
800
850
900
950
1000
m/z

Fig. 7

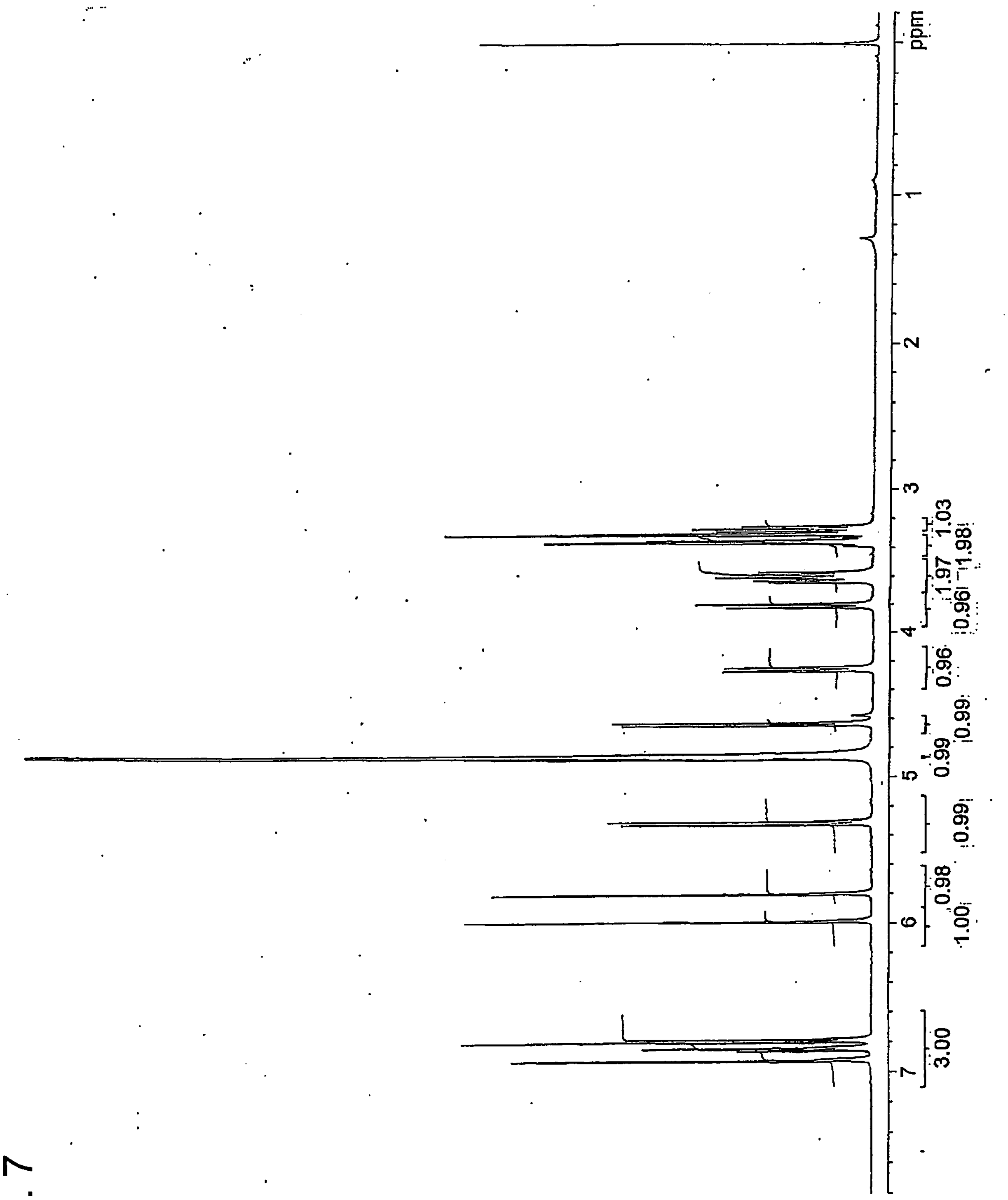

Fig. 8

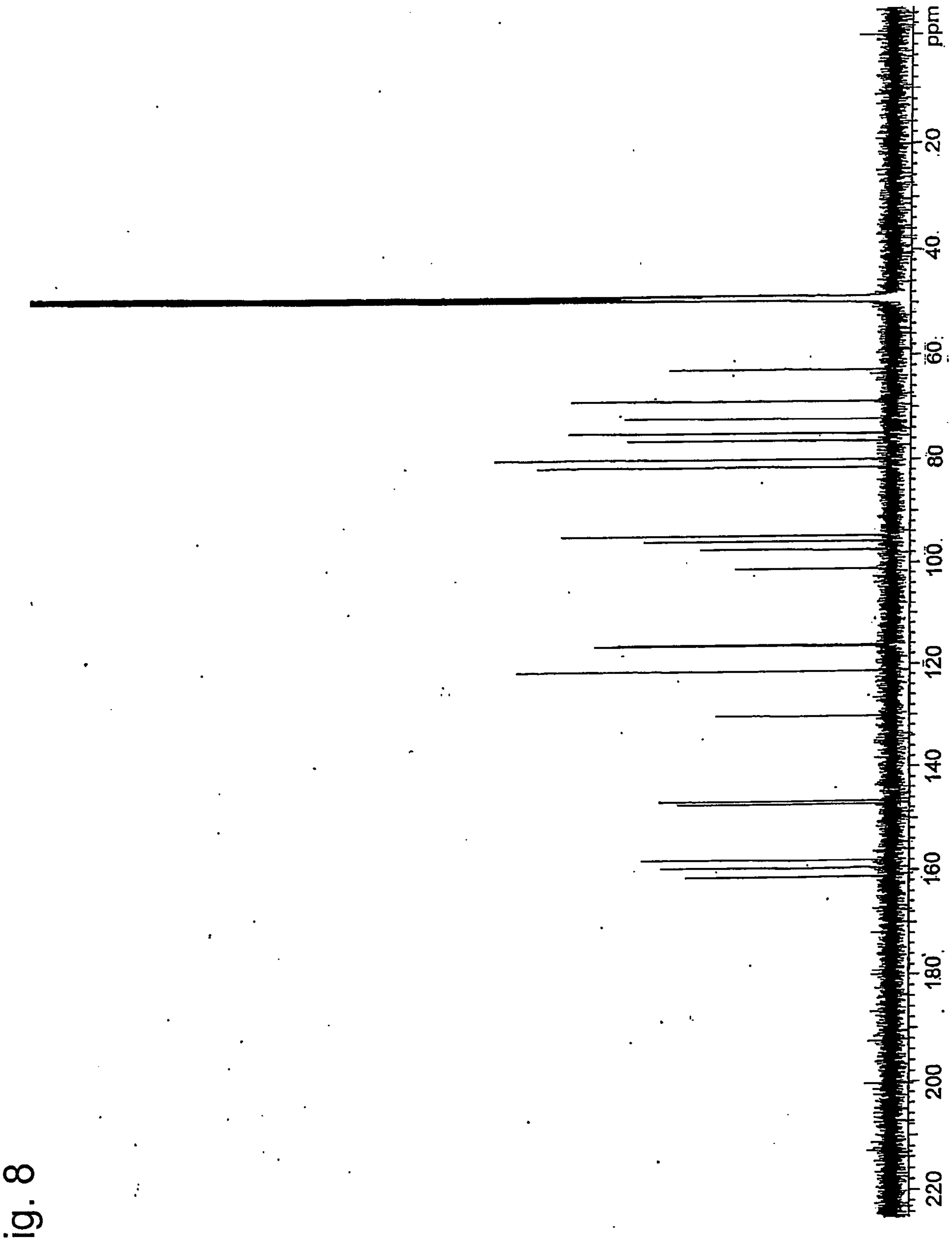

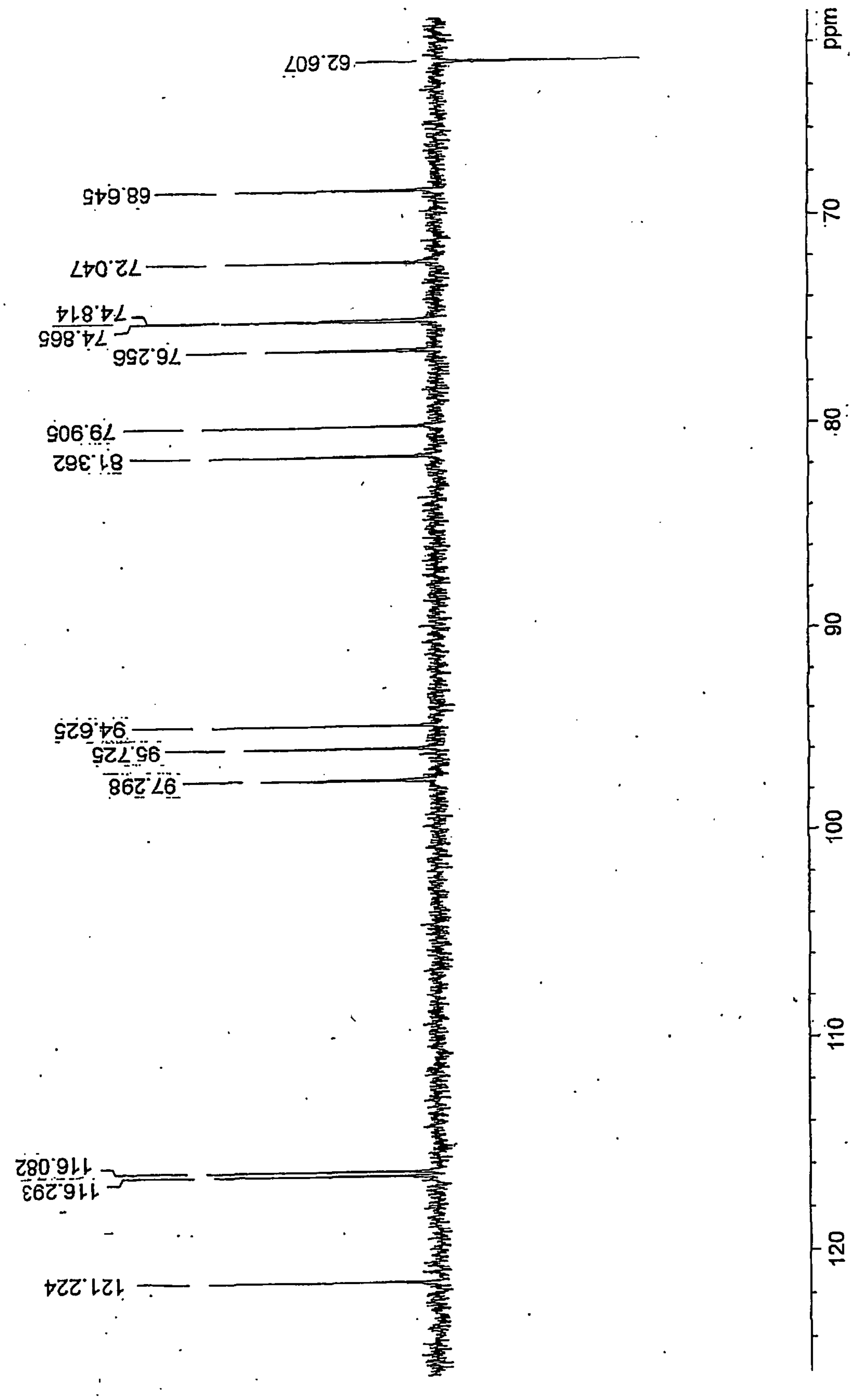
62.607
68.645
72.047
74.814
74.865
76.256
79.905
81.362
94.625
95.725
97.298
116.082
116.293
121.224
ppm
70
80
90
100
110
120

Fig. 9

Fig. 10

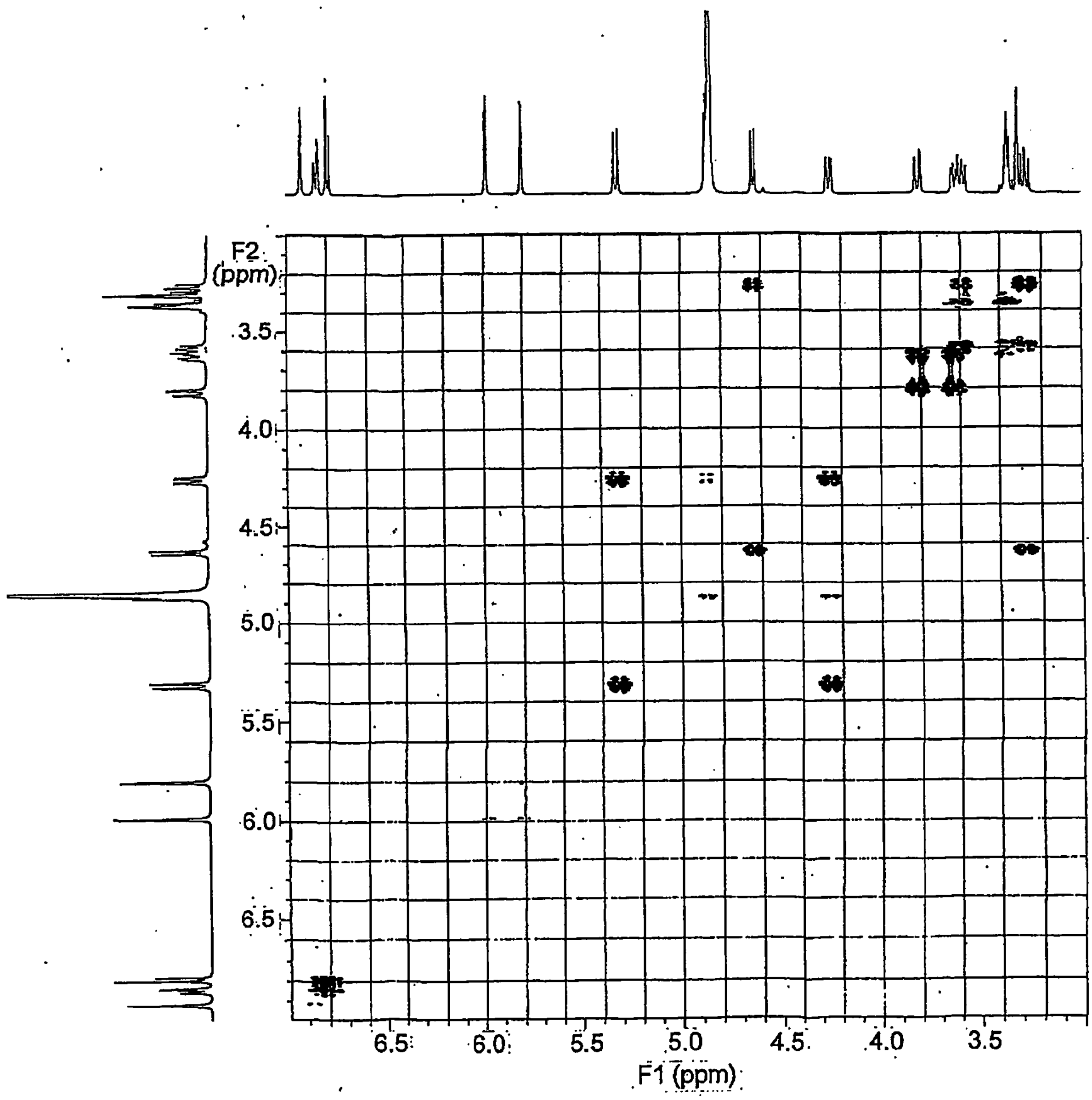

Fig. 11

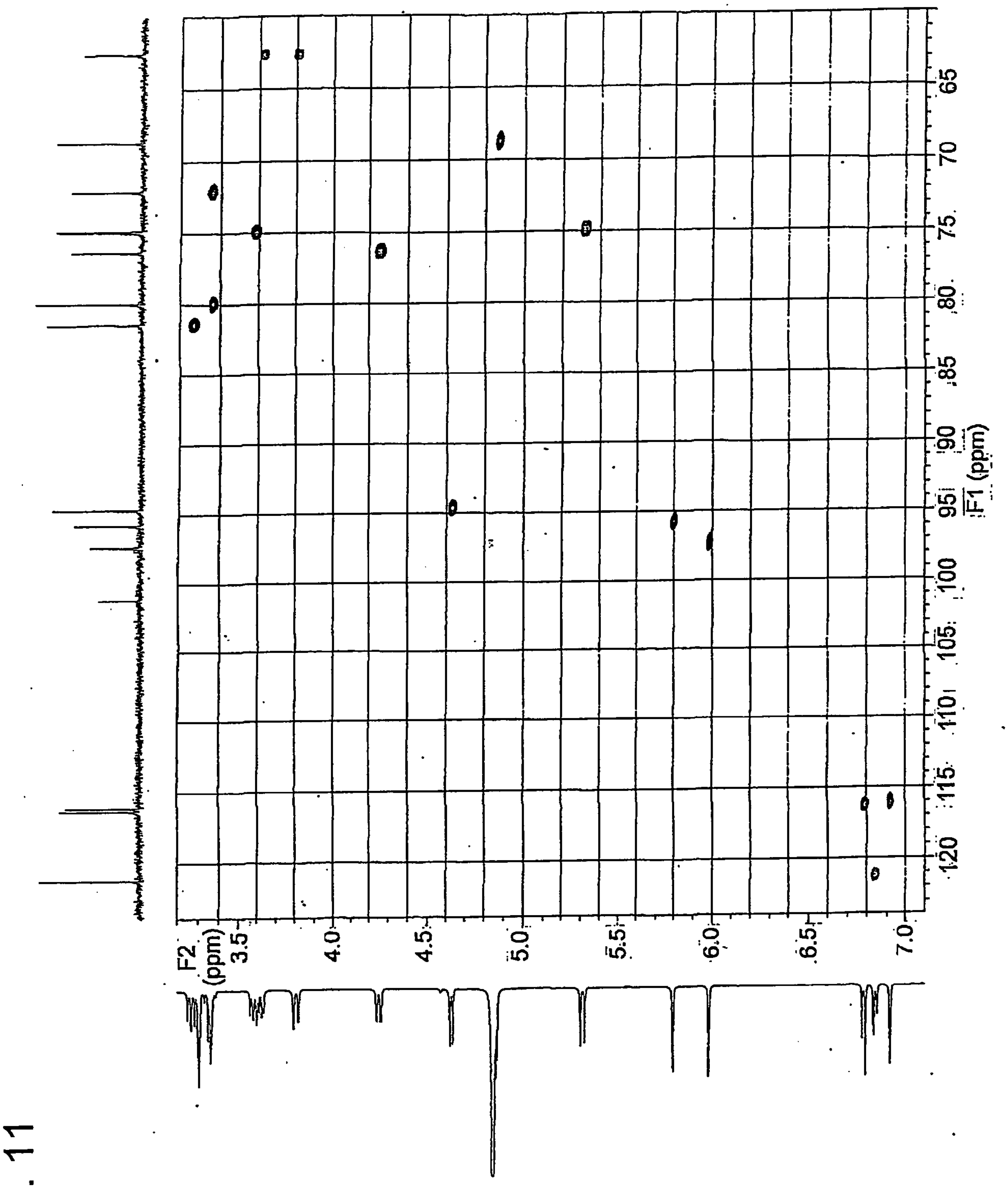
F2 (ppm)
3.5
4.0
4.5
5.0
5.5
6.0
6.5
7.0
65
70
75
80
85
90
95
100
105
110
115
120
F1 (ppm)

Fig. 12

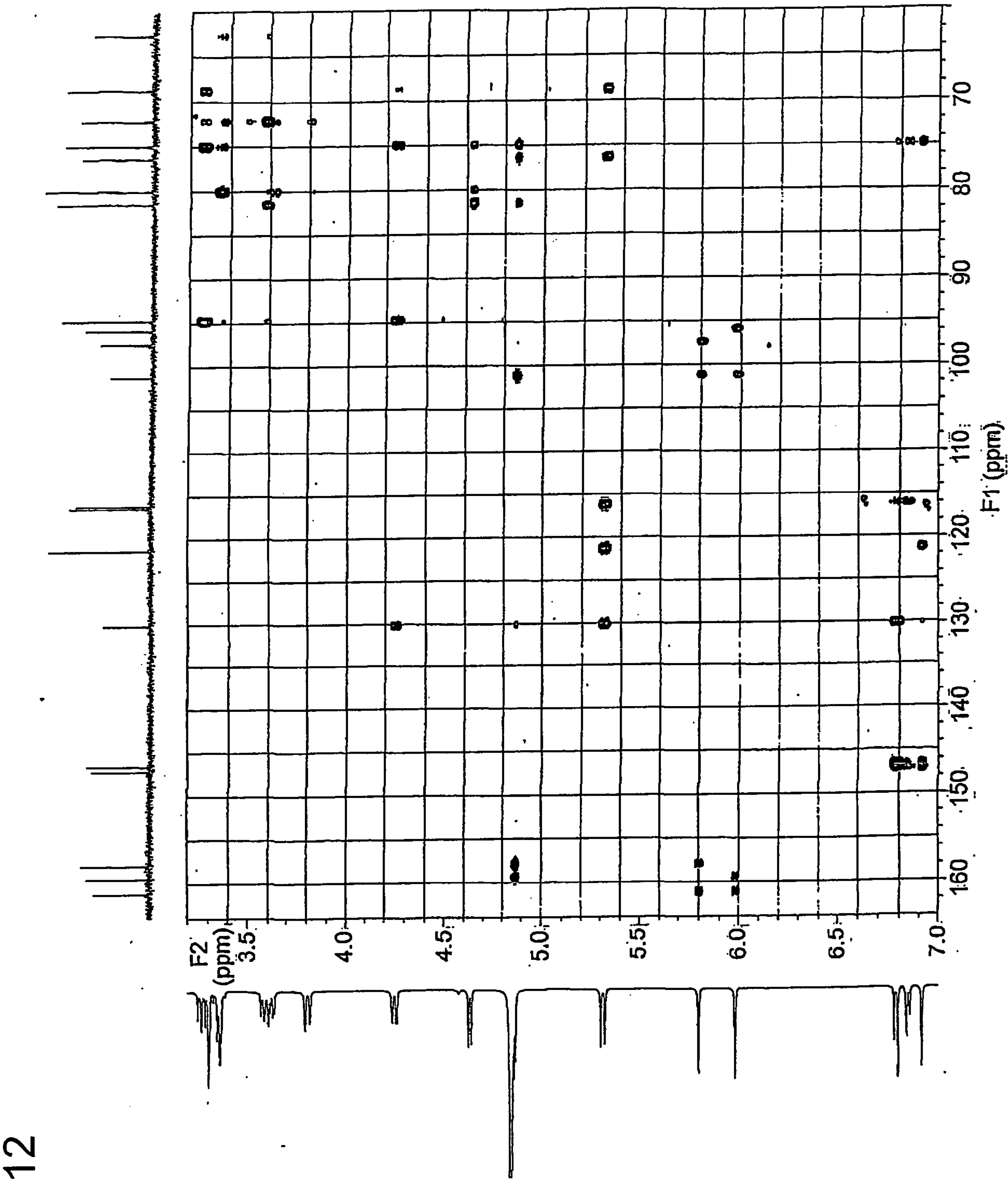
F2 (ppm)
3.5
4.0
4.5
5.0
5.5
6.0
6.5
7.0
70
80
90
100
110
120
130
140
150
160
F1 (ppm)

Fig. 13

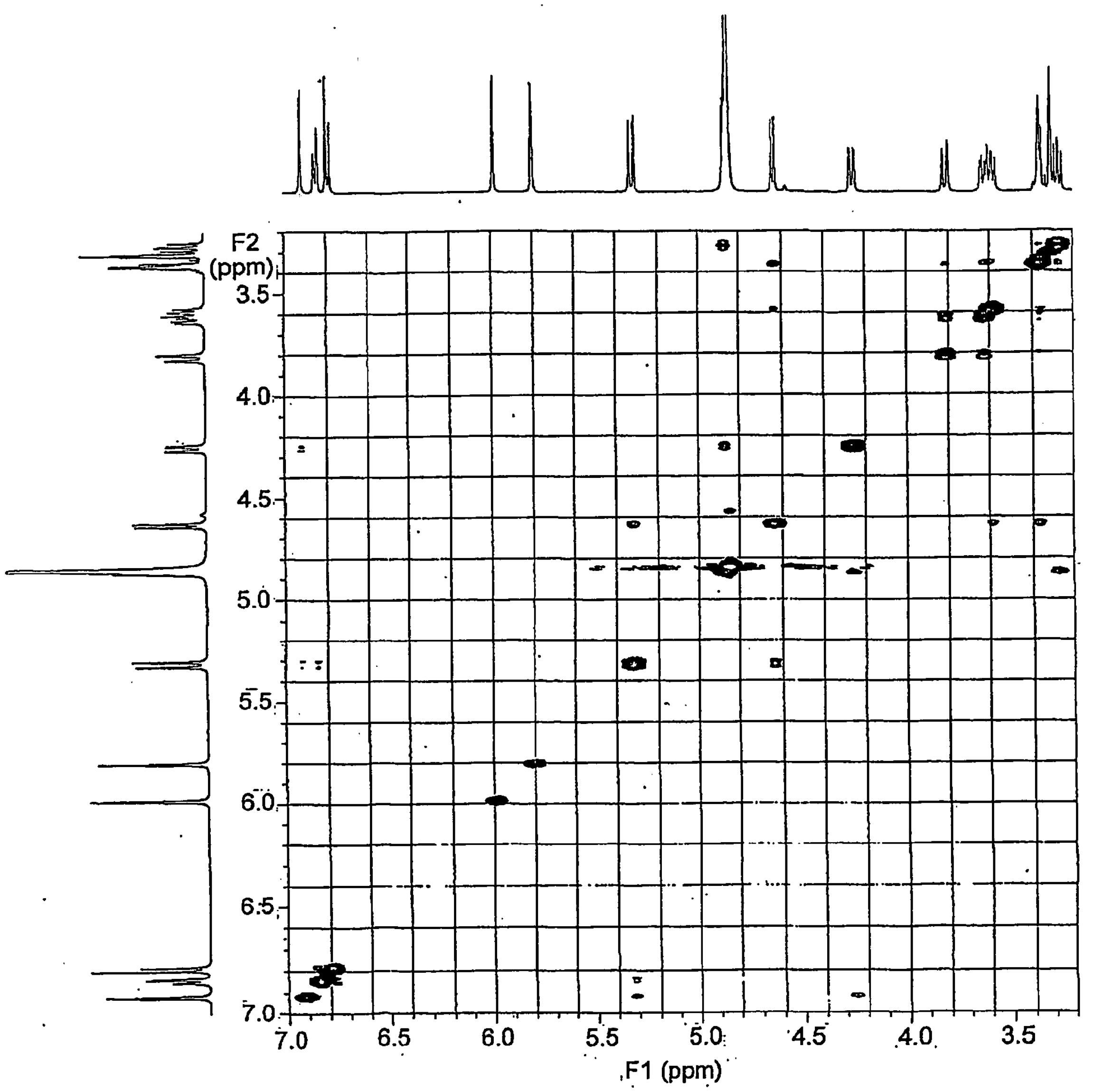

Fig. 14

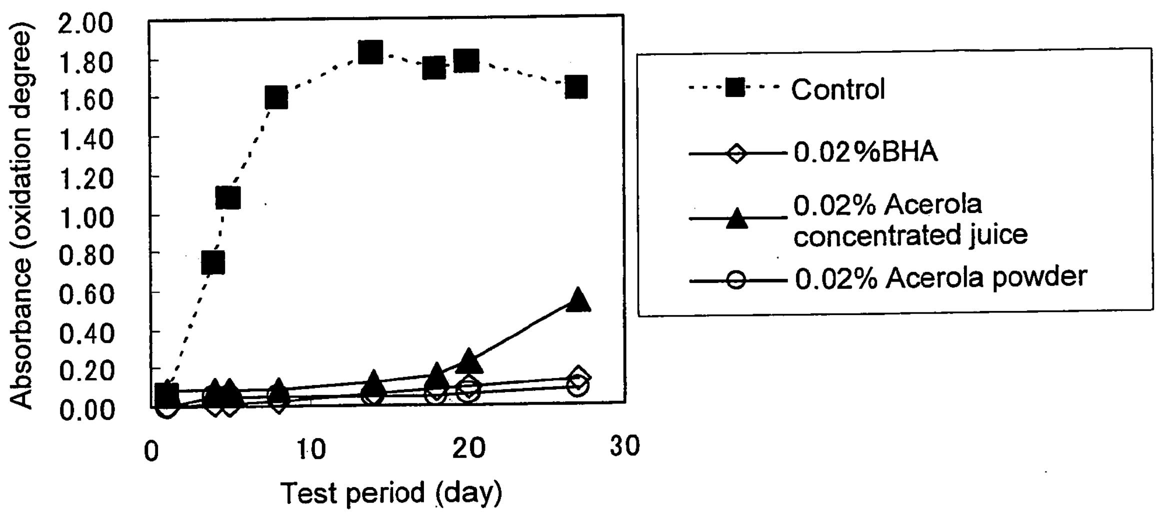
Absorbance (oxidation degree)
2.00
1.80
1.60
1.40
1.20
1.00
0.80
0.60
0.40
0.20
0.00
0
10
20
30
Test period (day)
Control
0.02%BHA
0.02% Acerola concentrated juice
0.02% Acerola powder

Fig. 15

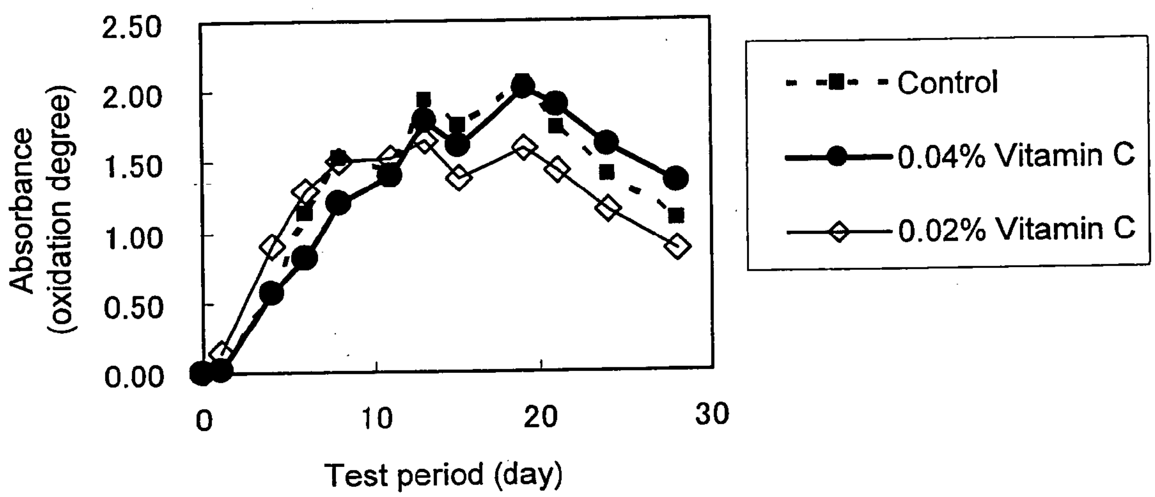
2.50
2.00
1.50
1.00
0.50
0.00
Absorbance
(oxidation degree)
0
10
20
30
Test period (day)
Control
0.04% Vitamin C
0.02% Vitamin C

Fig. 16

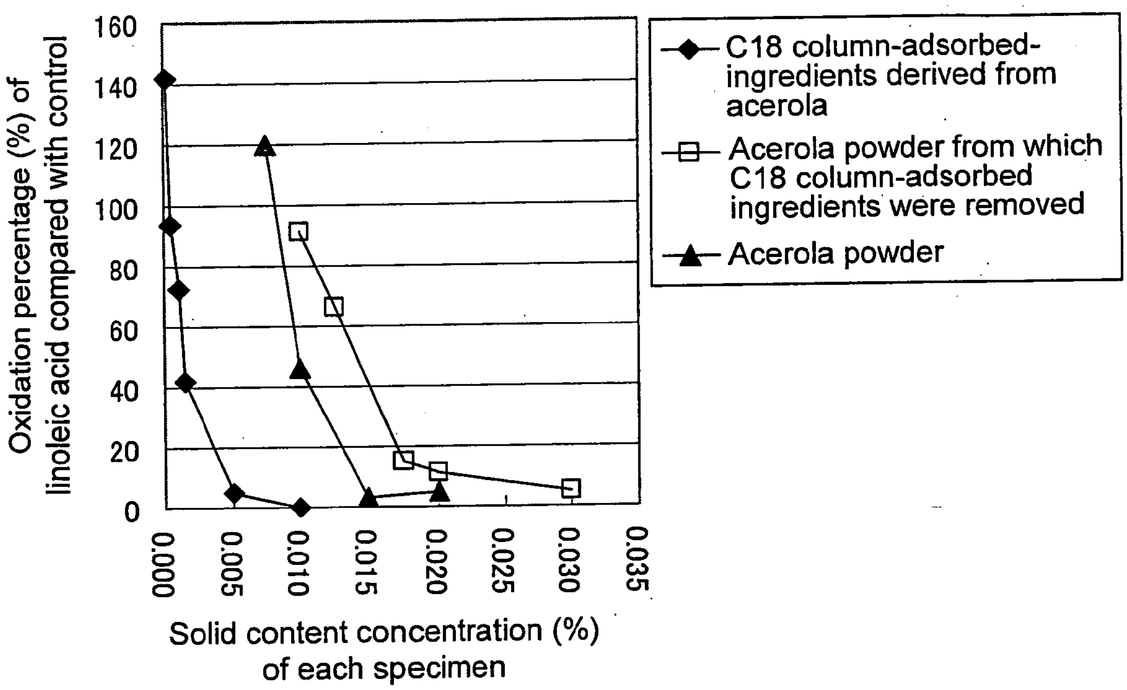
Oxidation percentage (%) of linoleic acid compared with control
160
140
120
100
80
60
40
20
0
0.000
0.005
0.010
0.015
0.020
0.025
0.030
0.035
Solid content concentration (%) of each specimen
C18 column-adsorbed-ingredients derived from acerola
Acerola powder from which C18 column-adsorbed ingredients were removed
Acerola powder

Fig. 17

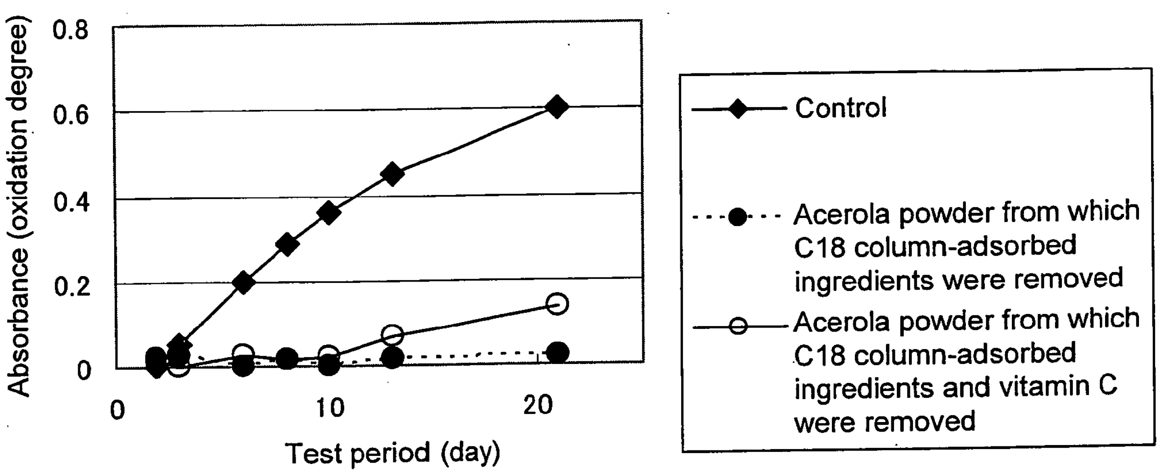
Absorbance (oxidation degree)
0.8
0.6
0.4
0.2
0
0
10
20
Test period (day)
Control
Acerola powder from which C18 column-adsorbed ingredients were removed
Acerola powder from which C18 column-adsorbed ingredients and vitamin C were removed

Fig. 18

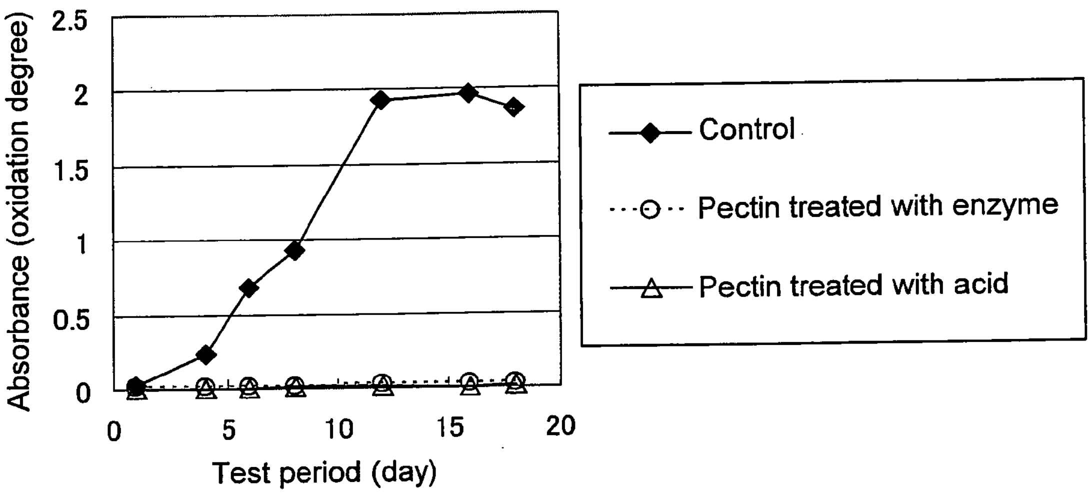
Absorbance (oxidation degree)
2.5
2
1.5
1
0.5
0
0
5
10
15
20
Test period (day)
Control
Pectin treated with enzyme
Pectin treated with acid

Fig. 19

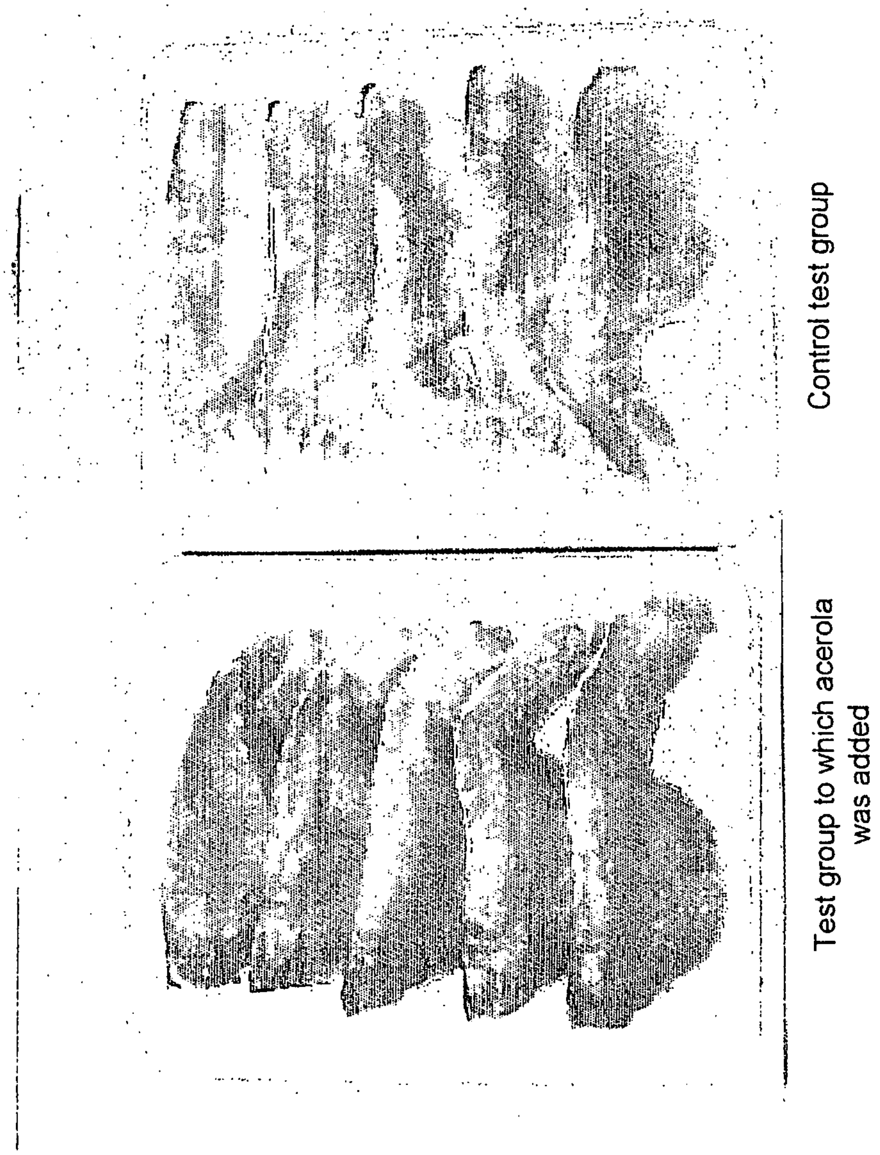

Fig. 20

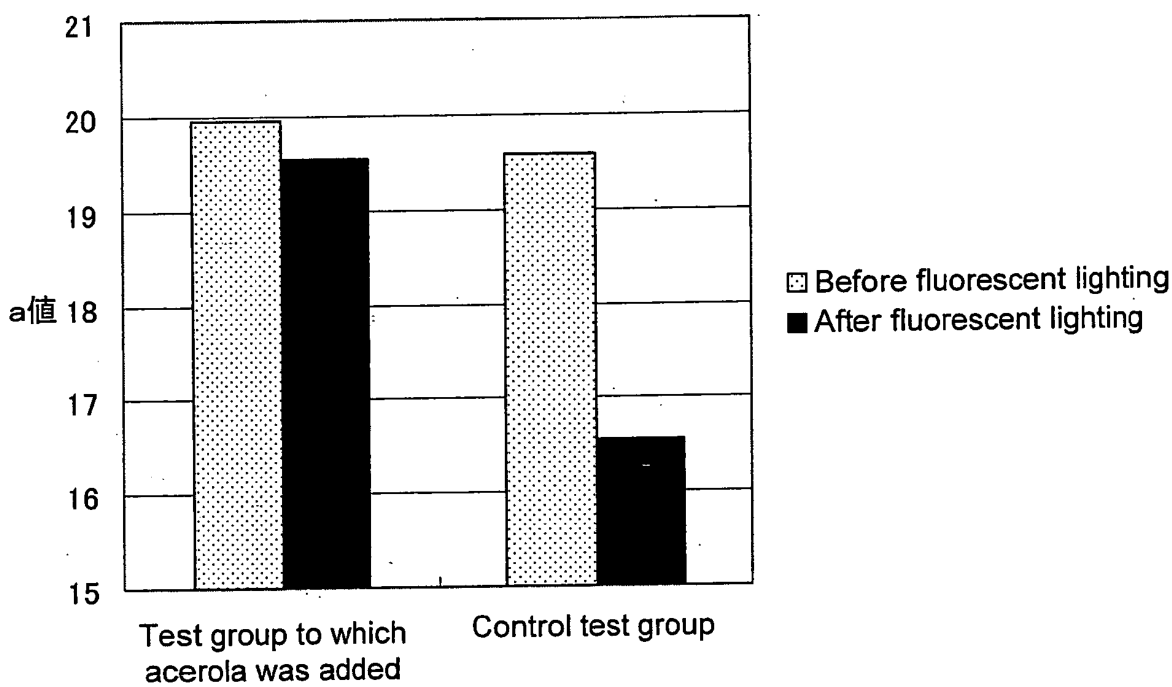
21
20
19
a値 18
17
16
15
Test group to which acerola was added
Control test group
Before fluorescent lighting
After fluorescent lighting

Fig. 21

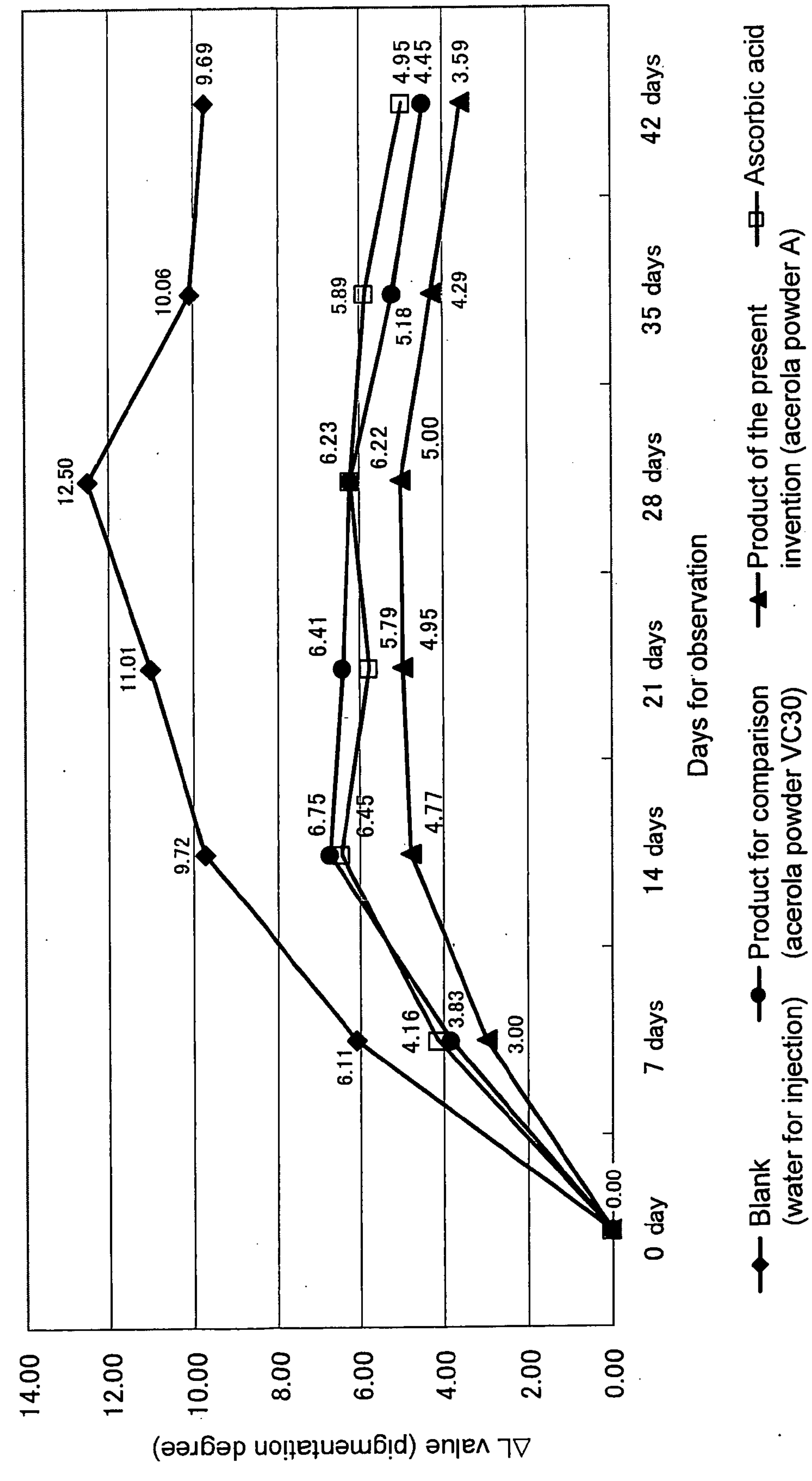
ΔL value (pigmentation degree)
14.00
12.00
10.00
8.00
6.00
4.00
2.00
0.00
0 day
7 days
14 days
21 days
28 days
35 days
42 days
Days for observation
0.00
6.11
4.16
3.83
3.00
9.72
6.75
6.45
4.77
11.01
6.41
5.79
4.95
12.50
6.23
6.22
5.00
10.06
5.89
5.18
4.29
9.69
4.95
4.45
3.59
Blank (water for injection)
Product for comparison (acerola powder VC30)
Product of the present invention (acerola powder A)
Ascorbic acid

**INTERNATIONAL SEARCH REPORT**

International application No. PCT/JP2006/304413

A. CLASSIFICATION OF SUBJECT MATTER
***C08B37/06***(2006.01), ***A61K8/97***(2006.01), ***A61K31/7048***(2006.01), ***A61K31/732***(2006.01), ***A61K36/18***(2006.01), ***A61P17/18***(2006.01), ***A61P43/00***(2006.01), ***A23L3/3472***(2006.01), ***A23L3/3544***(2006.01), ***A23L3/3562***(2006.01), ***C07H17/06***

According to International Patent Classification (IPC) or to both national classification and IPC

B. FIELDS SEARCHED

Minimum documentation searched (classification system followed by classification symbols)
A23L3/3472, A23L3/3544, A23L3/3562, A61K8/97, A61K31/7048, A61K31/732, A61K36/18, A61P17/18, A61P43/00, C07H17/06, C08B37/06, C09K15/06, C09K15/08, C09K15/34

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| P,X | JP 2005-343842 A (Kabushiki Kaisha Nichirei Fuzu), 15 December, 2005 (15.12.05), Full text (Family: none) | 18-21,25 |
| P,X | JP 2005-253463 A (Toyo Shinyaku Co., Ltd.), 22 September, 2005 (22.09.05), Full text (Family: none) | 20,21,25 |
| P,X | JP 2005-185188 A (Nichirei Corp.), 14 July, 2005 (14.07.05), Full text (Family: none) | 18-21,25 |

[X] Further documents are listed in the continuation of Box C. [ ] See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| 02 June, 2006 (02.06.06) | 20 June, 2006 (20.06.06) |
| Name and mailing address of the ISA/ Japanese Patent Office | Authorized officer |
| Facsimile No. | Telephone No. |

Form PCT/ISA/210 (second sheet) (April 2005)

**INTERNATIONAL SEARCH REPORT**

International application No.
PCT/JP2006/304413

C (Continuation). DOCUMENTS CONSIDERED TO BE RELEVANT

| Category* | Citation of document, with indication, where appropriate, of the relevant passages | Relevant to claim No. |
|---|---|---|
| P,X | JP 2005-154432 A (Nichirei Corp.), 16 June, 2005 (16.06.05), Full text (Family: none) | 18-21,25 |
| P,X | JP 2005-104841 A (Nichirei Corp.), 21 April, 2005 (21.04.05), Full text & WO 2005/030233 A1 | 18-21,25 |
| P,X | JP 2005-6540 A (Miki Foods Co., Ltd.), 13 January, 2005 (13.01.05), Full text; particularly, example 3 (Family: none) | 20,21,25 |
| P,A | JP 2005-320262 A (Nichirei Corp.), 17 November, 2005 (17.11.05), Full text (Family: none) | 1-27 |
| P,A | JP 2005-220085 A (Kose Corp.), 18 August, 2005 (18.08.05), Full text; particularly, Par. No. [0045] (Family: none) | 1-27 |
| P,A | JP 2005-220084 A (Kose Corp.), 18 August, 2005 (18.08.05), Full text; particularly, Par. No. [0003] (Family: none) | 1-27 |
| P,A | JP 2005-139093 A (Nichirei Corp.), 02 June, 2005 (02.06.05), Full text; particularly, Par. No. [0038] & WO 2005/044290 A1 | 1-27 |
| X<br>A | JP 9-276382 A (Kobayashi Pharmaceutical Co., Ltd.), 28 October, 1997 (28.10.97), Full text (Family: none) | 9-15<br>1-8 |
| X<br>A | JP 7-46971 A (Nichirei Corp.), 21 February, 1995 (21.02.95), Full text (Family: none) | 9-15<br>1-8 |
| X | JP 2003-524427 A (Citrus Sensation Pty Ltd.), 19 August, 2003 (19.08.03), Full text & WO 2001/064041 A1 & EP 1272042 A1 | 16-21,25 |

Form PCT/ISA/210 (continuation of second sheet) (April 2005)

**INTERNATIONAL SEARCH REPORT**

International application No.
PCT/JP2006/304413

C (Continuation). DOCUMENTS CONSIDERED TO BE RELEVANT

| Category* | Citation of document, with indication, where appropriate, of the relevant passages | Relevant to claim No. |
|---|---|---|
| X | JP 2001-187724 A (Kanebo, Ltd.),<br>10 July, 2001 (10.07.01),<br>Full text<br>(Family: none) | 16-19 |
| X | JP 2000-212026 A (Kose Corp.),<br>02 August, 2000 (02.08.00),<br>Full text; particularly, test example 1<br>(Family: none) | 16-19 |
| X | JP 2001-224299 A (Okumoto Seifun Kabushiki Kaisha),<br>21 August, 2001 (21.08.01),<br>Full text; particularly, Par. No. [0006]<br>(Family: none) | 20,21,25 |
| X | JP 10-109927 A (Kanebo, Ltd.),<br>28 April, 1998 (28.04.98),<br>Full text<br>(Family: none) | 20,21,25 |
| A | JP 2004-238345 A (Suzuko UECHI),<br>26 August, 2004 (26.08.04),<br>Full text<br>(Family: none) | 22-24,26,27 |
| A | JP 2000-212032 A (Kose Corp.),<br>02 August, 2000 (02.08.00),<br>Full text<br>(Family: none) | 22-24,26,27 |
| A | JP 10-316533 A (Amway Corp.),<br>02 December, 1998 (02.12.98),<br>Full text<br>& US 5747006 A | 22-24,26,27 |
| A | JP 2004-189698 A (Nichirei Corp.),<br>08 July, 2004 (08.07.04),<br>Full text<br>& WO 2004/054520 A1 & EP 1582195 A1 | 1-27 |
| A | JP 2004-175856 A (Nichirei Corp.),<br>24 June, 2004 (24.06.04),<br>Full text; particularly, example 1<br>& WO 2004/048498 A1 & EP 1602704 A1<br>& US 2006/51439 A1 | 1-27 |
| A | JP 2-200610 A (Nichirei Corp.),<br>08 August, 1990 (08.08.90),<br>Full text<br>(Family: none) | 1-27 |

Form PCT/ISA/210 (continuation of second sheet) (April 2005)

**INTERNATIONAL SEARCH REPORT**

International application No.
PCT/JP2006/304413

Continuation of A. CLASSIFICATION OF SUBJECT MATTER
(International Patent Classification (IPC))

(2006.01), ***C09K15/06***(2006.01), ***C09K15/08***(2006.01), ***C09K15/34***(2006.01)

(According to International Patent Classification (IPC) or to both national classification and IPC)

Form PCT/ISA/210 (extra sheet) (April 2005)

**INTERNATIONAL SEARCH REPORT**

International application No.
PCT/JP2006/304413

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

In this international application, the following four groups of inventions are included: the inventions of claims 1 to 8, 16 and 22 and parts of claims 20, 21, 24 and 25; the inventions of claims 9 to 15, 17 and 23 and parts of claims 20, 21, 24 and 25; the inventions of claims 18 and 19 and parts of claims 20 and 21; and the inventions of claims 26 and 27.

It appears, *prima facie*, that the special technical feature common to these groups resides in an acerola fruit extract. However, since an acerola fruit extract is publicly known as disclosed in JP 9-276382 A which is a

(continued to extra sheet)

1. ☐ As all required additional search fees were timely paid by the applicant, this international search report covers all searchable claims.
2. ☒ As all searchable claims could be searched without effort justifying an additional fee, this Authority did not invite payment of any additional fee.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** the

☐ The additional search fees were accompanied by the applicant's protest and, where applicable, payment of a protest fee..

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2005)

INTERNATIONAL SEARCH REPORT

International application No.
PCT/JP2006/304413

Continuation of Box No.III of continuation of first sheet(2)

document cited in the international search report, it cannot be considered that the special technical feature mentioned above goes beyond the prior art.

Consequently, the four groups of inventions do not comply with the requirement of unity of invention.

Form PCT/ISA/210 (extra sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 3513871 B
- JP 3076787 B
- JP 3596953 B
- JP 2005053479 A
- JP 2005088860 A
- JP 2004372266 A


### Non-patent literature cited in the description


- *Shokuhin-no-hoso,* 1986, vol. 17, 106
- *FOOD SCIENCE,* 1994, vol. 36 (11), 93-102
- **TAKAAKI MANABE.** Science and Food Texture of Pectin. Saiwai Shobo, 2001, 8-22
- **EIJI NARU et al.** *Fragrance Journal,* 2004, vol. 32 (8), 24-30